Europäisches Patentamt

European Patent Office

Office européen des brevets

(11) Publication number: **0 337 896 B1**

(12) **EUROPEAN PATENT SPECIFICATION**

(45) Date of publication of patent specification: **28.07.93** (51) Int. Cl.5: **C12Q 1/68**, C07H 21/00

(21) Application number: **89401045.3**

(22) Date of filing: **14.04.89**

(54) **Hybridization probes for detecting neisseria strains.**

(30) Priority: **15.04.88 EP 88400929**

(43) Date of publication of application:
**18.10.89 Bulletin 89/42**

(45) Publication of the grant of the patent:
**28.07.93 Bulletin 93/30**

(84) Designated Contracting States:
**AT BE CH DE ES FR GB GR IT LI LU NL SE**

(56) References cited:
**EP-A- 0 155 359**
**EP-A- 0 237 737**
**EP-A- 0 245 129**
**EP-A- 0 250 662**
**EP-A- 0 272 009**

**FEMS MICROBIOLOGY LETTERS, vol. 43,
1987, Amsterdam (NL); G.HAUN et al., pp.
187-193\***

**CHEMICAL ABSTRACTS, vol. 104, no. 25, 23
June 1986, Columbus, OH (US); R.ROSSAU,
p. 326, no. 221758w\***

**BIOLOGICAL ABSTRACTS/RRM, vol. 35, 1988;
R.ROSSAU et al., no. 35069014\***

**BIOLOGICAL ABSTRACTS/RRM, vol. 35, 1988;
M.E.HARPER et al., no. 35049041\***

(73) Proprietor: **N.V. INNOGENETICS S.A.
Industriepark Zwijnaarde 7, Box 4
B-9710 Gent(BE)**

(72) Inventor: **Rossau, Rudi
Wilgehoevestraat 45
B-2070 Ekeren(BE)**
Inventor: **Van Heuverswijn, Hugo
Colmanstraat 62
B-9288 Laarne(BE)**

(74) Representative: **Grosset-Fournier, Chantal
Catherine et al
SC Ernest Gutmann/Yves Plasseraud 67 bou-
levard Haussmann
F-75008 Paris (FR)**

EP 0 337 896 B1

**Description**

The invention relates to hybridization probes for detecting Neisseria strains and isolates belonging to the genus Neisseria and related taxa. Hereafter, the word strains also encompasses isolates or organisms contained in a biological sample.

Most of the probes known so far and which can be either total genomic deoxyribonucleic acid (DNA), indigenous plasmids, cloned DNA fragments, or synthetic oligonucleotides, target the DNA of the organism to be detected.

It has been suggested in a few occurences to target the ribosomal ribonucleic acid (rRNA), by means of a rRNA derived probe.Targeting the ribosomal ribonucleic acid would increase the sensitivity of a diagnostic test, because ribosomes are very abundant in a cell.

Yet and in contrast, the high sequence conservation observed among rRNA cistrons and accordingly, the absence of specificity of a rRNA derived probe are important drawbacks which are an obstacle to the use of a rRNA derived probe for selecting related taxa. In fact rRNA derived probes known so far are mainly used to detect large groups of organisms such as Legionella (Wilkinson et al., 1986 ; Edelstein, 1986) or the Pseudomonas fluorescens group (Festl et al., 1986), or to differentiate relatively distantly related species such as Mycoplasma (Goebel et al., 1987) and Chlamydia species (Palmer et al., 1986) from one another. One report described the differentiation between the species Proteus vulgarus and Proteus mirabilis (Haun and Goebel, 1987). Both species have a DNA homology value of about 50 % ; for the moment this is the highest specificity which could be reached using rRNA derived probes without the use of Southern-blot analysis.

EP 0 272 009 discloses a method for preparing probes, as well as several probes for use in qualitative or quantitative hybridization assays. The method comprises constructing an oligonucleotide that is sufficiently complementary to hybridize to a region of rRNA selected to be unique to a non-viral organism or group of non-viral organisms sought to be detected, said region of rRNA being selected by comparing one or more variable region rRNA sequences of said non-viral organism or group of non-viral organisms with one or more variable region rRNA sequences from one or more non-viral organisms sought to be distinguished. A sequence disclosed in relation to Neisseria is TCA TCG GCC GCC GAT ATT GGC.

Therefore it was unexpected, as found by the inventors of the present invention, that specific rRNA derived probes could be devised which could not only differentiate between highly related bacterial species but also between taxa related at the subspecies level using a simple direct hybridization format. In particular, Neisseria gonorrhoeae strains could be discriminated from other Neisseria strains, including N. meningitidis strains, by means of a dot-spot hybridization assay by some of the probes described herein.

Thus an object of the invention is to provide rRNA-related probes for detecting one or more Neisseria strains.

Another object of the invention is to provide rRNA-related probes for differentiating Neisseria gonorrhoeae from other bacterial species and in particular from other Neisseria species and from Neisseria meningitidis.

A further object of the invention is to provide probes for detecting one or more Neisseria strains by a simple hybridization test, such as a dot-spot hybridization test, without resorting to any complementary analysis, such as the Southern-blot analysis.

Still another object of the invention is to provide a probe and a simple method for the in vitro diagnosis of one or more Neisseria strains.

"rRNA-related" as used herein refers to the fact that the probes concerned hybridize with sequences normally present in ribosomal RNAs, no matter whether said probes are themselves formed of DNA or RNA fragments, or whether they consist of cloned fragments (in the case of DNA) or of synthetic oligonucleotides.

The word "Neisseria" as used herein not only refers to bacteria named Neisseria but also to named or unnamed taxa, such as Kingella, Eikenella, Simonsiella, Alysiella and the CDC groups EF-4 and M-5, which are highly interrelated with bacteria belonging to the genus Neisseria. These taxa are found within a Tm(e) range of approximately 6°C versus ribosomal RNA of flavescens ATCC 13120. Tm(e) is defined in Rossau, R., A. Van Landschoot, W. Mannheim, and J. De Ley. 1986. Inter- and intrageneric similarities of ribosomal ribonucleic acid cistrons of the Neisseriaceae. Int. J. Syst. Bacteriol. 36:323-332.

It should be noted that misnamed bacteria, which are not found within the delta Tm(e) range indicated, such as Neisseria caviae, Neisseria cuniculi, Neisseria ovis, Neisseria catarrhalis (Branhamella (Moraxella) catarrhalis), Kingella indologenes and Alysiella sp., do not belong to the Neisseria group.

A hybridization probe of the invention for detecting one or more Neisseria strains contains :

- either a sequence belonging to a nucleic acid selected from the following groups of nucleic acids and which includes itself of from 10 to the maximum number of nucleotides of the selected nucleic acid

**Group 4 :**

GGTACCGTCATCGGCCGCCGATATTGGCAACAGCCTTTTCTTCCCTGACAAAAGTCC

(4)

GGUACCGUCAUCGGCCGCCGAUAUUGGCAACAGCCUUUUCUUCCCUGACAAAAGUCC

(4bis)

GGACTTTTGTCAGGGAAGAAAAGGCTGTTGCCAATATCGGCGGCCGATGACGGTACC

(4ter)

GGACUUUUGUCAGGGAAGAAAAGGCUGUUGCCAAUAUCGGCGGCCGAUGACGGUACC

(4quater)

**Group 5 :**

ACGCTACCAAGCAATCAAGTTGCCCAACAGCTAA          (5)

ACGCUACCAAGCAAUCAAGUUGCCCAACAGCUAA          (5bis)

```
TTAGCTGTTGGGCAACTTGATTGCTTGGTAGCGT                              (5ter)
UUAGCUGUUGGGCAACUUGAUUGCUUGGUAGCGU                              (5quater)
```

Group 6 :

```
ATACCGTGGTAAGCGGACTCCTTGCGGTTACCCTACCTACTTCTGGTATCCCCCAC
                                                                (6)
AUACCGUGGUAAGCGGACUCCUUGCGGUUACCCUACCUACUUCUGGUAUCCCCCAC
                                                                (6bis)
GTGGGGGATACCAGAAGTAGGTAGGGTAACCGCAAGGAGTCCGCTTACCACGGTAT
                                                                (6ter)
GUGGGGGAUACCAGAAGUAGGUAGGGUAACCGCAAGGAGUCCGCUUACCACGGUAU
                                                                (6quater)
```

Group 7 :

```
TCAGTCCGATTTCCGCCGGACCTAGGT                                     (7)
UCAGUCCGAUUUCCGCCGGACCUAGGU                                     (7bis)
ACCTAGGTCCGGCGGAAATCGGACTGA                                     (7ter)
ACCUAGGUCCGGCGGAAAUCGGACUGA                                     (7quater)
```

Group 8 :

```
TCAGTCCGATTTCCGACCGGACCTAGGT                                    (8)
UCAGUCCGAUUUCCGACCGGACCUAGGU                                    (8bis)
ACCTAGGTCCGGTCGGAAATCGGACTGA                                    (8ter)
ACCUAGGUCCGGUCGGAAAUCGGACUGA                                    (8quater)
```

Group 9 :

```
CGCCACCCGAGAAGCAAGCTTCCCTGTGCTGC                                (9)
CGCCACCCGAGAAGCAAGCUUCCCUGUGCUGC                                (9bis)
GCAGCACAGGGAAGCTTGCTTCTCGGGTGGCG                                (9ter)
GCAGCACAGGGAAGCUUGCUUCUCGGGUGGCG                                (9quater)
```

Group 10 :

```
CGGGGCTTACGGAGCAAGTCCTTAACCTTAGAGGGCATA                         (10)
CGGGGCUUACGGAGCAAGUCCUUAACCUUAGAGGGCAUA                         (10bis)
TATGCCCTCTAAGGTTAAGGACTTGCTCCGTAAGCCCCG                         (10ter)
UAUGCCCUCUAAGGUUAAGGACUUGCUCCGUAAGCCCCG                         (10quater)
```

Group 11 :

```
GCGGAATCATAGCTTTATTGCCAGCTCCCCCGC                               (11)
GCGGAAUCAUAGCUUUAUUGCCAGCUCCCCCGC                               (11bis)
GCGGGGGGAGCTGGCAATAAAGCTATGATTCCGC                              (11ter)
```

```
GCGGGGGAGCUGGCAAUAAAGCUAUGAUUCCGC                    (11quater)
```

Group 12 :

```
GACACACTCGAGTCACCCAGTTCAGAAC                         (12)
GACACACUCGAGUCACCCAGUUCAGAAC                         (12bis)
GTTCTGAACTGGGTGACTCGAGTGTGTC                         (12ter)
GUUCUGAACUGGGUGACUCGAGUGUGUC                         (12quater)
```

Group 13 :

```
TGCTTTCCCTCTCAAGACGTATGC                             (13)
UGCUUUCCCUCUCAAGACGUAUGC                             (13bis)
GCATACGTCTTGAGAGGGAAAGCA                             (13ter)
GCAUACGUCUUGAGAGGGAAAGCA                             (13quater)
```

Group 14 :

```
TCTCGACAGTTATTACGTACA                                (14)
UCUCGACAGUUAUUACGUACA                                (14bis)
TGTACGTAATAACTGTCGAGA                                (14ter)
UGUACGUAAUAACUGUCGAGA                                (14quater)
```

Group 15 :

```
TTTCGTACGCTTAGTACCGCTGTTGAGA                         (15)
UUUCGUACGCUUAGUACCGCUGUUGAGA                         (15bis)
TCTCAACAGCGGTACTAAGCGTACGAAA                         (15ter)
UCUCAACAGCGGUACUAAGCGUACGAAA                         (15quater)
```

Group 16 :

```
GTGGTATCGGTTGCTTCGTGTCCGTAGACA                       (16)
GUGGUAUCGGUUGCUUCGUGUCCGUAGACA                       (16bis)
TGTCTACGGACACGAAGCAACCGATACCAC                       (16ter)
UGUCUACGGACACGAAGCAACCGAUACCAC                       (16quater)
```

Group 17 :

```
AAGCTATTCCAACAGCTTGCCAACCTAA                         (17)
AAGCUAUUCCAACAGCUUGCCAACCUAA                         (17bis)
TTAGGTTGGCAAGCTGTTGGAATAGCTT                         (17ter)
UUAGGUUGGCAAGCUGUUGGAAUAGCUU                         (17quater)
```

Group 18 :

```
TGGTGGGCCTTTACCCCGCCAACCAGCT                         (18)
UGGUGGGCCUUUACCCCGCCAACCAGCU                         (18bis)
AGCTGGTTGGCGGGGTAAAGGCCCACCA                         (18ter)
```


```
AGCUGGUUGGCGGGGUAAAGGCCCACCA                         (18quater)
```

in which the letters mean the following nucleotides :
A :    adenylic residue,

5

C : cytidylic residue,
G : guanidylic residue,
T : thymidylic residue,
U : uracylic residue,

provided that the probe does not consist of the following sequence:

TCA TCG GCC GCC GAT ATT GGC

- or a variant sequence which differs from any of the preceding sequences (4) to (18)
  . either by addition to or removal from any of their respective extremities of one or several nucleotides,
  . or changing within any of said sequences of one or more nucleotides,
  . or both,
  yet provided that in any of the above circumstances said probe still hybridizes with the same RNA or DNA target as the corresponding unmodified sequence.

Under the expression "target" is meant a sequence complementary to any of the sequences of groups 4 to 18 as herein before defined. This in case where the probe of the invention would comprise nucleic acid elongations on either side or both of said above defined sequences-- e.g. nucleic acid fragments of a cloning vector or linker fragments resulting from the cleavage of said probe out of said cloning vector-- it is understood that such elongations should be selected such as to avoid the possibility that they could themselves hybridize with any other corresponding complementary nucleic acid sequence outside of the above target in a DNA of any microorganism likely to be tested by the process of this invention as later defined. Such hybridization would be of a parasitical nature and reduce the specificity of the probe.

Preferred probes consist of nucleic acid fragments formed of any of the sequences under (4) to (18), said fragments containing from 10 to the maximum number of nucleotides of the relevant nucleic acid sequence.

It is understood that in the above nucleotide sequences (and in the other ones referred to hereafter), the left end of the formulae always corresponds to a 5' extremity and the right end to a 3' extremity of the sequence concerned.

When reference is further made therein to a "probe of group "x"" - with "x" from 1 to 18 - it should be understood that such probe has a sequence included in one of the nucleic acids belonging to that group as defined above or further defined hereafter.

It is also understood that the word "nucleotide" as used herein refers indistinctly to ribonucleotides and deoxyribonucleotides and modified nucleotides such as inosine unless otherwise specified. The expression "nucleotides" also encompasses those which further comprise modification groups, e.g. chemical modification groups which do not affect their hybridization capabilities. Such modification groups aim, for instance, at facilitating their coupling, either directly or indirectly, with suitable markers or labels for the subsequent detection of the probes so marked or labeled, particularly in their hybridization products with the relevant rRNA or DNA strand, e.g. that or those initially contained in a biological sample together with other DNA(s) and/or RNA(s).

For instance, such modification groups are recognizable by antibodies which, in turn, can be recognized specifically by other antibodies carrying a suitable enzymatic or fluorescent or chemiluminescent label. Possible labeling procedures will further be examplified later herein.

The invention also relates to probes having any of the sequences defined above and in which some nucleotides are different, provided that the different nucleotide(s) do(es) not alter the specificity of the probes defined above. Some probes may consist of one of the nucleic acids belonging to any of the groups 4 to 10 which are set forth above or of part thereof, said probes however including nucleotidic elongation on either sides thereof to the extent that such elongations do not alter the specificity of said probes with the genetic material of Neisseria as discussed hereafter.

Most of these probes, can be caused to hybridize with a large number if not all Neisseria. However the probes of group 4, 5, 9, 11, 13 and 18 are capable of hybridizing more selectively with corresponding regions of the RNAs or DNAs of Neisseria gonorrhoeae, and in some instances under controlled hybridization conditions - not with other Neisseria.

This applies particularly to subgroups selected from the probes of group 4, which subgroups are hereafter generally designated as groups 1 to 3, each of said groups comprising more specific probes (still normally containing at least ten nucleotides) whose probes contain :

- either a sequence belonging to a nucleic acid selected from the following groups of nucleic acids and which includes itself of from 10 to the maximum number of nucleotides of the selected nucleic acid

6

**Group 1 :**

TCGGCCGCCGATATTGGCAACAGCCTT (1)

UCGGCCGCCGAUAUUGGCAACAGCCUU (1bis)

AAGGCTGTTGCCAATATCGGCGGCCGA (1ter)

AAGGCUGUUGCCAAUAUCGGCGGCCGA (1quater)

**Group 2 :**

TCGGCCGCCGATATTGGCAACAGCCTTTTCTTCCCTG (2)

UCGGCCGCCGAUAUUGGCAACAGCCUUUUCUUCCCUG (2bis)

CAGGGAAGAAAAGGCTGTTGCCAATATCGGCGGCCGA (2ter)

CAGGGAAGAAAAGGCUGUUGCCAAUAUCGGCGGCCGA (2quater)

**Group 3 :**

GGTACCGTCATCGGCCGCCGATATTGGCAACAGCCTTTTCTTCCCTG (3)

GGUACCGUCAUCGGCCGCCGAUAUUGGCAACAGCCUUUUCUUCCCUG (3bis)

CAGGGAAGAAAAGGCTGTTGCCAATATCGGCGGCCGATGACGGTACC (3ter)

CAGGGAAGAAAAGGCUGUUGCCAAUAUCGGCGGCCGAUGACGGUACC (3quater)

provided that the probe does not consist of the following sequence:
TCA TCG GCC GCC GAT ATT GGC

- or a variant sequence which differs from any of the preceding sequences (1) to (3)
  . either by addition to or removal from any of their respective extremities of one or several nucleotides,
  . or changing within any of said sequences of one or more nucleotides,
  . or both,

yet provided that in any of the above circumstances said probe still hybridizes with the same RNA or DNA target as the corresponding unmodified sequence.

The invention thus provides for probes which are either replicas (those designated by numbers followed by "ter" or "quater") in terms of nucleotide sequence of sequences contained in the rRNAs of most Neisseria, with occasionnally a few insignificant differences in nucleotide sequences or formed of sequences, those designated by bare numbers or by numbers followed by "bis", complementary to sequences included in the natural rRNAs of Neisseria.

More particularly, it should be appreciated that the target sequences in the rRNAs concerned consist in any of the following successive sequences present in most, if not all, Neisseria, subject to possible insignificant natural differences from one Neisseria to another, whereby such natural differences are not likely to affect the hybridization specificity of the probes of this invention with such targets :

```
(5') AAGGCUGUUGCCAAUAUCGGCGGCCGA (3')          (1quater)

(5') CAGGGAAGAAAAGGCUGUUGCÇAAUAUCGGCGGCCGA (3') (2quater)

(5') CAGGGAAGAAAAGGCUGUUGCCAAUAUCGGCGGCCGAUGACGGUACC (3')
                                                (3quater)

GGACUUUUGUCAGGGAAGAAAAGGCUGUUGCCAAUAUCGGCGGCCGAUGACGGUACC
(5')                                               (3')
                                                (4quater)

(5') UUAGCUGUUGGGCAACUUGAUUGCUUGGUAGCGU (3')       (5quater)

GUGGGGGAUACCAGAAGUAGGUAGGGUAACCGCAAGGAGUCCGCUUACCACGGUAU
(5')                                               (3')
                                                (6quater)

(5') ACCUAGGUCCGGCGGAAAUCGGACUGA   (3')        (7quater)

(5') ACCUAGGUCCGGUCGGAAAUCGGACUGA (3')         (8quater)

(5') GCAGCACAGGGAAGCUUGCUUCUCGGGUGGCG (3')      (9quater)

(5') UAUGCCCUCUAAGGUUAAGGACUUGCUCCGUAAGCCCCG(3')(10quater)

(5') GCGGGGGAGCUGGCAAUAAAGCUAUGAUUCCGC (3')     (11quater)

(5') GUUCUGAACUGGGUGACUCGAGUGUGUC (3')          (12quater)

(5') GCAUACGUCUUGAGAGGGAAAGCA (3')              (13quater)

(5') UGUACGUAAUAACUGUCGAGA (3')                 (14quater)

(5') UCUCAACAGCGGUACUAAGCGUACGAAA (3')          (15quater)

(5') UGUCUACGGACACGAAGCAACCGAUACCAC (3')        (16quater)

(5') UUAGGUUGGCAAGCUGUUGGAAUAGCUU (3')          (17quater)

(5') AGCUGGUUGGCGGGGUAAAGGCCCACCA (3')          (18quater)
```

The differences in hybridization capability of the nucleotide sequences of the different probes (or of (8quater), (9quater), (10quater) the related rRNA sequences) from one probe to another are of a sufficiently reduced magnitude as to ensure the selectivity of the sequences of groups (4)-(18) as regards the detection and identification of Neisseria (such as N. lactamica, N. mucosa, N. subflava, N. flavescens, N. elongata, etc...) in a biological sample suspected to contain same, yet to distinguish them from other taxa. However, within group (4) (and all the more so in groups 3, 2 and 1) and also in group 5, 9, 11, 13 and 18, these differences are becoming of sufficient magnitude to enable Neisseria gonorrhoeae strains to be differentiated from other Neisseria strains in some instances even under less stringent hybridization conditions (which will be referred to herein more accurately later), and in the presence of other DNAs or RNAs or both present in the sample studied, e.g. a sample originating from mammals, particularly humans. It will be mentioned later that the hybridizations may be carried out e.g. using dot-spot proceeding, if need be upon appropriately adjusting the hybridization conditions and subsequent washing conditions of the hybrids formed.

It will be appreciated that the above mentioned specificities, be it for the whole Neisseria taxon or for the Neisseria gonorrhoeae subtaxon tend to become lost when the number of nucleotides in the probes used fall below 10. Nevertheless and particularly in groups 1, 2, 3, 4, 5, 9, 11, 13 and 18 best results are obtained when the number of nucleotides does not grow beyond large numbers (bearing in mind a maximum number which is fixed in groups 4 to 18 by the maximum lengths of the sequences concerned). For the selective identification of Neisseria gonorrhoeae versus other Neisseria most preferred probes have sequences comprising from 15 to 27 nucleotides, said sequences being fully included in any of the sequences of group 1. Selectivity for N. gonorrhoeae is still retained with probes whose sequences overlap regions of corresponding nucleic acids of groups 1 and either 2 or 3 (or are specifically contained in sequences belonging to the nucleic acids of group 2 or 3 only), (or are specifically contained in sequences contained in the nucleic acids of group 3 only), yet subject to adjusting the hybridization and washing conditions more stringently.

The preferred probes are those which are complementary to the natural rRNAs concerned for they hybridize both with said RNAs and the corresponding DNA.

Yet, those which have sequences included in said rRNAs, therefore which will only hybridize with the relevant natural DNAs and therefore are less sensitive as the preceding ones, are also part of the invention.

Other groups of probes of the invention are constituted by those which are specific for Neisseria strains yet considered globally (as specified above) consist of groups 6, 7, 8, 10, 12, 14, 16 and 17 as above defined, if need be under appropriate adjustment of the hybridizing and washing conditions of the hybrid possibly formed.

The probes according to the invention can be formed by cloning of recombinant plasmids containing inserts including the corresponding nucleotide sequences, if need be cleaving the latter out from the cloned plasmids upon using the adequate nucleases and recovering them, e.g. by fractionation according to molecular weights.

The probes according to the invention can also be synthetized chemically, for instance by the conventional phospho-triester method.

Among the variants defined hereabove are included hybridization probes for detecting one or more Neisseria strains which target one of the sequences defined hereunder or the corresponding complementary sequence, when the hybridization medium or the wash medium or both as appropriate are the following ones :

hybridization medium : containing about 3xSSC,

(SSC = 0.15M NaCl, 0.015M sodium citrate, pH 7.0) about 25mM of phosphate buffer pH 7.1, 20% deionized formamide 0.02% ficoll, 0.02% bovine serum albumin, 0.02% polyvinylpyrrolidone, and about 0.1 mg/ml sheared, denatured salmon sperm DNA,

wash medium : containing about 3XSSC, 25 mM phosphate buffer pH 7.1, and 20% deionized formamide, wherein said target sequences and the corresponding relevant hybridization temperatures (HT) and wash temperatures (WT) respectively are as follows :

AAGGCUGUUGCCAAUAUCGGCGGCCGA

HT and/or WT : about 55°C

CAGGGAAGAAAAGGCUGUUGCCAAUAUCGGCGGCCGA

HT and/or WT : about 60°C

CAGGGAAGAAAAGGCUGUUGCCAAUAUCGGCGGCCGAUGACGGUACC

HT and/or WT : about 60°C

GGACUUUUGUCAGGGAAGAAAAGGCUGUUGCCAAUAUCGGCGGCCGAUGACGGUACC

HT and/or WT : about 65°C

UUAGCUGUUGGGCAACUUGAUUGCUUGGUAGCGU

HT and/or WT : about 55°C to about 60°C

GUGGGGGAUACCAGAAGUAGGUAGGGUAACCGCAAGGAGUCCGCUUACCACGGUAU

HT and/or WT : about 65°C to about 70°C

ACCUAGGUCCGGCGGAAAUCGGACUGA

HT and/or WT : about 55°C

ACCUAGGUCCGGUCGGAAAUCGGACUGA

HT and/or WT : about 55°C to about 60°C

GCAGCACAGGGAAGCUUGCUUCUCGGGUGGCG

HT and/or WT : about 55°C to about 60°C

UAUGCCCUCUAAGGUUAAGGACUUGCUCCGUAAGCCCCG

HT and/or WT : about 60°C to about 65°C,

GCGGGGGAGCUGGCAAUAAAGCUAUGAUUCCGC

HT and/or WT : about 55°C,

GUUCUGAACUGGGUGACUCGAGUGUGUC

HT and/or WT : about 55°C to about 60°C,

GCAUACGUCUUGAGAGGGAAAGCA

HT and/or WT : about 45°C,

UGUACGUAAUAACUGUCGAGA

HT and/or WT : about 40°C to about 45°C,

UCUCAACAGCGGUACUAAGCGUACGAAA

HT and/or WT : about 50°C to about 55°C,

UGUCUACGGACACGAAGCAACCGAUACCAC

HT and/or WT : about 50°C to about 60°C,

```
UUAGGUUGGCAAGCUGUUGGAAUAGCUU
```
**HT and/or WT : about 50˚C to about 55˚C,**
```
AGCUGGUUGGCGGGGUAAAGGCCCACCA
```
**HT and/or WT : about 45˚C.**

It should be emphasized that the indicated temperatures are valid only under the conditions mentioned above. Other hybridization or wash media can be used as well. However, when modifications are introduced, be it either in the probes or in the media, the temperatures at which the probes can be used to obtain the required specificity, should be changed according to known relationships, such as those described in the following reference : B.D. Hames and S.J. Higgins, (eds.). Nucleic acid hybridization. A practical approach, IRL Press, Oxford, U.K., 1985.

Among the variants defined hereabove are included hybridization probes for detecting one or more Neisseria gonorrhoeae strains which target one of the sequences defined hereunder or the corresponding complementary sequence, when the hybridization medium or the wash medium or both as appropriate are the following ones :

hybridization medium : containing about 3xSSC, (SSC = 0.15M NaCl, 0.015M sodium citrate, pH 7.0) about 25mM of phosphate buffer pH 7.1, 20% deionized formamide 0.02% ficoll, 0.02% bovine serum albumin, 0.02% polyvinylpyrrolidone, and about 0.1 mg/ml sheared, denatured salmon sperm DNA,

wash medium : containing about 3XSSC, 25 mM phosphate buffer pH 7.1, and 20% deionized formamide, wherein said target sequences and the corresponding relevant hybridization temperatures (HT) and wash temperatures (WT) respectively are as follows :

```
AAGGCUGUUGCCAAUAUCGGCGGCCGA
```
**HT and/or WT : about 50˚C to about 65˚C**

```
CAGGGAAGAAAAGGCUGUUGCCAAUAUCGGCGGCCGA
```
**HT and/or WT : about 60˚C to about 70˚C**
```
CAGGGAAGAAAAGGCUGUUGCCAAUAUCGGCGGCCGAUGACGGUACC
```
**HT and/or WT : about 65˚C to about 70˚C**
```
GGACUUUUGUCAGGGAAGAAAAGGCUGUUGCAAUAUCGGCGGCCGAUGACGGUACC
```
**HT and/or WT : about 70˚C to about 75˚C**
```
UUAGCUGUUGGGCAACUUGAUUGCUUGGUAGCGU
```
**HT and/or WT : about 65˚C**
```
GCAGCACAGGGAAGCUUGCUUCUCGGGUGGCG
```
**HT and/or WT : about 65˚C**
```
GCGGGGGAGCUGGCAAUAAAGCUAUGAUUCCGC
```
**HT and/or WT : about 65˚C**
```
GCAUACGUCUUGAGAGGGAAAGCA
```
**HT and/or WT : about 50˚C to about 55˚C**
```
AGCUGGUUGGCGGGGUAAAGGCCCACCA
```
**HT and/or WT : about 60˚C.**

Preferred probes of the invention for detecting Neisseria gonorrhoeae strains are those of groups (1) to (4), provided that the probe does not consist of the following sequence:

TCA TCG GCC GCC GAT ATT GGC

The invention also relates to probes of the above mentioned sequences which can discriminate between organisms with DNA:DNA hybridization homology values between about 55% to about 75%.

In view of the evolution of rRNA molecules, it seems reasonable that rRNA derived hybridization probes, which allow discrimination between highly related taxa (more than 55 % DNA homology) other than those found within the Neisseria group (e.g. in Bordetella), can be constructed also.

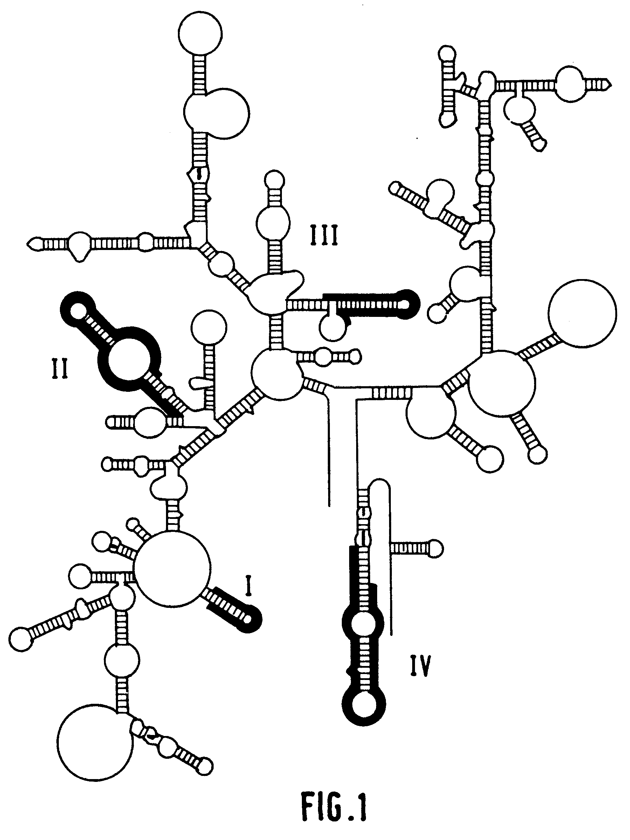

It is conceivable that these highly specific probes can be obtained from the sequences found in the same regions of the rRNA molecules than those in which specific sequences for Neisseria gonorrhoeae were found (e.g. regions I, II and III in Fig. 1, in the case of 16S rRNA). These regions can be easily located after proper alignment of the total or partial rRNA sequence of the organism concerned with the rRNA sequence of E. coli. These regions will correspond to the regions found between nucleotides :

(i) 69 to 99 for region I

(ii) 432 to 488 for region II

(iii) 825 to 858 for region III

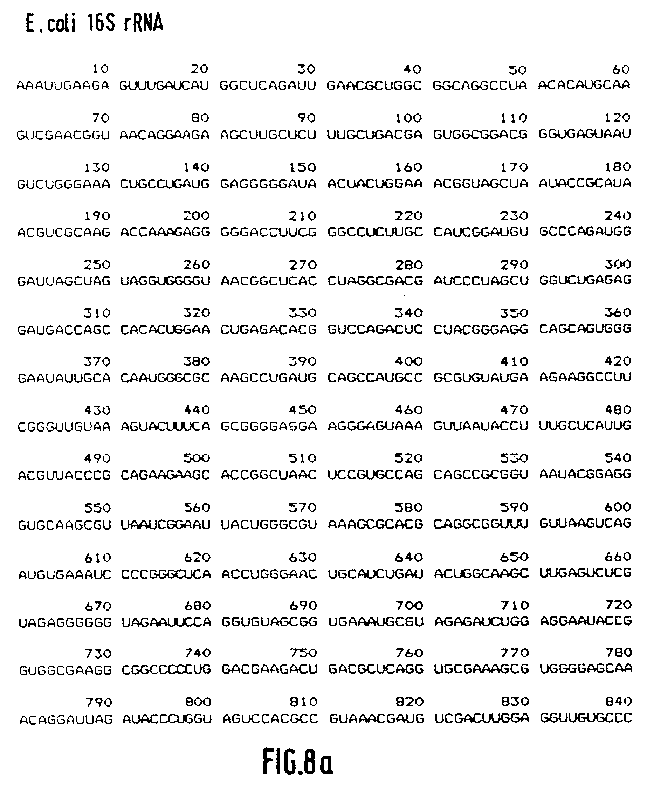

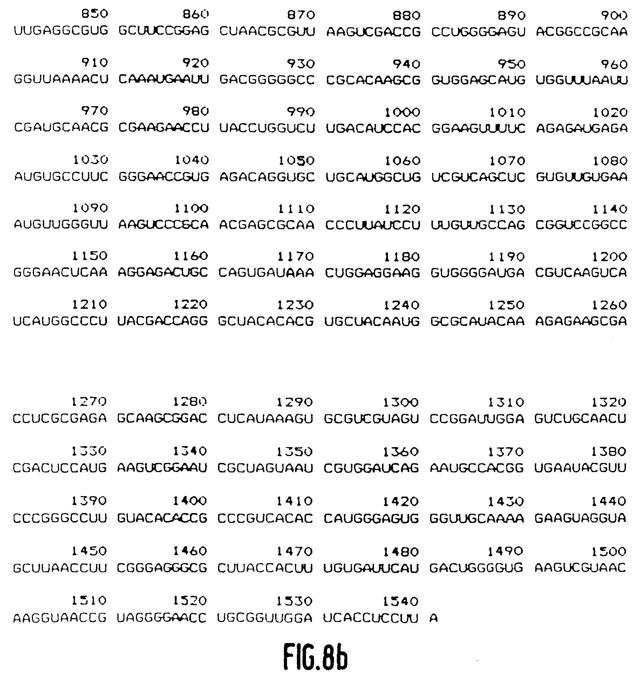

in the 16S rRNA molecule of E. coli, in which the numbering refers to the 16S rRNA sequence of E. coli shown in Fig. 8, and between nucleotides : 77 to 109

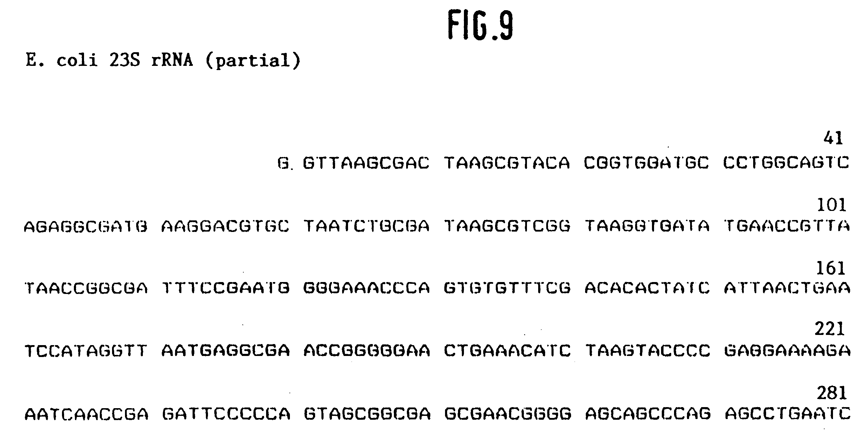

in the 23S rRNA molecule of E. coli, in which the numbering refers to the partial 23S rRNA sequence of E. coli shown in Fig. 9.

However it should be emphasized that :

(i) highly specific probes can be constructed from other regions as well, and

(ii) some of these regions can be absent in the rRNA molecules of certain taxa (due to mutations during evolution).

The invention also relates to a process for detecting Neisseria strains in a biological sample, wherein said process comprises contacting said biological sample in which the nucleic acids (DNAs and RNAs) have been made accessible to hybridization, if need be under suitable denaturation conditions, with a probe of the invention under conditions enabling hybridization between the probe and complementary nucleic acids of the Neisseria strains, which may be present in the sample, and detecting the hybrids possibly formed.

The process relates to the detection of Neisseria strains being directly in the sample of after the strain has been cultured.

The detection of a hybrid can be interpreted as meaning that a Neisseria infection was present in the biological sample, when any of the probes of groups 1 to 18 is being used, and even more specifically that a Neisseria gonorrhoeae infection was present when the probe used had a sequence belonging to a nucleic acid of groups 4, 5, 9, 11, 13 or 18, possibly under suitable hybridizing conditions, and even more so when the probe had a sequence belonging to a sequence of group 3, group 2 or even more preferably group 1.

According to an advantageous embodiment of the invention, in the process for detecting Neisseria strains, the probes used are the ones hybridizing both with DNA globally and RNA of the Neisseria strains which may be present in the biological sample.

The hybridization conditions can be monitored relying upon several parameters, e.g. hybridization temperature, the nature and concentration of the components of the media, and the temperature under which the hybrids formed are washed.

The hybridization temperature is limited in upper value, according to the probe (its nucleic acid composition, kind and length) and the maximum hybridization temperature of the probes described herein is about 55°C to 75°C. At higher temperatures duplexing competes with the dissociation (or denaturation) of the hybrid formed between the probe and the target.

The hybridization temperature is preferably comprised from about 45°C to about 70°C, particularly from about 45°C to about 65°C.

A preferred hybridization medium contains about 3xSSC, (SSC = 0,15 M NaCl, 0,015 M sodium citrate, pH 7.0) about 25mM of phosphate buffer pH 7.1, and 20% deionized formamide, 0.02% ficoll, 0.02% bovine serum albumin, 0.02% polyvinylpyrrolidone, and about 0.1 mg/ml sheared, denatured salmon sperm DNA.

The washing temperature is comprised in the range from about 50°C to about 75°C.

The process for detecting Neisseria strains generally, according to the invention can be carried out by suitably adjusting the hybridization temperature to a value at which hybridization is specific, and in such a case washing under more stringent conditions is not necessary.

According to another embodiment of the process of the invention, the hybridization temperature needs not necessarily be adjusted to the value at which hybridization is specific and in particular can be lower than the temperature at which hybridization is specific, provided washing is carried out at a temperature

EP 0 337 896 B1

corresponding to the value at which hybridization is specific.

In a process embodiment for detecting Neisseria strains generally (and for distinguishing them from other bacterial taxa) with a probe of group 6, the hybridization temperature is suitably adjusted to range of about 65°C and/or the wash temperature to range from about 65°C to about 70°C, the media being those above defined.

In another process embodiment for detecting Neisseria strains generally the probe used is anyone of group 7 above defined, the hybridization temperature is suitably adjusted to range of about 55°C and/or the wash temperature to range of about 55°C.

In a further process embodiment for detecting Neisseria strains generally, the probe used is anyone of group 1 above defined.

The hybridization temperature is suitably adjusted to range of about 55°C, preferably of about 53°C, and/or the wash temperature to range of about 55°C, preferably of about 53°C, the hybridization medium being the one above defined.

In a further process embodiment for detecting Neisseria strains generally, the probe used is anyone of group 2 above defined.

The hybridization temperature is suitably adjusted to range of about 60°C and/or the wash temperature to range of about 60°C, the hybridization medium being the one above defined.

In a further process embodiment for detecting Neisseria strains generally, the probe used is anyone of group 3 above defined.

The hybridization temperature is suitably adjusted to range of about 60°C and/or the wash temperature to range of about 60°C, the hybridization medium being the one above defined.

In a further process embodiment for detecting Neisseria strains generally, the probe used is anyone of group 4 above defined.

The hybridization temperature is suitably adjusted to range of about 65°C and/or the wash temperature to range of about 65°C, the hybridization medium being the one above defined.

In a further process embodiment for detecting Neisseria strains generally, the probe used is anyone of group 5 above defined.

The hybridization temperature is suitably adjusted to range of about 55°C and/or the wash temperature to range of about 55°C, the hybridization medium being the one above defined.

In a further process embodiment for detecting Neisseria strains generally, the probe used is anyone of group 8 above defined.

The hybridization temperature is suitably adjusted to range of about 55°C and/or the wash temperature to range of about 55°C to about 60°C, the hybridization medium being the one above defined.

In a further process embodiment for detecting Neisseria strains generally, the probe used is anyone of group 9 above defined.

The hybridization temperature is suitably adjusted to range of about 60°C and/or the wash temperature to range of about 60°C, the hybridization medium being the one above defined.

In a further process embodiment for detecting Neisseria strains generally, the probe used is anyone of group 10 above defined.

The hybridization temperature is suitably adjusted to range of about 55°C and/or the wash temperature to range of about 55°C to about 60°C, the hybridization medium being the one above defined.

In a further process embodiment for detecting Neisseria strains generally, the probe used is anyone of group 11 above defined.

The hybridization temperature is suitably adjusted to range of about 55°C and/or the wash temperature to range of about 55°C, the hybridization medium being the one above defined.

In a further process embodiment for detecting Neisseria strains generally, the probe used is anyone of group 12 above defined.

The hybridization temperature is suitably adjusted to range of about 55°C to about 60°C and/or the wash temperature to range of about 55°C to about 60°C, the hybridization medium being the one above defined.

In a further process embodiment for detecting Neisseria strains generally, the probe used is anyone of group 13 above defined.

The hybridization temperature is suitably adjusted to range of about 45°C and/or the wash temperature to range of about 45°C, the hybridization medium being the one above defined.

In a further process embodiment for detecting Neisseria strains generally, the probe used is anyone of group 14 above defined.

The hybridization temperature is suitably adjusted to range of about 40°C to about 45°C and/or the wash temperature to range of about 40°C to about 45°C, the hybridization medium being the one above

13

defined.

In a further process embodiment for detecting Neisseria strains generally, the probe used is anyone of group 15 above defined.

The hybridization temperature is suitably adjusted to range of about 50°C to about 55°C and/or the wash temperature to range of about 50°C to about 55°C, the hybridization medium being the one above defined.

In a further process embodiment for detecting Neisseria strains generally, the probe used is anyone of group 16 above defined.

The hybridization temperature is suitably adjusted to range of about 50°C to about 60°C and/or the wash temperature to range of about 50°C to about 60°C, the hybridization medium being the one above defined.

In a further process embodiment for detecting Neisseria strains generally, the probe used is anyone of group 17 above defined.

The hybridization temperature is suitably adjusted to range of about 50°C to about 55°C and/or the wash temperature to range of about 50°C to about 55°C, the hybridization medium being the one above defined.

In a further process embodiment for detecting Neisseria strains generally, the probe used is anyone of group 18 above defined.

The hybridization temperature is suitably adjusted to range of about 45°C and/or the wash temperature to range of about 45°C, the hybridization medium being the one above defined.

The invention further relates also to a process for detecting Neisseria gonorrhoeae strains from other Neisseria strains in a biological sample, and advantageously for differentiating Neisseria gonorrhoeae strains from highly related taxa such as N. meningitidis, wherein said process comprises contacting said biological sample, in which the nucleic acids (DNAs and RNAs) have been made accessible to hybridization, if need be, under suitable denaturation conditions, with a probe of the invention specific for Neisseria gonorrhoeae strains and selected from groups 1 to 5, 9, 11, 13 and 18, whenever required, under hybridization and washing conditions adjusted such as to ensure specific hybridization with complementary nucleic acids of the Neisseria gonorrhoeae strains, which may be present in the sample, yet not with complementary DNA or RNA of other Neisseria species, and detecting the hybrids possibly formed.

The other Neisseria strains from which the N. gonorrhoeae strains can be specifically differentiated are for instance N. lactamica, N. mucosa, N. subflava, N. flavescens, and N. elongata.

In this respect, the hybridization temperature is preferably comprised from about 50°C to about 75°C.

A preferred hybridization medium contains about 3xSSC, (SSC = 0.15M NaCl, 0.015M sodium citrate, pH 7.0) about 25mM of phosphate buffer pH 7.1, and 20% deionized formamide, 0.02% ficoll, 0.02% bovine serum albumin, 0.02% polyvinylpyrrolidone, and about 0.1 mg/ml sheared, denatured salmon sperm DNA, and a preferred wash medium contains about 3XSSC, 25 mM phosphate buffer pH 7.1, and 20% deionized formamide.

The wash temperature is comprised from about 50°C to about 70°C.

In a process embodiment for detecting Neisseria gonorrhoeae, the probe used belongs to group 2 above defined, the hybridization temperature is suitably adjusted to range about 60°C, and/or the wash temperature to range from about 65°C to about 70°C, preferably about 65°C, the medium being the one above defined.

In a process embodiment for detecting Neisseria gonorrhoeae, the probe used belongs to group 3 above defined, the hybridization temperature is suitably adjusted to range of about 60°C, and/or the wash temperature to range from about 65°C to about 70°C, preferably about 65°C, the medium being the one above defined.

In another process embodiment for detecting Neisseria gonorrhoeae, the probe used belongs to group 4 above defined, the hybridization temperature is suitably adjusted to range of about 65°C, and/or the wash temperature for range from about 70°C to about 75°C, preferably about 70°C.

In a further preferred embodiment for detecting Neisseria gonorrhoeae, the probe used belongs to group 5 above defined, the hybridization temperature is suitably adjusted to range of about 60°C, and/or the wash temperature to range of about 65°C.

In a further preferred embodiment for detecting Neisseria gonorrhoeae, the probe used belongs to group 9 above defined, the hybridization temperature is suitably adjusted to range of about 60°C to about 65°C, and the wash temperature to range of about 65°C.

In another process embodiment for detecting Neisseria gonorrhoeae, the probe used belong to group 11, the hybridization temperature and/or the wash temperature is suitably adjusted to about 65°C.

14

In a process embodiment for detecting Neisseria gonorrhoeae, the probe used belongs to group 13 above defined, the hybridization temperature is suitably adjusted to range about 50°C to about 55°C, and/or the wash temperature to range from about 50°C to about 55°C, the medium being the one above defined.

In a process embodiment for detecting Neisseria gonorrhoeae, the probe used belongs to group 18 above defined, the hybridization temperature is suitably adjusted to range about 60°C, and/or the wash temperature to range about 60°C, the medium being the one above defined.

According to another particularly preferred process embodiment for detecting Neisseria gonorrhoeae, the probe used belongs to group 1 above defined, the hybridization temperature is suitably adjusted to range of about 55°C, preferably of about 53°C, and/or the wash temperature to range from about 55°C to about 65°C, preferably from about 53°C to about 65°C, more preferably of about 53°C.

In a preferred process of the invention for detecting N. gonorrhoeae, the probe used belongs to anyone of groups (1) to (4), provided that the probe does not consist of the following sequence:
TCA TCG GCC GCC GAT ATT GGC

The invention also relates to a kit for the detection in vitro of a large number, preferably all Neisseria strains in a biological sample containing :
- at least one probe selected among any of those which have been defined above ;
- the buffer or components necessary for producing the buffer enabling an hybridization reaction between these probes and the DNAs and/or RNAs of a strain of Neisseria to be carried out,
- when appropriate means for detecting the hybrids resulting from the preceding hybridization.

The invention further relates to a kit for detecting specifically N. gonorrhoeae strains containing :
- at least one probe selected among any of those that are specific for N. gonorrhoeae as above defined, e.g. a probe of groups (2), (3), (4), (5), (9), (11), (13) or (18), preferably a probe of group (1) ;
- the buffer or components necessary for producing the buffer enabling an hybridization reaction between these probes and only the DNAs and/or RNAs of a strain of Neisseria gonorrhoeae to be carried out.

In a preferred kit for detecting N. gonorrhoeae strains, the probe belongs to groups (1) to (4), provided that the probe does not consist of the following sequence:
TCA TCG GCC GCC GAT ATT GGC

The invention relates to a kit for detecting a large number, preferably all Neisseria strains and specifically Neisseria gonorrhoeae strains containing :
- at least one probe selected among any of those that have been above defined, e.g. a probe of groups (1) to (18), more preferably a probe of groups (6), (7), (8), (10), (12), (14), (15), (16) or (17) ;
- the buffer ready for use or components in appropriate proportions necessary for producing the buffer enabling an hybridization reaction between these probes and the DNAs and/or RNAs of a large number, preferably all strains of Neisseria to be carried out,
- at least one probe selected among any of those that are specific for Neisseria gonorrhoeae as above defined, e.g. a probe of groups (2), (3), (4), (5), (9), (11), (13) or (18) or more preferably a probe of group (1) ;
- the buffer ready for use or components necessary in appropriate proportions for producing the buffer enabling an hybridization reaction between these probes and only the DNAs and/or RNAs of a strain of Neisseria gonorrhoeae to be carried out.

In a preferred kit for detecting Neisseria strains and specifically N. gonorrhoeae strains, the probe used for detecting N. gonorrhoeae strains belongs to groups (1) to (4) provided that the probe does not consist of the following sequence:
TCA TCG GCC GCC GAT ATT GGC


CONDITIONS OF THE USE OF PROBES :


The probes of the invention are advantageously labeled. Any conventional label can be used. The probes can be labeled by means of radioactive tracers such as $^{32}$P, $^{35}$S, $^{125}$I, $^{3}$H and $^{14}$C.

The radioactive labeling can be carried out according to any conventional method such as terminal labeling at the 3' or 5' position with the use of a radio-labeled nucleotide, a polynucleotide kinase (with or without dephosphorylation by a phosphatase) or a ligase (according to the extremity to be labeled). One of the probes of the invention can be the matrix for the synthesis of a chain consisting of several radioactive nucleotides or of several radioactive and non radioactive nucleotides. The probes of the invention can also be prepared by a chemical synthesis using one or several radioactive nucleotides. Another method for radioactive labeling is a chemical iodination of the probes of the invention which leads to the binding of

several $^{125}$I atoms on the probes.

If one of the probes of the invention is made radioactive to be used for hybridization with a non radioactive RNA or DNA, the method of detecting hybridization will depend on the radioactive tracer used. Generally, autoradiography, liquid scintillation, gamma counting or any other conventional method enabling one to detect an ionizing ray issued by the radioactive tracer can be used.

Non radioactive labeling can also be used by associating the probes of the invention with residues having : immunological properties (e.g. antigen or hapten), a specific affinity for some reagents (e.g. ligand), properties providing a detectable enzymatic reaction (e.g. enzyme, co-enzyme, enzyme substrate or substrate taking part in an enzymatic reaction), or physical properties such as fluorescence or emission or absorbtion of light at any wave length. Antibodies which specifically detect the hybrids formed by the probe and the target can also be used.

A non-radioactive label can be provided when chemically synthesising a probe of the invention, the adenosine, guanosine, cytidine, thymidine and uracyl residues, thereof being liable to be coupled to other chemical residues enabling the detection of the probe or the hybrids formed between the probe and a complementary DNA or RNA fragment. However, the nucleotide sequence of the probe when modified by coupling one or more nucleotides to other chemical residues, would be the same as the nucleotidic sequence of one of the probes of the invention.

The invention also relates to processes for detecting by hybridization RNA and/or DNA with the probes of the invention, which have been labeled and can be detected as described above. In this regard, conventional methods of hybridization can be used.

For detecting cells coming from or being themselves living organisms, the RNA and/or DNA of these cells if need be, is made accessible by partial or total lysis of the cells, using chemical or physical processes, and contacted with one or several probes of the invention which can be detected. This contact can be carried out on an appropriate support such as a nitrocellulose, cellulose, or nylon filter in a liquid medium or in solution. This contact can take place under sub-optimal, optimal conditions or under restrictive conditions (i.e. conditions enabling hybrid formation only if the sequences are perfectly homologous on a length of molecule). Such conditions include temperature, concentration of reactants, the presence of substances lowering the optimal temperature of pairing of nucleic acids (e.g. formamide, dimethylsulfoxide and urea) and the presence of substances apparently lowering the reaction volume and/or accelerating hybrid formation (e.g. dextran sulfate, polyethyleneglycol or phenol).

The elimination of probe of the invention which has not hybridized can be carried out by washing with a buffer solution of appropriate ionic strength and at an appropriate temperature, with or without treatment with S1 nuclease or any other enzyme digesting single strand DNA or RNA but not digesting DNA-RNA hybrids or double strand DNA.

In a liquid medium, the hybrids of the probes of the invention paired to the cellular DNA or RNA fragments can be separated from the rest of the liquid medium in different ways, e.g. by chromatography over hydroxyapatite.

Then the hybridized probes are detected by means of the label on the probe.

In order to target the Neisseria chromosomal DNA fragments carrying the genes coding for the RNA fragments from which the labeled probes of the invention derive, after treating DNA by one or several enzymes and denaturation of DNA fragments (i.e. separation of both chains), one of the probes of the invention is contacted with the DNA fragments under the conditions enabling hybridization and after the time necessary to get to the end of the hybridization, the non-hybridized fragments are separated from the hybridized fragments and the label is detected as it has been described above for the detection of the cells.

Generally speaking, the different probes of the invention can also be contained in recombinant DNA enabling their cloning, if the presence of a heterologous DNA is not a nuisance for the specificity of the probes in the encompassed uses.

More precisely, the examples hereafter relate to the preparation of the probes of the invention respectively corresponding to the sequences (1), (2), (3), (4), (5), (6), (7), (8), (9), (10), (11), (12), (13), (14), (15), (16), (17) and (18) above described and hereafter mentioned respectively as probes n° 1, n° 2, n° 3, n° 4, n° 5, n° 6, n° 7, n° 8, n° 9, n° 10, n° 11, n° 12, n° 13, n° 14, n° 15, n° 16, n° 17 and n° 18.

## MATERIAL AND METHODS

### 1) Organisms and media used :

The following strains were cultured as described by Rossau et al. (1986) : Neisseria gonorrhoeae NCTC 8375$^T$, Neisseria meningitidis NCTC 10025$^T$, Neisseria lactamica NCTC 10617$^T$, Neisseria mucosa CIP 59.51$^T$, Neisseria subflava ATCC 10555, Neisseria flavescens ATCC 13120$^T$, Neisseria elongata ssp. elongata NCTC 10660$^T$, and Moraxella (Branhamella) catarrhalis NCTC4103. Eight randomly chosen N. gonorrhoeae strains and nine N. meningitidis strains (from different serotypes) were cultured overnight on blood agar plates at the Institute of Tropical Medicine, Antwerp, Belgium. The identity of the strains was checked by conventional methods. Purified genomic DNA from the remaining strains was provided by J. De Ley (Lab. Microbiology, State University Gent, Belgium).

2) DNA preparations :

High-molecular weight genomic DNA was prepared essentially by the method described by Marmur (1961). Plasmid DNA was isolated by the method described by Kahn at al. (1979) and purified by CsCl-gradient centrifugation.

3) Fixation of denatured DNA on nitrocellulose membranes :

The DNA solution was heated for 10 min. at 95°C, put on ice, and adjusted to 6XSSC (SSC : 0.15M NaCl, 0.015M sodium citrate, pH 7.0). The appropriate amount of solution was applied to a BA85 membrane (Schleicher & Schuell, W.-Germany) in a dot-spot manifold. After air drying, the membrane was baked at 80°C for 2 h.

4) Construction of pNGD1 and pNGK3 :

The plasmids pNGD1 and pNGK3 contain the probes n° 3 and n° 6 as an insert respectively. They were constructed from pNG4 and pNG3 respectively, which are pTZ18R (Pharmacia, Sweden) derived recombinant plasmids which contain part of the 16S rRNA gene of Neisseria gonorrhoeae NCTC 8375$^T$. In the case of pNGD1, a 71 basepair StuI-KpnI fragment was subcloned and 25 basepairs beginning from the StuI site were subsequently removed by Exonuclease III (Stratagene, U.S.A.) and mung bean nuclease (Stratagene, U.S.A.) treatment. For the construction of pNGK3, 63 basepairs were likewise removed from the 3′ end of the insert of pNG3. The resulting plasmid was cleaved with SphI and BstXI, followed by blunting of the sticky-ends and intramolecular ligation. The restriction enzymes were purchased from Boehringer Mannheim (W.-Germany) or Bio Labs (U.S.A.) and used as recommended by the suppliers.

5) DNA sequence determination :

The inserts of pNGD1 and pNGK3 were sequenced by the dideoxy chain-termination method on supercoiled plasmid DNA as described in the GemSeq K/RT™ Sequencing System technical manual (Promega, U.S.A.).

6) Oligonucleotide synthesis and purification :

The oligonucleotides were synthetized by the phosphite-triester method on a Gene Assembler 18-5800-01 (Pharmacia, Sweden) or a Cyclone 8400 (New Brunswick, U.S.A.). The deprotected oligonucleotides were purified on a 15% polyacrylamide gel in 7M ureum. After overnight elution, they were desalted on a Sephadex® G-25 (Pharmacia, Sweden) column.

7) Probe labeling :

The synthetic oligonucleotides used as probes were labeled using T4-polynucleotide kinase (Pharmacia, Sweden) and gamma-$^{32}$P-dATP (Amersham, U.K.) (Maniatis et al., 1982).

To eliminate interference due to vector sequences during hybridization, the inserts of pNGD1 and pNGK3 were cut out using restriction enzymes and purified by agarose gel electrophoresis. The purified inserts were labeled by filling in the sticky-ends with alpha-$^{32}$P-dATP (Amersham, U.K.) using Klenow enzyme (Boehringer Mannheim, W.-Germany) (Maniatis et al., 1982). Unincorporated label was removed using a Bio-Gel® P-6DG (Bio-Rad Laboratories, U.S.A.) spin-column.

8) Hybridizations :

A general hybridization protocol was followed in all experiments except that the conditions were adapted to the nature of the probes used. Prehybridization was usually performed in plastic bags in 3xSSC, 25 mM phosphate buffer pH 7.1 (FB), 20% deionized formamide (FA), 0.02% ficoll, 0.02% bovine serum albumin, 0.02% polyvinylpyrrolidone, and 0.1mg/ml sheared, denatured salmon sperm DNA at the same temperature as the hybridization for 30 min. to 4 h. Hybridizations were performed during 1 h to overnight in the same solution to which approximately 0.5 to 1 x 10$^6$ cpm/ml $^{32}$P-probe was added. The hybridization temperature (HT) varied from experiment to experiment. Following a brief rinse in 3xSSC, 25mM FB and 20% FA at room temperature, the membranes were washed for 15 to 30 min. in 3xSSC, 25mM FB and 20% FA at the wash temperature (WT) indicated in the figures. Afterwards the membranes were rinsed in 1.5xSSC at room temperature for approximately 10 min., dried and autoradiographed.

RESULTS:

1) Probes used:

In order to select Neisseria gonorrhoeae specific probes, one of the rRNA cistrons of the type strain of N. gonorrohoeae was cloned and sequenced. Evolutionarily less-conserved regions within the cistron were identified by alignment with known sequences. Some of the regions were subcloned (probes n° 3 and 6) or chemically synthesized (probes n° 1, 2, 4, 5, 7, 8, 9, 10, 11 12, 13, 14, 15, 16, 17 and 18) and used as hybridization probes. Fig. 1 represents the allocations of the regions for which DNA-probes were constructed and used on the 16S rRNA secondary structure of Escherichia coli (Woese et al., 1983). These regions are indicated by an heavy bar and numbered by Roman numerals. Probe n° 9 was derived from region I, probes n° 1 to 4 were derived from region II, and probes n° 5 and 6 from regions III and IV respectively.

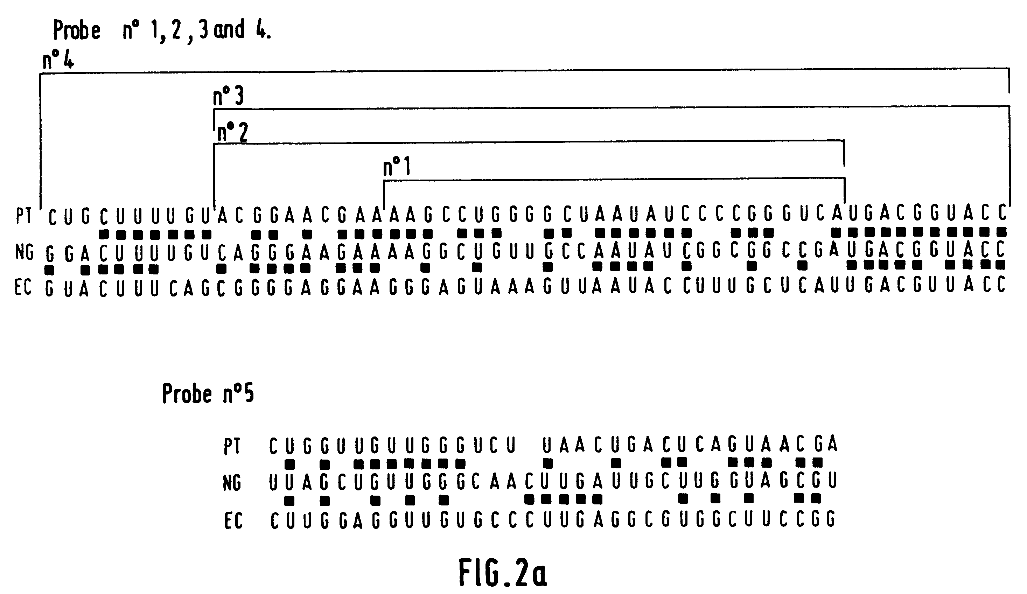

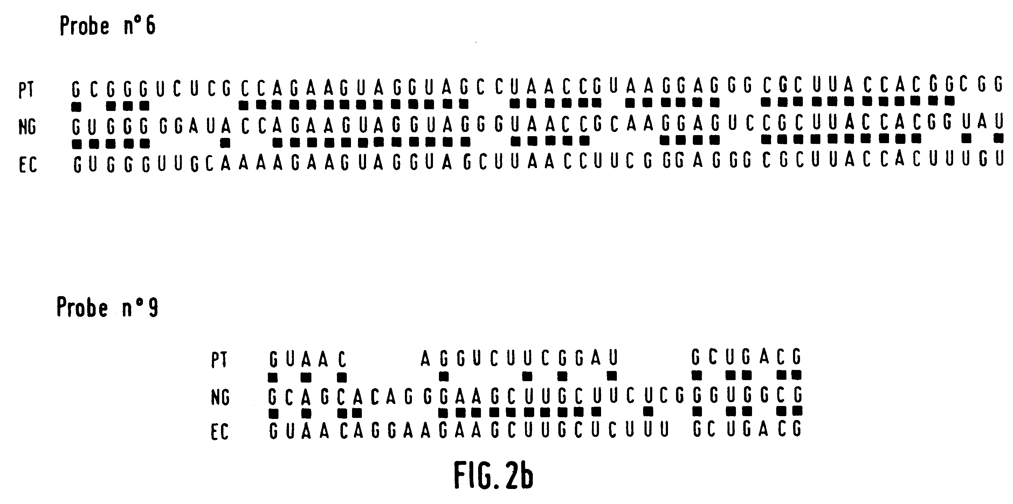

In Fig. 2 the complementary sequences of the probes according to the invention n° 1, n° 2, n° 3, n° 4, n° 5, n° 6 and n° 9 are aligned with the corresponding sequences of Pseudomonas testosteroni ATCC 11996 (Yang et al., 1985), the closest phylogenetic neighbour of Neisseria from which the 16S rRNA sequence is published and with the corresponding sequences of Escherichia coli (Brosius et al., 1978).

In Fig. 3 the complementary sequences of probes n° 7, n° 8, n° 10 and n° 11 derived from the 23S rRNA gene are aligned with the corresponding Escherichia coli sequences (Brosius et al., 1980).

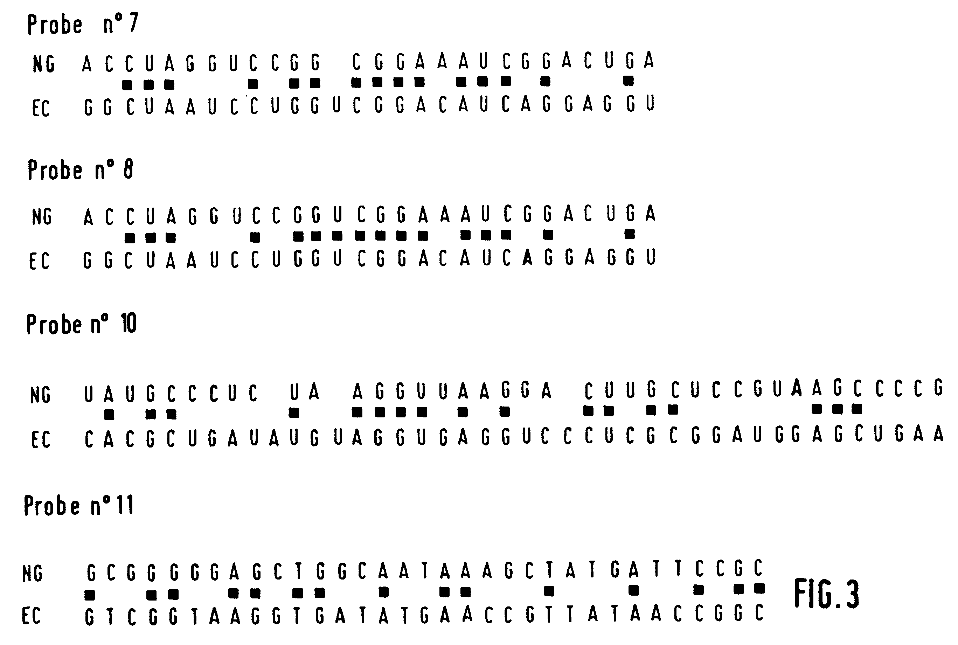

From region II (in Fig. 1) of the 16S rRNA, four probes with different lengths (27, 37, 47 and 57 bases for probes n° 1, n° 2, n° 3 and n° 4 respectively) were tested. The probes n° 7 and n° 8 were derived from the same region in the 23S rRNA. The sequence of probe n° 8 is identical to the sequence of probe n° 7, except that in probe n° 8 an adenosine residue was inserted (see Fig. 3). From all other regions one probe only was used in the experiments.

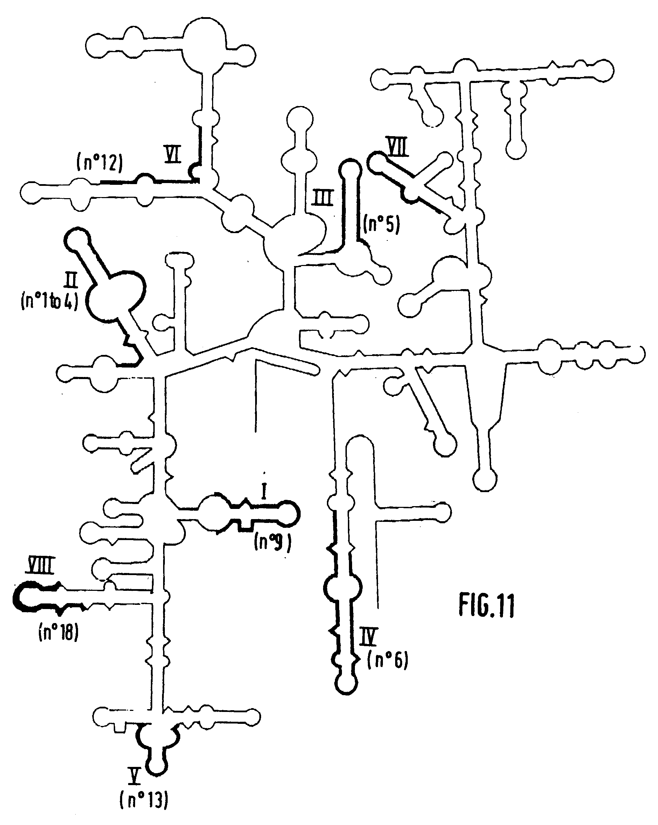

Figure 11 represents the allocations of the regions from which probes of the invention were constructed on the presumptive 16S rRNA recombinant structure of Neisseria gonorrhoeae.

These regions are indicated by heavy bars and numbered by Roman numerals. The corresponding probe-number is indicated between brackets.

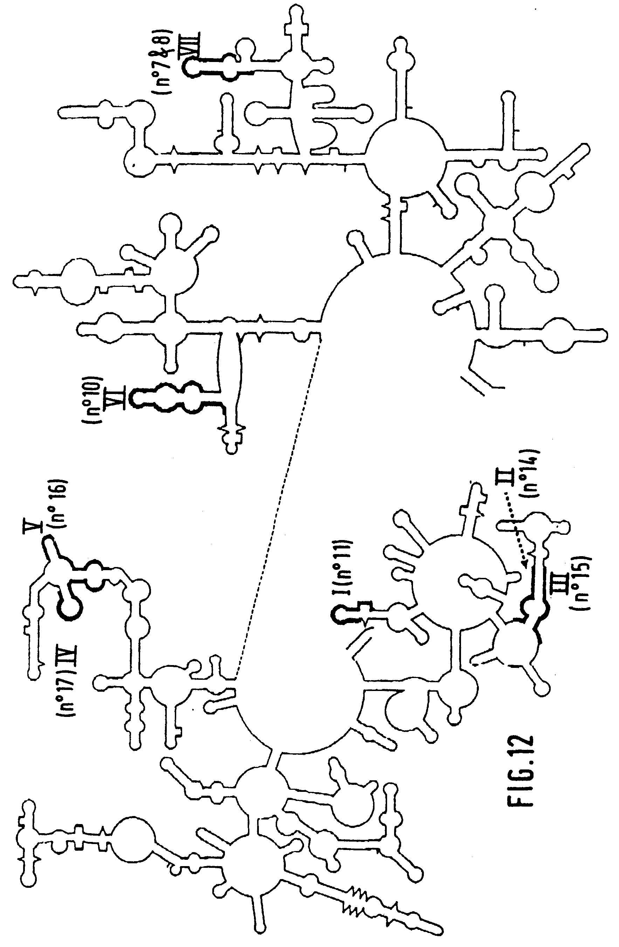

Figure 12 represents the allocation of the regions from which probes of the invention were constructed on the 23S rRNA secondary structure of Escherichia coli (Noller, Ann. Rev. Biochem. 53: 119-162, 1984).

These regions are indicated by heavy bars and numbered by Roman numerals. The corresponding probe-number is indicated between brackets.

2) Specificity of the probes :

a.1) Study of probes n° 1 to n° 11 :

The criterion for specificity was the ability to differentiate between Neisseria gonorrhoeae and N. meningitidis strains. Several independent studies (Kingsbury, 1967 ; Elwell and Falkow, 1977 ; Hoke and Vedros, 1982 ; Riou at al., 1983 ; Guibourdenche et al., 1986) have shown that, despite their distinct pathogenic character both species are genotypically extremely highly related. The DNA:DNA hybridization homology values reported between representatives of both species range between 64 and 93%. These values are most often found among members of the same species, being recalled that a consensus on the definition of a species has been reached (Wayne et al., 1987) which states that "a species generally would include strains with approximately 70% or greater DNA-DNA relatedness and with 5°C or less delta Tm".

EP 0 337 896 B1

According to this definition N. gonorrhoeae and N. meningitidis should be considered as subspecies of one and the same species (Guibourdenche et al., 1986).

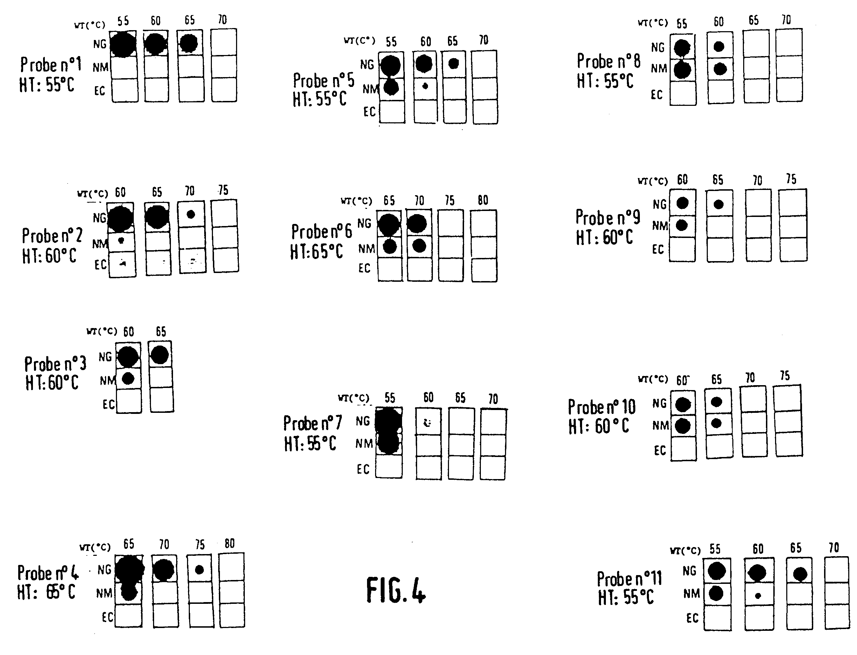

In a first series of experiments, 1 μg of denatured DNA of Neisseria gonorrhoeae NCTC 8375$^T$ (NG), Neisseria meningitidis NCTC 10025$^T$ (NM), and Escherichia coli B (EC) was spotted onto membranes and hybridized at the temperature (HT:i.e. hybridization temperature) and with the probes indicated in Fig. 4. Following hybridization the membranes were washed for 15 min. at different wash temperatures (WT), dried and autoradiographed for 24 h with an intensifying screen at -70°C. From the autoradiographs in Fig. 4, it is clear that all probes derived from regions I, II and III (Fig. 1) of the 16S rRNA (probes n° 1 to 5 and probe n° 9) and one probe (n° 11) of the 23S rRNA are specific for N. gonorrhoeae at the appropriate wash temperature, i.e.

- at a wash temperature of 55, 60 and 65°C for probe n° 1,
- at a wash temperature of 60, 65 and 70°C for probe n° 2,
- at a wash temperature of 65°C for probe n° 3,
- at a wash temperature of 70 and 75°C for probe n° 4,
- at a wash temperature of 65°C for probe n° 5.
- at a wash temperature of 65°C for probe n° 9.
- at a wash temperature of 65°C for probe n° 11.

By comparison, the autoradiographs in Fig. 4 show that probes n° 6, n° 7, n° 8 and n° 10 are not specific for N. gonorrhoeae.

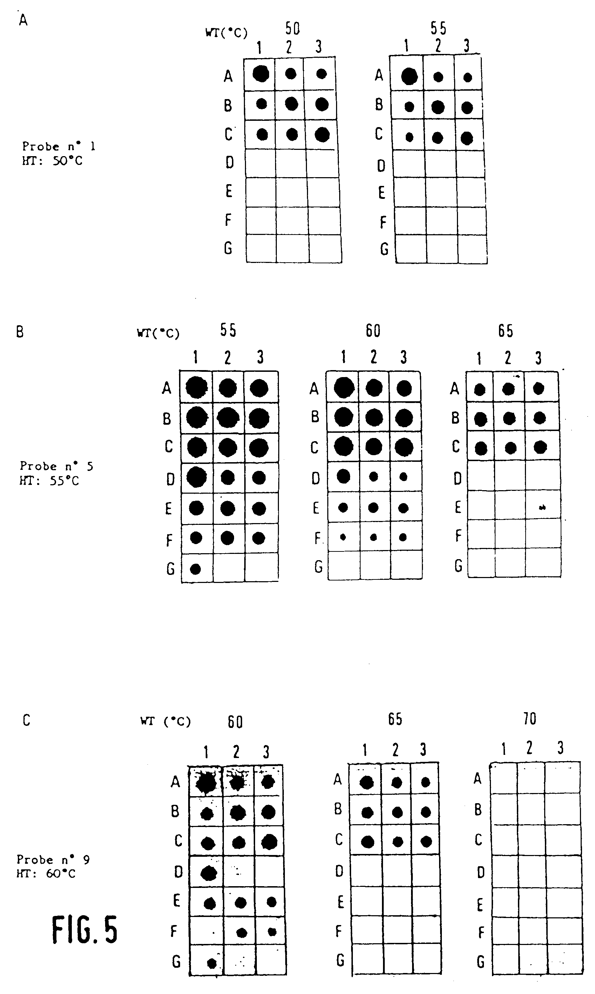

The hybridization results of probes n° 1, n° 5 and n° 9 with genomic DNA from randomly chosen N. gonorrhoeae and N. meningitidis strains are presented in Fig. 5. One μg denatured DNA from nine Neisseria gonorrhoeae strains (row A, B and C, 1 to 3), ten Neisseria meningitidis strains (row D, E and F, 1 to 3 and row G, 1), and one strain each of Pseudomonas testosteroni (row G, 2) and Escherichia coli (row G, 3) were spotted onto nitrocellulose, hybridized with probe n° 1 (panel A), probe n° 5 (panel B) and probe n° 9 (panel C) at 50°C, 55°C, and 60°C respectively and washed at the temperature indicated. The autoradiographs in Fig. 5 show that the probes n° 1, n° 5 and n° 9 duplex with the DNA from all N. gonorrhoeae strains tested. Probes N° 5 and n° 9 were specific for N. gonorrhoeae strains at high stringencies only (for instance hybridization temperature of 55°C, wash temperature of 65°C, in the case of probe n° 5) whereas probe n° 1 remained specific at low stringency conditions (hybridization temperature of 50°C, wash temperature of 50°C or 55°C). In view of the sequence conservation within rRNA cistrons, it seems unlikely that the latter probe will not form stable duplexes with the nucleic acid from other N. gonorrhoeae strains under these non-stringent conditions.

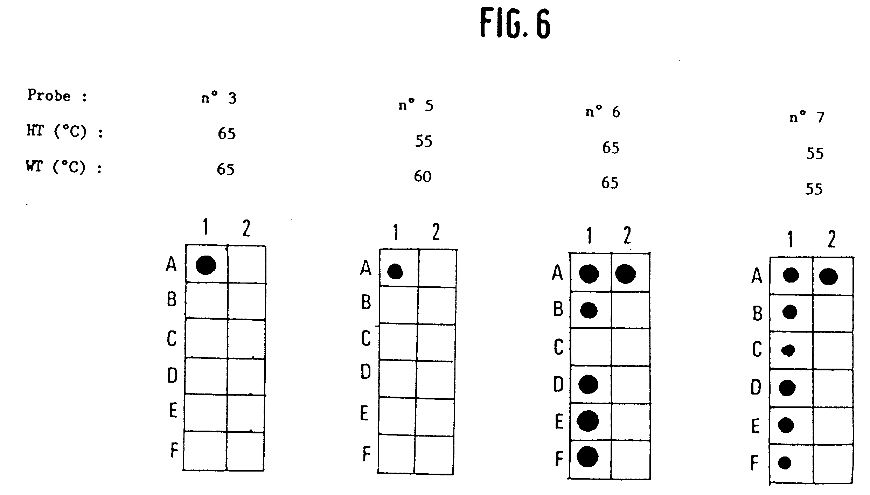

If a taxon-specific rRNA-derived DNA-probe does not detect nucleic acid from closely related organisms, the probability that it will detect nucleic acid from more remotely organisms can considered to be very small. This is shown by Fig. 6 corresponding to an experiment in which probes n° 3, n° 5, n° 6 and n° 7 were hybridized with genomic DNA of seven Neisseria species and some other Gram-negative bacteria. More precisely, Fig. 6 represents hybridization results of selected rRNA-derived DNA-probes with one μg of denatured DNA of Neisseria gonorrhoeae NCTC 8375$^T$ (row A,1), N. meningitidis NCTC 10025$^T$ - (row B,1), N. lactamica NCTC 10617$^T$ (row C,1), N. mucosa CIP 59.51$^T$ (row D,1), N. subflava ATCC 10555 (row E,1), N. flavescens ATCC 13120$^T$ (row F,1), N. elongata ssp. elongata NCTC 10660$^T$ (row A,2), Chromobacterium violaceum NCTC 9757$^{NT}$ (row B,2), Pseudomonas testosteroni ATCC 17407 (row C,2), Haemophilus ducreyi CIP 542 (row D,2), Moraxella (Branhamella) catarrhalis NCTC 4103 (row E,2), and Escherichia coli B (row F,2), spotted on nitrocellulose, hybridized at the temperature and with the probe indicated and washed at the temperature indicated. Under the conditions used, probes n° 3 and 5 did not form stable duplexes with any of the DNAs tested, except for N. gonorrhoeae DNA. Even the less-specific probes (n° 6 and 7) did not produce a detectable signal with non-neisserial DNA. It should be mentioned that probe n° 6 did hybridize with DNA from the type strain of Simonsiella crassa at 60°C in 3SSC and 20% FA.

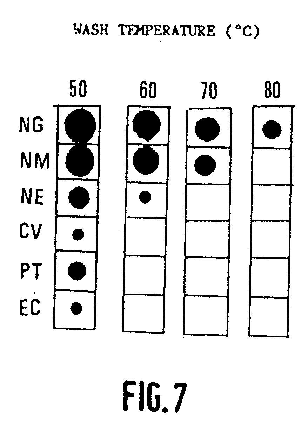

From the results shown in Fig. 4, 5 and 6, it is obvious that the specificity of the probes is highly dependent on the hybridization and wash conditions used. For instance, by simply altering the wash temperature, the detection-range of the probes can be extended, so that one and the same probe can be used to detect N. gonorrhoeae specifically, or a larger group of organisms. This is illustrated in Fig. 7 in which 1 μg of dot-spotted denatured DNA from Neisseria gonorrhoeae NCTC 8375$^T$ (NG), N. meningitidis NCTC 10025$^T$ (NM), N. elongata ssp. elongata NCTC 10660 (NE), Chromobacterium violaceum NCTC 9575$^T$ (CV), Pseudomonas testosteroni ATCC 17407 (PT), and Escherichia coli B (EC) was hybridized at 50°C with $^{32}$P-labeled probe n° 3 in the absence of formamide. Afterwards the membranes were washed at 50, 60, 70 and 80°C respectively in 3SSC and 25mM FB. The membranes were dried and autoradiographed overnight at -70°C with an intensifying screen. It results from this figure that the specificity of the

19

probe decreases when wash temperatures are lowered. At 80°C, probe n° 3 is specific for N. gonorrhoeae DNA. At 70°C, this probe duplexes with N. gonorrhoeae and N. meningitidis DNA, and at 60°C with DNA from the three Neisseria species tested. However, even non-neisserial DNA could be detected when the wash temperature was 50°C.

Table I below summarizes the temperature range in which the probes of the invention are specific for N. gonorrhoeae. For each probe this temperature range is between Ts and Td. Ts is the lowest temperature at which the probe is still specific for N. gonorrhoeae and Td is the temperature at which the probe-target duplex is completely dissociated. These values were obtained under the following conditions : 3 SSC, 25 mM FB pH 7.1 and 20% FA. If these conditions the nature of the probe or the target are changed, the temperatures will change accordingly. The temperatures were experimentally determined using intervals of 5°C, hence the delta 5°C range of Ts and Td. It is to be noted that probe n° 1 may also be specific at a temperature lower than 45-50°C and that probes n° 6, n° 7, n° 8 and n° 10 are not specific for Neisseria gonorrhoeae.

TABLE I

| Probe | Ts(°C) | Td(°C) |
|-------|--------|--------|
| 1 | 45-50 | 65-70 |
| 2 | 55-60 | 70-75 |
| 3 | 60-65 | 70-75 |
| 4 | 65-70 | 75-80 |
| 5 | 60-65 | 65-70 |
| 6 | - | 70-75 |
| 7 | - | 55-60 |
| 8 | - | 60-65 |
| 9 | 60-65 | 65-70 |
| 10 | - | 65-70 |
| 11 | 60-65 | 65-70 |

a.2) Results concerning probe n° 1:

Probe n° 1 was extensively tested using 202 N. gonorrhoeae strains and 84 N. meningitidis strains.
The following method was used:
A few colonies of each strain were applied to Biodyne A membranes which were placed for 3 minutes on Whatman® 3MM paper saturated with 10% SDS. After drying, the membranes were baked for 2 h. at 80°C and hybridized for 1 to 2 h. at 53°C in hybrididization mixture to which about $10^6$ cpm/ml $^{32}$P-labeled probe (n° 1) was added.

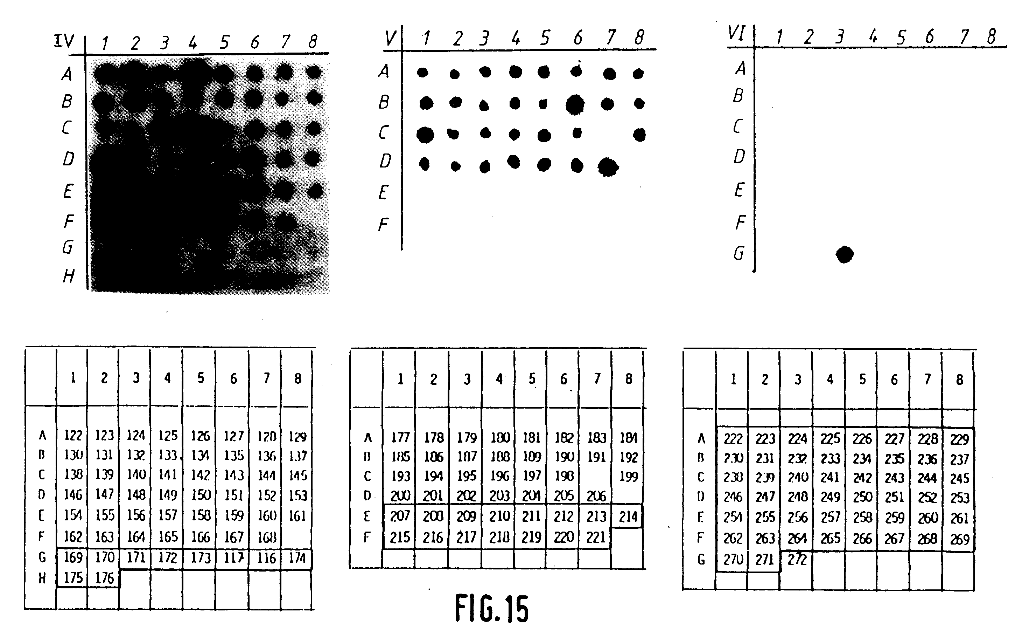

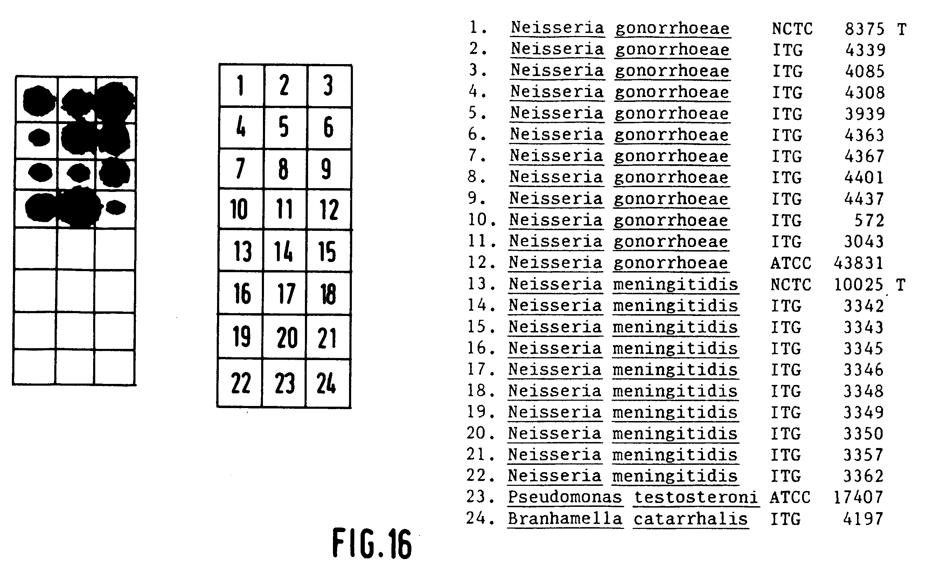

The results are illustrated in Figure 15. In Figure 15, each number represents either a Neisseria gonorrhoeae or a N. meningitidis strain. The N. meningitidis strains are boxed.

All N. gonorrhoeae strains gave an unequivocal positive signal after 3 h. exposure (at -80°C with intensifying screen). None of the N. meningitidis strains gave a positive results even after overnight exposure. The presence of a detectable amount of rRNA in the N. meningitidis spots was afterwards confirmed using a non-specific rRNA-derived probe.

Other experiments showed that probe n° 1 also hybridized to DNA of Neisseria species strain ATCC 43831 (see Fig. 16). In Figure 16, the hybridization results with probe n° 1 and DNA of Neisseria species strain ATCC 43831 are represented: the hybridization and wash temperature was 52.5°C (in 3SSC, 25 mM PB, pH 7.1 and 20% FA).

This ATCC 43831 strain is an unclassified strain with intermediate characteristics between N. gonorrhoeae and N. menigitidis.

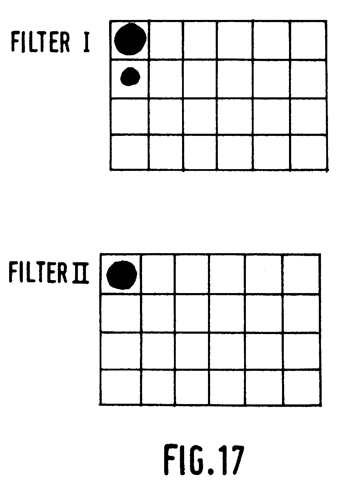

As shown in Figure 17, probe n° 1 did not cross-hybridize with DNA of a variety of other bacteria.

In Figure 17, the hybridization results of probe n°1 with 1 microgram of dot spotted genomic DNA of a variety of bacterial strains are represented: the location of the strains is given in Table II hereafter given and the hybridization and wash temperature was 52.5°C (in 3SSC, 25 mM PB, pH 7.1 and 20% FA).

20

TABLE II

| Strain n° | Name | Culture Collection n° |
|---|---|---|
| 1. | Neisseria gonorrhoeae | N NCTC[a] 8375[T] |
| 2. | Neisseria gonorrhoeae | ITG 4339 |
| 3. | Neisseria gonorrhoeae | ITG 4085 |
| 4. | Neisseria gonorrhoeae | ITG 4308 |
| 5. | Neisseria gonorrhoeae | ITG 3939 |
| 6. | Neisseria gonorrhoeae | ITG 4363 |
| 7. | Neisseria gonorrhoeae | ITG 4367 |
| 8. | Neisseria gonorrhoeae | ITG 4401 |
| 9. | Neisseria gonorrhoeae | ITG 4437 |
| 10. | Neisseria meningitidis | N NCTC 10025[T] |
| 11. | Neisseria meningitidis | ITG 3342 |
| 12. | Neisseria meningitidis | ITG 3343 |
| 13. | Neisseria meningitidis | ITG 3345 |
| 14. | Neisseria meningitidis | ITG 3346 |
| 15. | Neisseria meningitidis | ITG 3348 |
| 16. | Neisseria meningitidis | ITG 3349 |
| 17. | Neisseria meningitidis | ITG 3350 |
| 18. | Neisseria meningitidis | ITG 3357 |
| 19. | Neisseria meningitidis | ITG 3362 |
| 20. | Neisseria polysaccharea | CIP N462[T] |
| 21. | Neisseria lactamica | N NCTC 10616 |
| 22. | Neisseria lactamica | N NCTC 10617[T] |
| 23. | Neisseria lactamica | ITG 3689 |
| 24. | Neisseria lactamica | ITG 3690 |
| 25. | Neisseria cinerea | N NCTC 10294 |
| 26. | Neisseria mucosa | CIP 59.51[T] |
| 27. | Neisseria mucosa | CIP 59.48 |
| 28. | Neisseria mucosa | CIP 59.47 |

TABLE II (continued)

| | | |
|---|---|---|
| 29. | Neisseria macacae | CIP N4176ᵀ |
| 30. | Neisseria flavescens | ATCC 13120ᵀ |
| 31. | Neisseria subflava | ATCC 10555 |
| 32. | Neisseria subflava | ITG 3821 |
| 33. | Neisseria sicca | ITG 3882 |
| 34. | Neisseria elongata subsp. elongata | N NCTC 10660ᵀ |
| 35. | Neisseria elongata subsp. glycolytica | NNCTC 11050 |
| 36. | Neisseria canis | ATCC 14687ᵀ |
| 37. | Neisseria animalis | N NCTC 10212ᵀ |
| 38. | Neisseria denitrificana | ATCC 14686ᵀ |
| 39. | CDC group M-5 | CCUG 4007 |
| 40. | CDC group EF-4a | CDC T-191/78 |
| 41. | Kingella denitrificana | N NCTC 10995ᵀ |
| 42. | Kingella denitrificana | NNCTC 10997 |
| 43. | Kingella kingae | N NCTC 10529ᵀ |
| 44. | Simonsiella muelleri | ATCC 29452 |
| 45. | Simonsiella crassa | ATCC 15533ᵀ |
| 46. | Simonsiella steedae | ATCC 27398 |
| 47. | Simonsiella species | ATCC 27381 |
| 48. | Alysiella filiformis | CCUG 3710ᵀ |
| 49. | Eikenella corrodens | N NCTC 10596ᵀ |
| 50. | Eikenella corrodens | H NIM 801-1 |
| 51. | Chromobacterium violaceum | N NCTC 9757ᵀ |
| 52. | Chromobacterium fluviatile | LMG 6574 |
| 53. | Aquaspirillum dispar | ATCC 27650 |
| 54. | Pseudomonas testosteroni | ATCC 17407 |
| 55. | Oligella urethralis | LMG 6227 |
| 56. | Haemophilus ducrevi | CIP 542ᵀ |
| 57. | Kingella indologenes | N NCTC 10717ᵀ |
| 58. | Moraxella (Branhamella) catarrhelis | N NCTC 4103 |
| 59. | Escherichia coli | B |

22

LOCATION OF THE STRAINS ON FILTER I & II

| 1 | 20 | 24 | 28 | 32 | 36 |
|----|----|----|----|----|----|
| 7 | 21 | 25 | 29 | 33 | 37 |
| 10 | 22 | 26 | 30 | 34 | 38 |
| 17 | 23 | 27 | 31 | 35 | 51 |

FILTER I

| 1 | 40 | 44 | 48 | 52 | 56 |
|----|----|----|----|----|----|
| 10 | 41 | 45 | 49 | 53 | 57 |
| 20 | 42 | 46 | 50 | 54 | 58 |
| 39 | 43 | 47 | 51 | 55 | 59 |

FILTER II

In conclusion, under the conditions used, probe n°1 proved to be 100% specific and 100% reliable for N. gonorrhoeae as compared to conventional identification techniques. The probe also proved to be more reliable than the cryptic plasmid probe initially described by Totten et al., J. Infect. Dis., 148:462-471, 1983 (results not shown).

a.3) Results concerning probe n° 10:

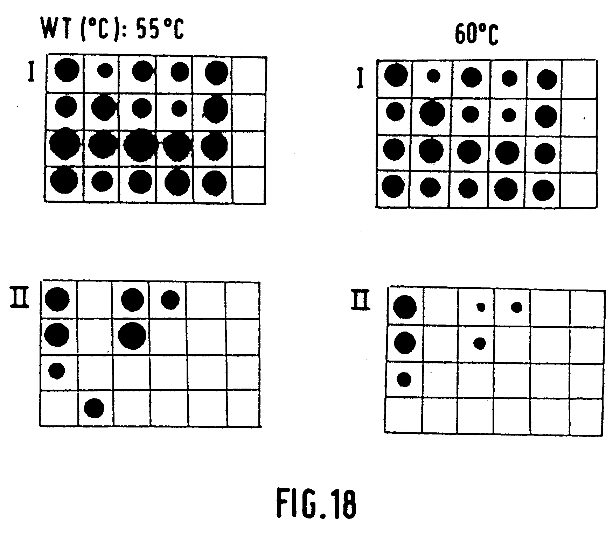

Probe n° 10 was hybridized with a great variety of bacterial DNAs. The results are shown in Figure 18.

In Figure 18, the hybridization results of probe n°10 with 1 microgram dot-spotted genomic DNA from a variety of bacterial strains are represented.

The hybridization temperature was 55°C (in 3SSC, 25 mM PB, pH 7.1 and 20% FA). The wash temperatures are indicated (the same medium was used). The location of the strains is given in Table II.

Probe n°10 hybridizes to DNA of almost all Neisseria strain. At 60°C weak crossreactions are observed also with DNA from Simonsiella and Alysiella strains, which are close relatives of the neisseriae (Rossau et al., IJSB, 1989 in press).

b) Study of probes n° 12 to n° 18 :

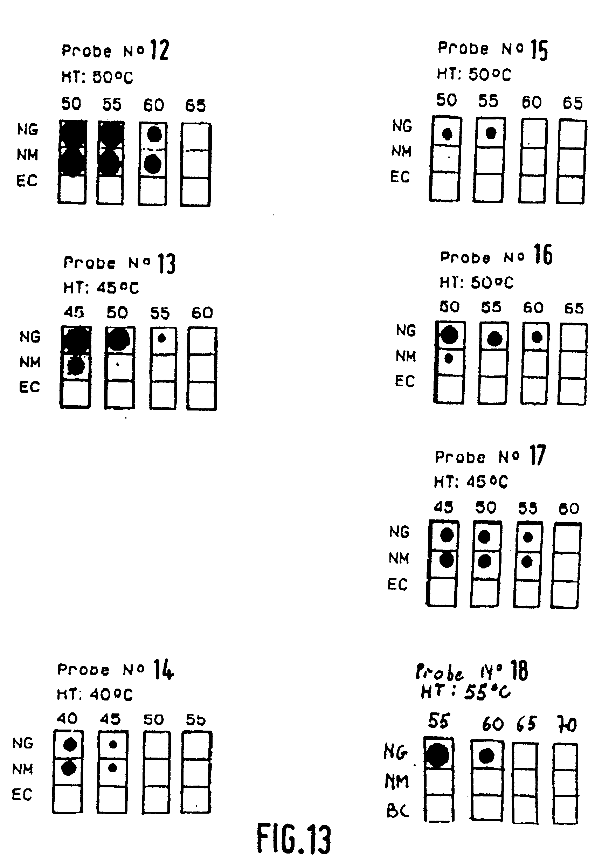

The specificity of probes n°12 to n° 18 was tested as described hereabove (same method, same media). The hybridization (HT) and wash temperature (WT) used, are indicated in Figure 13.

In Figure 13, the specificity of the probes at different wash temperature is determined as follows:

Following hybridization at the temperature (HT) and with the probe indicated, the membranes, on which 1 μg of denatured DNA of Neisseria gonorrhoeae NCTC 8375T (NG), Neisseria meningitidis NCTC 10025T (NM), Escherichia coli B (EC) and Branhamella catarrhalis ITG 4197 (BC) was spotted, were washed for 15 min. at the indicated wash temperature, dried and autoradiographed for 24 h. with an intensifying screen at 70°C.

It is clear from the results shown on Figure 13 that probes n° 12, 14 and 17 are not specific for Neisseria gonorrhoeae. They can be used to detect one or more Neisseria strains at the following hybridization temperature (HT) and wash temperature (WT):

probe n° 12: HT and WT between about 55°C and about 60°C

probe n° 14: HT and WT between about 40°C and about 45°C

probe n° 17: HT and WT between about 50°C and about 55°C

Although probes n° 15 and 16 do not hybridize with DNA of the type strain of N. meningitidis under stringent conditions (see Figure 13), further experiments showed that they are not sufficiently specific for N. gonorrhoeae as they crosshybridize to some other Neisseria strains, mainly N. meningitidis strains. Both probes can thus be used only to detect one or more Neisseria strains at the following HT and WT:

probe n° 15: HT and WT between about 50°C and about 55°C

probe n° 16: HT and WT between about 50°C and about 60°C.

Under highly stringent conditions probes n° 13 and 18 hybridized to all N. gonorrhoeae strains tested and not with any of the N. meningitidis strains tested at the following HT and WT:

probe n° 13: HT and WT from about 50 to about 55°C

probe n° 18: about 60°C

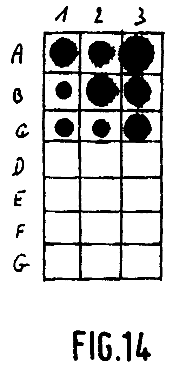

An example is given with respect to the hybridization results with probe n° 18 in Figure 14.

Probe n° 18 was hybridized with 1 μg denatured dot-spotted DNA from 9 N. gonorrhoeae strains (row A, B and C, 1 to 3), 10 N. meningitidis strains (row D, E and F, 1 to 3 and row G, 1) and two reference strains not related to Neisseria (row G, 2 and 3). The hybridization and wash temperature was 60°C.

Table III below summarizes the temperature range in which the probes n° 12 and n° 18 of the invention are specific for N. gonorrhoeae. In this Table, Ts and Td have the meaning above explained under the conditions hereabove detailed (cf. Table I).

TABLE III

| Probe | Ts(°C) | Td(°C) |
|-------|--------|--------|
| 12 | - | 60-65 |
| 13 | 50-55 | 55-60 |
| 14 | - | 45-50 |
| 15 | - | 55-60 |
| 16 | - | 60-65 |
| 17 | | 55-60 |
| 18 | 55-60 | 60-65 |

The probes of the invention can be used in a sandwich hybridization system which enhances the specificity of a nucleic acid probe based assay.

The principle and the use of sandwich hybridizations in a nucleic acid probe based assay have been already described (e.g.: Dunn and Hassel, Cell, 12: 23-36, 1977; Ranki et al., Gene 21, 77-85, 1983). Although direct hybridization assays have favourable kinetics, sandwich hybridizations are advantageous with respect to a higher signal to noise ratio. Moreover sandwich hybridizations can enhance the specificity of a nucleic acid probe based assay. If properly designed, a sandwich hybridization assay indeed maximizes the specificity of a nucleic acid probe based test when using two probes recognizing two different nucleic acid stretches of one and the same organism. The only demands which must be met are that both probes (i) hybridize to nucleic acid of the target organism and (ii) do not hybridize to the same non-target organisms.

For two given probes I and II, the sandwich hybridization system can be described as follows:

Probe n° I hybridizes to nucleic acid from organisms A and B (not with C).

Probe n° II hybridizes to nucleic acid from organisms A and C (not with B).

Since it is absolutely required that both probes hybridize to the target nucleic acid, a detectable signal will be generated only if nucleic acid from organism A is present in the sample.

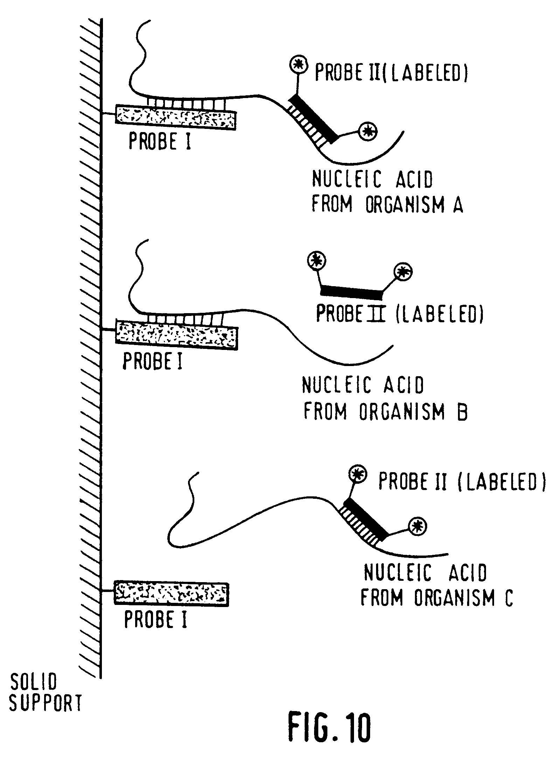

In Figure 10, a sandwich hybridization assay with enhanced specificity is represented:

- Probe I hybridizes with nucleic acid from organisms A and B and is fixed to the solid support.
- Probe II hybridizes with nucleic acid from organisms A and C and is labeled.

The test will be positive only if the labeled probe (probe II) will become indirectly fixed to the support, i.e. if nucleic acid from organism A is present.

More particularly, on the first drawing of figure 10, probe I and probe II hybridize with the target nucleic acid (A). The labeled probe is fixed. The test is positive.

On the second drawing of figure 10, probe I hybridizes with nucleic acid from organism B, but probe II does not. The labeled probe is not fixed. There will be no signal ; the test is negative.

On the third drawing of figure 10, nucleic acid from organism C is not fixed. Consequently the labeled probe is not fixed. There will be not signal ; the test is negative.

Some of the probes of the invention can be combined in a sandwich hybridization assay which is highly specific for Neisseria gonorrhoeae. Advantageous combinations of probes of the invention which maximize the specificity for N. gonorrhoeae are:

- probe of group 9 and anyone of the probes of the following groups: 1, 2, 3, 5 and 13.

24

EP 0 337 896 B1

- probe of group 13 and anyone of the probes of the following groups: 1, 2, 3 and 5.
- probe of group 18 and anyone of the probes of the following groups: 1, 2, 3, 5, 9 and 13.
- probe of group 5 and anyone of the probes of the following groups: 1, 2 and 3.

Advantageous combinations of probes of the invention which are specific for N. gonorrhoeae are the following ones :
- probe of groupe 13 and anyone of the probes of the following groups 5, 9 and 18.

Preferred combinations of probes of the invention which are specific for N. gonorrhoeae are the following ones:
- probe of group 9 and of group 5.
- probe of group 18 and one of the probes of the following groups: 5 and 9.

These combinations have the 16S rRNA molecule as target. Combinations between 16S rRNA- and 23S rRNA-derived probes (e.g. probe of group 11 and probe of group 13 or group 18) are only possible if the genomic DNA is the target-molecule.

In the sandwich hybridization process for detecting N. gonorrhoeae, the probes can be added simultaneously or not, to the biological sample in which the target DNA or RNA is sought.

The advantageous approximate hybridization temperature and wash temperature for the above mentioned combinations are given in the following Table IV:

Table IV

| Number of the probe | 1 | 2 | 3 | 5 | 9 | 13 | 18 |
|---|---|---|---|---|---|---|---|
| 1 | * | | | | | | |
| 2 | - | * | | | | | |
| 3 | - | - | * | | | | |
| 5 | 53 | 60 | 65 | * | | | |
| 9 | 55 | 55 | 65 | 65 | * | | |
| 13 | 52 | 52 | 52 | 52 | 52 | * | |
| 18 | 53 | 60 | 60 | 60 | 60 | 52 | * |

This Table represents preferred approximate hybridization and wash temperatures (in °C) for the different combinations of probes in a sandwich hybridization assay. The combinations indicated by "-" should not be used.

For instance,
- an assay in which probes n° 1 and n° 5 are combined should be performed at about 53°C, and
- an assay in which probes n° 18 and n° 9 are combined should be performed at about 60°C.

The invention also relates to a kit for sandwich hybridization assay, for the detection in vitro of Neisseria gonorrhoeae strains in a biological sample, said kit containing :
- at least two probes specific for N. gonorrhoeae as above selected from the following combinations:
- probe of group 9 and anyone of the probes of the following groups: 1, 2, 3, 5 and 13.
- probe of group 13 and anyone of the probes of the following groups: 1, 2, 3 and 5.
- probe of group 18 and anyone of the probes of the following groups: 1, 2, 3, 5, 9 and 13.
- probe of group 5 and anyone of the probes of the following groups: 1, 2 and 3 and particularly from the following combination:
- probe of group 13 and anyone of the probes of the following groups: 5, 9 and 18 and more particularly from the following combinations:
- probe of group 9 and of group 5.
- probe of group 18 and one of the probes of the following groups: 5 and 9
- the buffer or components necessary for producing the buffer enabling hybridization reaction between these probes and the DNAs and/or RNAs of a strain of Neisseria gonorrhoeae to be carried out, - when appropriate means for detecting the hybrids resulting from the preceding hybridization.

The fact that Neisseria gonorrhoeae and Neisseria meningitidis strains can be distinguished from each other by some of the probes of the invention has some widespread implications on the use of rRNA-derived probes in general, because both taxa are genotypically related at the subspecies level. Hence, the probes according to the invention cannot be used exclusively to detect large groups of organisms, but can be used also to differentiate between organisms at the subspecies level. There is no reason to believe that this should be the case in Neisseria only, and not in other taxa. Provided that the probe sequence and the hybridization conditions are carefully chosen, differentiation at the subspecies level can be accomplished in

25

a simple direct hybridization format, making the tedious Southern-blot analysis obsolete.

The specific probes of the invention, in particular probes n° 1 to n° 5, n° 9, n° 11, n° 13 and n° 18 should find application in the culture confirmation of Neisseria gonorrhoeae and the diagnosis of N. gonorrhoeae in all types of clinical samples, since no interference is to be expected from other microorganisms under the appropriate conditions.

Moreover, the probes of the invention can be used for taxonomic or epidemiological investigations based on restriction fragment length polymorphism analysis (Grimont and Grimont, 1986), or to identify and classify related microorganisms.

There follows a bibliography by way of articles which belong to the background of this invention.

BIBLIOGRAPHY

1. Brosius, J., T.J. Dull, and H.F. Noller. 1980. Complete nucleotide sequence of a 23S ribosomal RNA gene from Escherichia coli. Proc. Natl. Acad. Sci. USA 77:201-204.

1a. Brosius, J., J.L. Palmer, J.P. Kennedy, and H.F. Noller. 1978 . Complete nucleotide sequence of 16S ribosomal RNA gene from Escherichia coli. Proc. Natl. Acad. Sci. U.S.A. 75:4801-4805.

2. Edelstein, P.H. 1986. Evaluation of the Gen-Probe DNA probe for the detection of legionellae in culture. J. Clin. Microbiol. 23: 481-484.

3. Elwell, L.P., and S. Falkow. 1977. Plasmids of the genus Neisseria. p. 138-154. In : Roberts R.B., (ed.). The gonococcus. John Wiley and Sons. New York.

4. Festl, H., W. Ludwig, adn K.H. Schleifer. 1986. DNA hybridization probe for the Pseudomonas fluorescens group. Appl. Env. Microbiol. 52:1190-1194.

5. Gobel, U.B., A. Geiser, and E.J. Stanbridge. 1987. Oligonucleotide probes complementary to variable regions of ribosomal RNA discriminate between Mycoplasma species. J. Gen. Microbiol. 133: 1969-1974.

6. Grimont, F., and P.A.D. Grimont. 1986. Ribosomal ribonucleic acid gene restriction patterns as potential taxonomic tools. Ann. Microbiol. (Inst. Pasteur) 137B: 165-175.

7. Guibourdenche, M., M.Y. Popoff, and J.Y. Riou. 1986. Deoxyribonucleic acid relatedness among Neisseria gonorrhoeae, N. meningitidis, N. lactamica, N. cinearea and "Neisseria polysaccharea". Ann. Microbiol. (Inst. Pasteur). 137B:177-185.

8. Haun, G., and U. Gùbel, 1987. Oligonucleotide probes for genus-, species- and subspecies-specific indetification of representatives of the genus Proteus. FEMS (Fed. Eur. Microbiol. Soc.) Microbiol. Lett. 43:187-193.

9. Hoke, C., and N.A. Vedros. 1982. Taxonomy of the neisseriae : deoxyribonucleic acid base composition, interspecific transformation, and deoxyribonucleic acid hybridization. Int. J. Syst. Bacteriol. 32:57-66.

10. Kahn, M., R. Kolter, C. Thomas, D. Figurski, R. Meyer, E. Remaut, and D.R. Helinski. 1979. Plasmid cloning vehicles derived from plasmids ColE1, F, R6K, RK2. Methods Enzymol. 68:268.

11. Kingsbury, D.T. 1967. Deoxyribonucleic acid homologies among species of the genus Neisseria. J. Bacteriol. 94:870-874.

12. Maniatis, T., E.F. Fritsch, and J. Sambrook (ed.). 1982. Molecular cloning : a laboratory manual. Cold Spring Harbor, New York.

13. Marmur, J.A. 1961. A procedure for the isolation of deoxyribonucleic acid from micro-organisms. J. Mol. Biol. 3:208-218.

14. Palmer, L., S. Falkow, and L. Klevan. 1986. 16S ribosomal RNA genes of Chlamydia trachomatis. p. 89-91. In. : D. Oriel, G. Ridgway, J. Schachter, D. Taylor-Robinson, and M. Ward (ed.). Chlamydia infections. Cambridge University Press, Cambridge, U.K.

15. Riou, J.Y., M. Guibourdenche, and M.Y. Popoff. 1983. A new taxon in the genus Neisseria. Ann. Microbiol. (Inst. Pasteur) 134B:257-267.

16. Rossau, R., A. Van Landschoot, W. Mannheim, and J. De Ley. 1986. Inter- and intrageneric similarities of ribosomal ribonucleic acid cistrons of the Neisseriaceae. Int. J. Syst. Bacteriol. 36:323-332.

17. Wayne, L.G., D.J. Brenner, R.R. Colwell, P.A.D. Grimont, O. Kandler, M.I. Krichevsky, L.H. Moore, W.E.C. Moore, R.G.E. Murray, E. Stackebrandt, M.P. Starr, and H.G. Truper. 1987. Report of the ad hoc committee on reconciliation of approaches to bacterial systematics. Int. J. Syst. Bacteriol. 37:463-464.

18. Wilkinson, H.W. J.S. Sampson, and B.B. Plikaytis. 1986. Evaluation of a commercial gene probe for identification of Legionella cultures. J. Clin. Microbiol. 23:217-220.

19. Woese, C.R., E. Gutell, R. Gupta, and H.F. Noller. 1983. Detailed analysis of the higher-order structure of 16S-like ribosomal ribonucleic acids. Microbiol. Rev. 47:621-669.

20. Yang, D., Y. Oyaizu, H. Oyaizu, G.J. Olsen, and C.R. Woese. 1985. Mitochondiral Origins. Proc. Natl. Acad. Sci. U.S.A. 82:4443-4447.

**Claims**

1.   Probe for detecting one or more Neisseria strains, containing :
   - either a sequence belonging to a nucleic acid selected from the following groups of nucleic acids and which includes itself of from 10 to the maximum number of nucleotides of the selected nucleic acid

```
GGTACCGTCATCGGCCGCCGATATTGGCAACAGCCTTTTCTTCCCTGACAAAAGTCC
                                               (4)
GGUACCGUCAUCGGCCGCCGAUAUUGGCAACAGCCUUUUCUUCCCUGACAAAAGUCC
                                               (4bis)
GGACTTTTGTCAGGGAAGAAAAGGCTGTTGCCAATATCGGCGGCCGATGACGGTACC
                                               (4ter)
GGACUUUUGUCAGGGAAGAAAAGGCUGUUGCCAAUAUCGGCGGCCGAUGACGGUACC
                                               (4quater)
ACGCTACCAAGCAATCAAGTTGCCCAACAGCTAA            (5)
ACGCUACCAAGCAAUCAAGUUGCCCAACAGCUAA            (5bis)
TTAGCTGTTGGGCAACTTGATTGCTTGGTAGCGT            (5ter)
UUAGCUGUUGGGCAACUUGAUUGCUUGGUAGCGU            (5quater)
ATACCGTGGTAAGCGGACTCCTTGCGGTTACCCTACCTACTTCTGGTATCCCCCAC
                                               (6)
AUACCGUGGUAAGCGGACUCCUUGCGGUUACCCUACCUACUUCUGGUAUCCCCCAC
                                               (6bis)
GTGGGGGATACCAGAAGTAGGTAGGGTAACCGCAAGGAGTCCGCTTACCACGGTAT
                                               (6ter)
GUGGGGGAUACCAGAAGUAGGUAGGGUAACCGCAAGGAGUCCGCUUACCACGGUAU
                                               (6quater)
TCAGTCCGATTTCCGCCGGACCTAGGT                   (7)
UCAGUCCGAUUUCCGCCGGACCUAGGU                   (7bis)
ACCTAGGTCCGGCGGAAATCGGACTGA                   (7ter)
ACCUAGGUCCGGCGGAAAUCGGACUGA                   (7quater)
TCAGTCCGATTTCCGACCGGACCTAGGT                  (8)
UCAGUCCGAUUUCCGACCGGACCUAGGU                  (8bis)
ACCTAGGTCCGGTCGGAAATCGGACTGA                  (8ter)
ACCUAGGUCCGGUCGGAAAUCGGACUGA                  (8quater)
```

```
CGCCACCCGAGAAGCAAGCTTCCCTGTGCTGC              (9)

CGCCACCCGAGAAGCAAGCUUCCCUGUGCUGC              (9bis)

GCAGCACAGGGAAGCTTGCTTCTCGGGTGGCG              (9ter)

GCAGCACAGGGAAGCUUGCUUCUCGGGUGGCG              (9quater)

CGGGGCTTACGGAGCAAGTCCTTAACCTTAGAGGGCATA       (10)

CGGGGCUUACGGAGCAAGUCCUUAACCUUAGAGGGCAUA       (10bis)

TATGCCCTCTAAGGTTAAGGACTTGCTCCGTAAGCCCCG       (10ter)

UAUGCCCUCUAAGGUUAAGGACUUGCUCCGUAAGCCCCG       (10quater)

GCGGAATCATAGCTTTATTGCCAGCTCCCCCGC             (11)

GCGGAAUCAUAGCUUUAUUGCCAGCUCCCCCGC             (11bis)

GCGGGGGAGCTGGCAATAAAGCTATGATTCCGC             (11ter)

GCGGGGGAGCUGGCAAUAAAGCUAUGAUUCCGC             (11quater)

GACACACTCGAGTCACCCAGTTCAGAAC                  (12)

GACACACUCGAGUCACCCAGUUCAGAAC                  (12bis)

GTTCTGAACTGGGTGACTCGAGTGTGTC                  (12ter)

GUUCUGAACUGGGUGACUCGAGUGUGUC                  (12quater)

TGCTTTCCCTCTCAAGACGTATGC                      (13)

UGCUUUCCCUCUCAAGACGUAUGC                      (13bis)

GCATACGTCTTGAGAGGGAAAGCA                      (13ter)

GCAUACGUCUUGAGAGGGAAAGCA                      (13quater)

TCTCGACAGTTATTACGTACA                         (14)

UCUCGACAGUUAUUACGUACA                         (14bis)

TGTACGTAATAACTGTCGAGA                         (14ter)

UGUACGUAAUAACUGUCGAGA                         (14quater)

TTTCGTACGCTTAGTACCGCTGTTGAGA                  (15)

UUUCGUACGCUUAGUACCGCUGUUGAGA                  (15bis)

TCTCAACAGCGGTACTAAGCGTACGAAA                  (15ter)

UCUCAACAGCGGUACUAAGCGUACGAAA                  (15quater)

GTGGTATCGGTTGCTTCGTGTCCGTAGACA                (16)

GUGGUAUCGGUUGCUUCGUGUCCGUAGACA                (16bis)

TGTCTACGGACACGAAGCAACCGATACCAC                (16ter)

UGUCUACGGACACGAAGCAACCGAUACCAC                (16quater)

AAGCTATTCCAACAGCTTGCCAACCTAA                  (17)

AAGCUAUUCCAACAGCUUGCCAACCUAA                  (17bis)

TTAGGTTGGCAAGCTGTTGGAATAGCTT                  (17ter)

UUAGGUUGGCAAGCUGUUGGAAUAGCUU                  (17quater)
```

```
TGGTGGGCCTTTACCCCGCCAACCAGCT            (18)

UGGUGGGCCUUUACCCCGCCAACCAGCU            (18bis)

AGCTGGTTGGCGGGGTAAAGGCCCACCA            (18ter)

AGCUGGUUGGCGGGGUAAAGGCCCACCA            (18quater)
```

provided that the probe does not consist of the following sequence:
TCA TCG GCC GCC GAT ATT GGC
- or a variant sequence which distinguishes of any of the preceding sequences (4) to (18)
  . either by addition to or removal from any of their respective extremities of one or several nucleotides,
  . or changing within any of said sequences of one or more nucleotides,
  . or both,
  yet provided that in any of the above circumstances said probe still hybridizes with the same RNA or DNA target as the corresponding unmodified sequence.

2.  The probe of claim 1, containing :
    - either a sequence belonging to a nucleic acid selected from the following groups of nucleic acids and which includes itself of from 10 to the maximum number of nucleotides of the selected nucleic acid

```
TCGGCCGCCGATATTGGCAACAGCCTT               (1)

UCGGCCGCCGAUAUUGGCAACAGCCUU               (1bis)

AAGGCTGTTGCCAATATCGGCGGCCGA               (1ter)

AAGGCUGUUGCCAAUAUCGGCGGCCGA               (1quater)

TCGGCCGCCGATATTGGCAACAGCCTTTTCTTCCCTG     (2)

UCGGCCGCCGAUAUUGGCAACAGCCUUUUCUUCCCUG     (2bis)

CAGGGAAGAAAAGGCTGTTGCCAATATCGGCGGCCGA     (2ter)

CAGGGAAGAAAAGGCUGUUGCCAAUAUCGGCGGCCGA     (2quater)

GGTACCGTCATCGGCCGCCGATATTGGCAACAGCCTTTTCTTCCCTG      (3)

GGUACCGUCAUCGGCCGCCGAUAUUGGCAACAGCCUUUUCUUCCCUG      (3bis)

CAGGGAAGAAAAGGCTGTTGCCAATATCGGCGGCCGATGACGGTACC      (3ter)

CAGGGAAGAAAAGGCUGUUGCCAAUAUCGGCGGCCGAUGACGGUACC(3quater)
```

provided that the probe does not consist of the following sequence:
TCA TCG GCC GCC GAT ATT GGC
- or a variant sequence which distinguishes of any of the preceding sequences (1) to (18)
  . either by addition to or removal from any of their respective extremities of one or several nucleotides,
  . or changing within any of said sequences of one or more nucleotides,
  . or both,
  yet provided that in any of the above circumstances said probe still hybridises with the same RNA or DNA target as the corresponding unmodified sequence.

3.  Probe for detecting one or more Neisseria strains, which targets one of the sequences defined hereunder or the corresponding complementary sequence, when the hybridization medium or the wash medium or both as appropriate are the following ones :
    hybridization medium : containing about 3xSSC, (SSC = 0.15M NaCl, 0.015M sodium citrate, pH 7.0) about 25mM of phosphate buffer pH 7.1, 20% deionized formamide 0.02% ficoll, 0.02% bovine serum albumin, 0.02% polyvinylpyrrolidone, and about 0.1 mg/ml sheared, denatured salmon sperm DNA,
wash medium : containing about 3XSSC, 25 mM phosphate buffer pH 7.1, and 20% deionized formamide
wherein said target sequences and the corresponding relevant hybridization temperatures (HT) and wash temperatures (WT) respectively are as follows :

```
AAGGCUGUUGCCAAUAUCGGCGGCCGA
HT and/or WT : about 55°C
CAGGGAAGAAAAGGCUGUUGCCAAUAUCGGCGGCCGA
HT and/or WT : about 60°C
CAGGGAAGAAAAGGCUGUUGCCAAUAUCGGCGGCCGAUGACGGUACC
HT and/or WT : about 60°C
```

GGACUUUUGUCAGGGAAGAAAAGGCUGUUGCCAAUAUCGGCGGCCGAUGACGGUACC

HT and/or WT : about 65°C

UUAGCUGUUGGGCAACUUGAUUGCUUGGUAGCGU

HT and/or WT : about 55°C to about 60°C

GUGGGGGAUACCAGAAGUAGGUAGGGUAACCGCAAGGAGUCCGCUUACCACGGUAU

HT and/or WT : about 65°C to about 70°C

ACCUAGGUCCGGCGGAAAUCGGACUGA

HT and/or WT : about 55°C

ACCUAGGUCCGGUCGGAAAUCGGACUGA

HT and/or WT : about 55°C to about 60°C

GCAGCACAGGGAAGCUUGCUUCUCGGGUGGCG

HT and/or WT : about 55°C to about 60°C

UAUGCCCUCUAAGGUUAAGGACUUGCUCCGUAAGCCCCG

HT and/or WT : about 60°C to about 65°C

GCGGGGGAGCUGGCAAUAAAGCUAUGAUUCCGC

HT and/or WT : about 55°C

GUUCUGAACUGGGUGACUCGAGUGUGUC

HT and/or WT : about 55°C to about 60°C,

GCAUACGUCUUGAGAGGGAAAGCA

HT and/or WT : about 45°C,

UGUACGUAAUAACUGUCGAGA

HT and/or WT : about 40°C to about 45°C,

UCUCAACAGCGGUACUAAGCGUACGAAA

HT and/or WT : about 50°C to about 55°C,

UGUCUACGGACACGAAGCAACCGAUACCAC

HT and/or WT : about 50°C to about 60°C,

UUAGGUUGGCAAGCUGUUGGAAUAGCUU

HT and/or WT : about 50°C to about 55°C,

AGCUGGUUGGCGGGGUAAAGGCCCACCA

HT and/or WT : about 45°C.

4. Probe for detecting one or more Neisseria gonorrhoeae strains from other bacterial strains, and in particular from other Neisseria strains, containing
   - either a sequence belonging to a nucleic acid selected from the following groups of nucleic acids and which includes itself of from 10 to the maximum number of nucleotides of the selected nucleic acid

```
       TCGGCCGCCGATATTGGCAACAGCCTT                    (1)
       UCGGCCGCCGAUAUUGGCAACAGCCUU                    (1bis)
       AAGGCTGTTGCCAATATCGGCGGCCGA                    (1ter)
       AAGGCUGUUGCCAAUAUCGGCGGCCGA                  (1quater)
TCGGCCGCCGATATTGGCAACAGCCTTTTCTTCCCTG   (2)
UCGGCCGCCGAUAUUGGCAACAGCCUUUUCUUCCCUG  (2bis)
CAGGGAAGAAAAGGCTGTTGCCAATATCGGCGGCCGA  (2ter)
CAGGGAAGAAAAGGCUGUUGCCAAUAUCGGCGGCCGA (2quater)
GGTACCGTCATCGGCCGCCGATATTGGCAACAGCCTTTTCTTCCCTG  (3)
GGUACCGUCAUCGGCCGCCGAUAUUGGCAACAGCCUUUUCUUCCCUG  (3bis)
CAGGGAAGAAAAGGCTGTTGCCAATATCGGCGGCCGATGACGGTACC  (3ter)
CAGGGAAGAAAAGGCUGUUGCCAAUAUCGGCGGCCGAUGACGGUACC (3quater)
GGTACCGTCATCGGCCGCCGATATTGGCAACAGCCTTTTCTTCCCTGACAAAAGTCC
                                                    (4)
GGUACCGUCAUCGGCCGCCGAUAUUGGCAACAGCCUUUUCUUCCCUGACAAAAGUCC
                                                    (4bis)
GGACTTTTGTCAGGGAAGAAAAGGCTGTTGCCAATATCGGCGGCCGATGACGGTACC
                                                    (4ter)
GGACUUUUGUCAGGGAAGAAAAGGCUGUUGCCAAUAUCGGCGGCCGAUGACGGUACC
                                                  (4quater)
ACGCTACCAAGCAATCAAGTTGCCCAACAGCTAA                 (5)
ACGCUACCAAGCAAUCAAGUUGCCCAACAGCUAA                 (5bis)
TTAGCTGTTGGGCAACTTGATTGCTTGGTAGCGT                 (5ter)
UUAGCUGUUGGGCAACUUGAUUGCUUGGUAGCGU               (5quater)
CGCCACCCGAGAAGCAAGCTTCCCTGTGCTGC                   (9)
CGCCACCCGAGAAGCAAGCUUCCCUGUGCUGC                   (9bis)
GCAGCACAGGGAAGCTTGCTTCTCGGGTGGCG                   (9ter)
GCAGCACAGGGAAGCUUGCUUCUCGGGUGGCG                 (9quater)
GCGGAATCATAGCTTTATTGCCAGCTCCCCCGC                  (11)
GCGGAAUCAUAGCUUUAUUGCCAGCUCCCCCGC                  (11bis)
GCGGGGGAGCTGGCAATAAAGCTATGATTCCGC                  (11ter)
GCGGGGGAGCUGGCAAUAAAGCUAUGAUUCCGC                (11quater)
TGCTTTCCCTCTCAAGACGTATGC                           (13)
UGCUUUCCCUCUCAAGACGUAUGC                           (13bis)
```

```
GCATACGTCTTGAGAGGGAAAGCA                    (13ter)
GCAUACGUCUUGAGAGGGAAAGCA                    (13quater)
TGGTGGGCCTTTACCCCGCCAACCAGCT                (18)
UGGUGGGCCUUUACCCCGCCAACCAGCU                (18bis)
AGCTGGTTGGCGGGGTAAAGGCCCACCA                (18ter)
AGCUGGUUGGCGGGGUAAAGGCCCACCA                (18quater)
```

provided that the probe does not consist of the following sequence:
TCA TCG GCC GCC GAT ATT GGC
- or a variant sequence which distinguishes of any of the preceding sequences (1) to (18)
  . either by addition to or removal from any of their respective extremities of one or several nucleotides,
  . or changing within any of said sequences of one or more nucleotides,
  . or both,
  yet provided that in any of the above circumstances said probe still hybridizes with the same RNA or DNA target as the corresponding unmodified sequence.

5.  Probe for detecting one or more Neisseria gonorrhoeae strains from other bacterial strains, and in particular from other Neisseria strains, which target one of the sequences defined hereunder or the corresponding complementary sequence, when the hybridization medium or the wash medium or both as appropriate are the following ones :
    hybridization medium : containing about 3xSSC, (SSC = 0.15M NaCl, 0.015M sodium citrate, pH 7.0) about 25mM of phosphate buffer pH 7.1, 20% deionized formamide 0.02% ficoll, 0.02% bovine serum albumin, 0.02% polyvinylpyrrolidone, and about 0.1 mg/ml sheared, denatured salmon sperm DNA,
    wash medium : containing about 3XSSC, 25 mM phosphate buffer pH 7.1, and 20% deionized formamide
    wherein said target sequences and the corresponding relevant hybridization temperatures (HT) and wash temperatures (WT) respectively are as follows :

AAGGCUGUUGCCAAUAUCGGCGGCCGA

HT and/or WT : about 50°C to about 65°C

CAGGGAAGAAAAGGCUGUUGCCAAUAUCGGCGGCCGA

HT and/or WT : about 60°C to about 70°C

CAGGGAAGAAAAGGCUGUUGCCAAUAUCGGCGGCCGAUGACGGUACC

HT and/or WT : about 65°C to about 70°C

GGACUUUUGUCAGGGAAGAAAAGGCUGUUGCAAUAUCGGCGGCCGAUGACGGUACC

HT and/or WT : about 70°C to about 75°C

UUAGCUGUUGGGCAACUUGAUUGCUUGGUAGCGU

HT and/or WT : about 65°C

GCAGCACAGGGAAGCUUGCUUCUCGGGUGGCG

HT and/or WT : about 65°C

GCGGGGGAGCUGGCAAUAAAGCUAUGAUUCCGC

HT and/or WT : about 65°C

GCAUACGUCUUGAGAGGGAAAGCA

HT and/or WT : about 50°C to about 55°C

AGCUGGUUGGCGGGGUAAAGGCCCACCA

HT and/or WT : about 60°C.

6. Process for detecting Neisseria strains in a biological sample from other bacterial strains, wherein said process comprises contacting said biological sample in which the nucleic acids (DNAs and RNAs) of the strains have been made accessible to hybridization, if need be, under suitable denaturation conditions with a probe according to any one of claims 1 to 5 under conditions enabling hybridization between the probe and complementary nucleic acids of the Neisseria strains, which may be present in the sample, and detecting the hybrids possibly formed.

7. Process for detecting Neisseria strains from other bacterial strains in a biological sample, according to claim 6, wherein the probes used are the ones hybridizing both with DNA and RNA of Neisseria strains which may be present in the biological sample.

8. Process for detecting Neisseria strains from other bacterial strains, according to claim 6 or 7, wherein the hybridization medium contains about 3xSSC, (SSC = 0,15M NaCl, 0,015M sodium citrate, pH 7.0) about 25mM of phosphate buffer pH 7.1, 20% deionized formamide, 0.02% ficoll, 0.02% bovine serum albumin, 0.02% polyvinylpyrrolidone, and about 0.1 mg/ml sheared, denatured salmon sperm DNA, or the wash medium contains about 3XSSC, 25 mM phosphate buffer pH 7.1, and 20% deionized formamide, and
wherein the probe used is
anyone of the probes (1), (1bis), (1ter) or (1quater) of claim 2, the hybridization temperature being suitably adjusted to the range of about 55°C, preferably of about 53°C, and/or the wash temperature to the range of about 55°C, preferably of about 53°C, or anyone of the probes (2), (2bis), (2ter) or (2quater) of claim 2, the hybridization temperature being suitably adjusted to the range of about 60°C and/or the wash temperature to the range of about 60°C, or anyone of the probes (3), (3bis), (3ter) or (3quater) of claim 2, the hybridization temperature being suitably adjusted to the range of about 60°C and/or the wash temperature to the range of about 60°C, or anyone of the probes (4), (4bis), (4ter) or (4quater) of claim 1, the hybridization temperature being suitably adjusted to the range of about 65°C and/or the wash temperature to the range of about 65°C, or anyone of the probes (5), (5bis), (5ter) or (5quater) of claim 1, the hybridization temperature being suitably adjusted to the range of about 55°C and/or the wash temperature to the range of about 55°C, or anyone of the probes (9), (9bis), (9ter) or (9quater) of claim 1, the hybridization temperature being suitably adjusted to the range of about 60°C and/or the wash temperature to the range of about 60°C, or anyone of the probes (6), (6bis), (6ter) or (6quater) of claim 1, the hybridization temperature being suitably adjusted to the range of about 65°C and/or the wash temperature to the range of about 65°C to about 70°C,

or anyone of the probes (7), (7bis), (7ter) or (7 quater) of claim 1, the hybridization temperature being suitably adjusted to the range of about 55°C and/or the wash temperature to the range of about 55°C, or anyone of the probes (8), (8bis), (8ter) or (8quater) of claim 1, the hybridization temperature being suitably adjusted to the range of about 55°C and/or the wash temperature to the range of about 55°C to about 60°C, or anyone of the probes (10), (10bis), (10ter) or (10quater) of claim 1, the hybridization temperature being suitably adjusted to the range of about 55°C and/or the wash temperature to the range of about 55°C to about 60°C

or anyone of the probes (11), (11bis), (11ter) or (11quater) of claim 1, the hybridization temperature being suitably adjusted to about 55°C and/or the wash temperature to about 55°C.

or anyone of the probes (12), (12bis), (12ter) or (12quater) of claim 1, the hybridization temperature being suitably adjusted to range of about 55°C to about 60°C and/or the wash temperature to range of about 55°C to about 60°C.

or anyone of the probes (13), (13bis), (13ter) or (13quater) of claim 1, the hybridization temperature being suitably adjusted to range of about 45°C and/or the wash temperature to range of about 45°C.

or anyone of the probes (14), (14bis), (14ter) or (14quater) of claim 1, the hybridization temperature being suitably adjusted to range of about 40°C to about 45°C and/or the wash temperature to range of about 40°C to about 45°C.

or anyone of the probes (15), (15bis), (15ter) or (15quater) of claim 1, the hybridization temperature being suitably adjusted to range of about 50°C to about 55°C and/or the wash temperature to range of about 50°C to about 55°C.

or anyone of the probes (16), (16bis), (16ter) or (16quater) of claim 1, the hybridization temperature being suitably adjusted to range of about 50°C to about 60°C and/or the wash temperature to range of about 50°C to about 60°C.

or anyone of the probes (17), (17bis), (17ter) or (17quater) of claim 1, the hybridization temperature being suitably adjusted to range of about 50°C to about 55°C and/or the wash temperature to range of about 50°C to about 55°C.

or anyone of the probes (18), (18bis), (18ter) or (18quater) of claim 1, the hybridisation temperature being suitably adjusted to range of about 45°C and/or the wash temperature to range of about 45°C.

9. Process for detecting Neisseria gonorrhoeae strains from other bacterial strains and particularly from other Neisseria strains, wherein said process comprises contacting said biological sample, in which the nucleic acids (DNAs and RNAs) have been made accessible to hybridization, if need be, under suitable denaturation conditions, with a probe of the invention specific for Neisseria gonorrhoeae strains and selected from the probes (1), (1bis), (1ter), (1quater), (2), (2bis), (2ter), 2(quater), (3), (3bis), (3ter), (3quater), (4), (4bis), (4ter), (4quater), (5), (5bis), (5ter), (5quater), (9), (9bis), (9ter), (9quater), (11), (11bis), (11ter), (11quater), (13), (13bis), (13ter), (13quater), (18), (18bis), (18ter) and (18quater) of claims 1 or 2, whenever required under hybridization and washing conditions adjusted such as to ensure specific hybridization with complementary nucleic acids of the Neisseria gonorrhoeae strains, which may be present in the sample, yet not with complementary DNA or RNA of other Neisseria species, and detecting the hybrids possibly formed.

10. Process for detecting N. gonorrhoeae strains from other bacterial strains and in particular from other Neisseria strains, according to claim 9,
wherein the hybridization medium contains about 3xSSC, (SSC = 0,15M NaCl, 0,015M sodium citrate, pH 7.0) about 25mM of phosphate buffer pH 7.1, 20% deionized formamide, 0.02% ficoll, 0.02% bovine serum albumin, 0.02% polyvinylpyrrolidone, and about 0.1 mg/ml sheared, denatured salmon sperm DNA, or the wash medium contains about 3XSSC, 25 mM phosphate buffer pH 7.1, and 20% deionized formamide, and
wherein the probe used is anyone of the probes (1), (1bis), (1ter) or (1quater) of claim 2,
the hybridization temperature being suitably adjusted to the range of about 55°C, preferably of about 53°C, and/or the wash temperature to the range from about 55°C to about 65°C, preferably from about 53°C to about 65°C and preferably of about 53°C, and the probe used is
anyone of the probes (2), (2bis), (2ter) or (2quater) of claim 2, the hybridization temperature being suitably adjusted to the range of about 60°C and/or the wash temperature to the range of about 65°C to about 70°C, or anyone of the probes (3), (3bis), (3ter) or (3quater) of claim 2,

the hybridization temperature being suitably adjusted to the range of about 60°C and/or the wash temperature to the range of about 65°C to about 70°C,

or anyone of the probes (4), (4bis), (4ter) or (4quater) of claim 1, the hybridization temperature being suitably adjusted to the range of about 65°C and/or the wash temperature to the range of about 70°C to about 75°C,

or anyone of the probes (5), (5bis), (5ter) or (5quater) of claim 1, the hybridization temperature being suitably adjusted to the range of about 60°C and/or the wash temperature to the range of about 65°C, or anyone of the probes (9), (9bis), (9ter) or (9quater) of claim 1, the hybridization temperature being suitably adjusted to the range of about 60°C to about 65°C and/or the wash temperature to the range of about 65°C,

or more preferably anyone of the probes (1), (1bis), (1ter) or (1quater) of claim 2,

the hybridization temperature being suitably adjusted to the range of about 55°C and/or the wash temperature to the range of about 55°C to about 65°C

or anyone of the probes (11), (11bis), (11ter) or (11quater) of claim 1, the hybridization temperature being suitably adjusted to about 65°C and/or the wash temperature to about 65°C

or anyone of the probes (13), (13bis), (13ter) or (13quater) of claim 1, the hybridization temperature being suitably adjusted to range about 50°C to about 55°C and/or the wash temperature to range about 50°C to about 55°C,

or anyone of the probes (18), (18bis), (18ter) or (18quater) of claim 1, the hybridization temperature being suitably adjusted to about 60°C and/or the wash temperature to about 60°C.

11. Kit for the detection in vitro of Neisseria strains in a biological sample, said kit containing :
   - at least one probe selected among any of those according to claims 1 to 4 ;
   - the buffer or components necessary for producing the buffer enabling hybridization reaction between these probes and the DNAs and/or RNAs of a large number, preferably all strains of Neisseria to be carried out ;
   - when appropriate means for detecting the hybrids resulting from the preceding hybridization.

12. Kit for the detection in vitro of Neisseria gonorrhoeae strains in a biological sample, said kit containing :
   - at least one probe specific for N. gonorrhoeae as above defined, e.g. a probe selected from the probes (2), (2bis), (2ter), (2quater), (3), (3bis), (3ter), (3quater), (4), (4bis), (4ter), (4quater), (5), (5bis), (5ter), (5quater), (9), (9bis), (9ter), (9quater), (11), (11bis), (11ter), (11quater), (13), (13bis), (13ter), (13quater), (18), (18bis), (18ter) and (18quater), according to claims 1 or 2, or more preferably a probe selected from anyone of the probes (1), (1bis), (1ter) or (1quater) according to claim 2 ;
   - the buffer or components necessary for producing the buffer enabling hybridization reaction between these probes and the DNAs and/or RNAs of a strain of Neisseria gonorrhoeae to be carried out,
   - when appropriate means for detecting the hybrids resulting from the preceding hybridization.

13. Process for detecting N. gonorrhoeae strains from other bacterial strains and in particular from other Neisseria strains, wherein said process comprises contacting said biological sample, in which the nucleic acids (DNA and RNAs) have been made accessible to hybridization, if need be, under suitable denaturation conditions, with two probes of anyone of claims 1 to 5, which are specific for Neisseria gonorrhoeae strains and are respectively not able to hybridize to the same non-target strains, and in particular, selected from the following combinations:
   - probe of group 9 and anyone of the probes of the following groups: 1, 2, 3, 5 and 13.
   - probe of group 13 and anyone of the probes of the following groups: 1, 2, 3 and 5.
   - probe of group 18 and anyone of the probes of the following groups: 1, 2, 3, 5, 9 and 13.
   - probe of group 5 and anyone of the probes of the following groups: 1, 2 and 3, and more particularly, selected from the following combinations:
   - probe of group 9 and one of group 5.
   - probe of group 18 and one of the probes of the following groups: 5 and 9,

whenever required under hybridization and washing conditions adjusted such as to ensure specific hybridization with complementary nucleic acids of the Neisseria gonorrhoeae strains, which may be present in the sample, yet not with complementary DNA or RNA of other Neisseria species, and detecting the hybrids possibly formed.

**14.** Kit for sandwich hybridization assay, for the detection <u>in</u> <u>vitro</u> of <u>Neisseria</u> <u>gonorrhoeae</u> strains in a biological sample,
said kit containing :
- at least two probes specific for <u>N.</u> <u>gonorrhoeae</u> selected from the following combinations:
- probe of group 9 and anyone of the probes of the following groups: 1, 2, 3, 5 and 13.
- probe of group 13 and anyone of the probes of the following groups: 1, 2, 3 and 5.
- probe of group 18 and anyone of the probes of the following groups: 1, 2, 3, 5, 9 and 13.
- probe of group 5 and anyone of the probes of the following groups: 1, 2 and 3. and more particularly from the following combinations:
- probe of group 9 and one of group 5.
- probe of group 18 and one of the probes of the following groups: 5, and 9
- the buffer or components necessary for producing the buffer enabling hybridization reaction between these probes and the DNAs and/or RNAs of a strain of <u>Neisseria</u> <u>gonorrhoeae</u> to be carried out,
- when appropriate means for detecting the hybrids resulting from the preceding hybridization.

**Patentansprüche**

**1.** Sonde zum Nachweisen eines oder mehrerer Neisseria-Stämme, umfassend:
- entweder eine Sequenz, die zu einer Nucleinsäure aus den folgenden Gruppen von Nucleinsäuren gehört, und welche 10 bis zur maximalen Anzahl von Nucleotiden der ausgewählten Nucleinsäure umfasst:

```
GGTACCGTCATCGGCCGCCGATATTGGCAACAGCCTTTTCTTCCCTGACA
AAAGTCC
(4)
GGUACCGUCAUCGGCCGCCGAUAUUGGCAACAGCCUUUUCUUCCCUGACA
AAAGUCC
(4bis)
GGACTTTTGTCAGGGAAGAAAAGGCTGTTGCCAATATCGGCGGCCGATGA
CGGTACC
(4ter)
GGACUUUUGUCAGGGAAGAAAAGGCUGUUGCCAAUAUCGGCGGCCGAUGA
CGGUACC
(4quater)
ACGCTACCAAGCAATCAAGTTGCCCAACAGCTAA
(5)
ACGCUACCAAGCAAUCAAGUUGCCCAACAGCUAA
(5bis)
TTAGCTGTTGGGCAACTTGATTGCTTGGTAGCGT
(5ter)
UUAGCUGUUGGGCAACUUGAUUGCUUGGUAGCGU
(5quater)
```

ATACCGTGGTAAGCGGACTCCTTGCGGTTACCCTACCTACTTCTGGTATC
CCCCAC

(6)

AUACCGUGGUAAGCGGACUCCUUGCGGUUACCCUACCUACUUCUGGUAUC
CCCCAC

(6bis)

GTGGGGGATACCAGAAGTAGGTAGGGTAACCGCAAGGAGTCCGCTTACCA
CGGTAT

(6ter)

GUGGGGGAUACCAGAAGUAGGUAGGGUAACCGCAAGGAGUCCGCUUACCA
CGGUAU

(6quater)

TCAGTCCGATTTCCGCCGGACCTAGGT

(7)

UCAGUCCGAUUUCCGCCGGACCUAGGU

(7bis)

ACCTAGGTCCGGCGGAAATCGGACTGA

(7ter)

ACCUAGGUCCGGCGGAAAUCGGACUGA

(7 quater)

TCAGTCCGATTTCCGACCGGACCTAGGT

(8)

UCAGUCCGAUUUCCGACCGGACCUAGGU

(8bis)

ACCTAGGTCCGGTCGGAAATCGGACTGA

(8ter)

ACCUAGGUCCGGUCGGAAAUCGGACUGA

(8quater)

CGCCACCCGAGAAGCAAGCTTCCCTGTGCTGC

(9)

CGCCACCCGAGAAGCAAGCUUCCCUGUGCUGC

(9bis)

GCAGCACAGGGAAGCTTGCTTCTCGGGTGGCG

(9ter)

GCAGCACAGGGAAGCUUGCUUCUCGGGUGGCG

(9quater)

CGGGGCTTACGGAGCAAGTCCTTAACCTTAGAGGGCATA

(10)

CGGGGCUUACGGAGCAAGUCCUUAACCUUAGAGGGCAUA

(10bis)

TATGCCCTCTAAGGTTAAGGACTTGCTCCGTAAGCCCCG

(10ter)

UAUGCCCUCUAAGGUUAAGGACUUGCUCCGUAAGCCCCG

(10quater)

GCGGAATCATAGCTTTATTGCCAGCTCCCCCGC

(11)

GCGGAAUCAUAGCUUUAUUGCCAGCUCCCCCGC

(11bis)

GCGGGGGAGCTGGCAATAAAGCTATGATTCCGC

(11ter)

GCGGGGGAGCUGGCAAUAAAGCUAUGAUUCCGC

(11quater)

GACACACTCGAGTCACCCAGTTCAGAAC

(12)

GACACACUCGAGUCACCCAGUUCAGAAC

(12bis)

GTTCTGAACTGGGTGACTCGAGTGTGTC

(12ter)

GUUCUGAACUGGGUGACUCGAGUGUGUC

(12quater)

TGCTTTCCCTCTCAAGACGTATGC

(13)

UGCUUUCCCUCUCAAGACGUAUGC

(13bis)

GCATACGTCTTGAGAGGGAAAGCA

(13ter)

GCAUACGUCUUGAGAGGGAAAGCA

(13quater)

```
TCTCGACAGTTATTACGTACA
(14)
UCUCGACAGUUAUUACGUACA
(14bis)
TGTACGTAATAACTGTCGAGA
(14ter)
UGUACGUAAUAACUGUCGAGA
(14quater)
TTTCGTACGCTTAGTACCGCTGTTGAGA
(15)
UUUCGUACGCUUAGUACCGCUGUUGAGA
(15bis)
TCTCAACAGCGGTACTAAGCGTACGAAA
(15ter)
UCUCAACAGCGGUACUAAGCGUACGAAA
(15quater)
GTGGTATCGGTTGCTTCGTGTCCGTAGACA
(16)
GUGGUAUCGGUUGCUUCGUGUCCGUAGACA
(16bis)
TGTCTACGGACACGAAGCAACCGATACCAC
(16ter)
UGUCUACGGACACGAAGCAACCGAUACCAC
(16quater)
AAGCTATTCCAACAGCTTGCCAACCTAA
(17)
AAGCUAUUCCAACAGCUUGCCAACCUAA
(17bis)
TTAGGTTGGCAAGCTGTTGGAATAGCTT
(17ter)
UUAGGUUGGCAAGCUGUUGGAAUAGCUU
(17quater)
TGGTGGGCCTTTACCCCGCCAACCAGCT
(18)
```

UGGUGGGCCUUUACCCCGCCAACCAGCU

(18bis)

AGCTGGTTGGCGGGGTAAAGGCCCACCA

(18ter)

AGCUGGUUGGCGGGGUAAAGGCCCACCA

(18quater)

unter der Voraussetzung, dass die Sonde nicht aus der folgenden Sequenz: TCA TCG GCC GCC GAT ATT GGC besteht,

- oder eine Sequenzvariante, die sich von irgendeiner der vorhergehenden Sequenzen (4) bis (18) entweder durch Hinzufügung oder Entfernung eines oder mehrerer Nucleotide von einem ihrer entsprechenden Enden oder durch Ändern eines oder mehrerer Nucleotide innerhalb irgendeiner der Sequenzen oder durch beides unterscheidet, jedoch unter der Voraussetzung, dass bei jeder der zuvor beschriebenen Tatsachen die Sonde noch mit der gleichen Ziel-RNA- oder -DNA wie die entsprechende unmodifizierte Sequenz hybridisiert.

2. Sonde nach Anspruch 1, umfassend:
entweder eine Sequenz, die zu einer Nucleinsäure aus den folgenden Gruppen von Nucleinsäuren gehört, und die 10 bis zur maximalen Anzahl von Nucleotiden der ausgewählten Nucleinsäure umfasst:

TCGGCCGCCGATATTGGCAACAGCCTT (1)

UCGGCCGCCGAUAUUGGCAACAGCCUU (1bis)

AAGGCTGTTGCCAATATCGGCGGCCGA (1ter)

AAGGCUGUUGCCAAUAUCGGCGGCCGA (1quater)

TCGGCCGCCGATATTGGCAACAGCCTTTTCTTCCCTG (2)

UCGGCCGCCGAUAUUGGCAACAGCCUUUUCUUCCCUG (2bis)

CAGGGAAGAAAAGGCTGTTGCCAATATCGGCGGCCGA (2ter)

CAGGGAAGAAAAGGCUGUUGCCAAUAUCGGCGGCCGA (2quater)

GGTACCGTCATCGGCCGCCGATATTGGCAACAGCCTTTTCTTCCCTG

(3)

GGUACCGUCAUCGGCCGCCGAUAUUGGCAACAGCCUUUUCUUCCCUG

(3bis)

CAGGGAAGAAAAGGCTGTTGCCAATATCGGCGGCCGATGACGGTACC

(3ter)

CAGGGAAGAAAAGGCUGUUGCCAAUAUCGGCGGCCGAUGACGGUACC

(3quater)

unter der Voraussetzung, dass die Sonde nicht aus der nachfolgenden Sequenz: TCA TCG GCC GCC GAT ATT GGC besteht,
oder eine Sequenzvariante, die sich von irgendeiner der vorhergehenden Sequenzen (1) bis (18) entweder durch Hinzufügung oder Entfernung eines oder mehrerer Nucleotide von einem ihrer entsprechenden Extremitäten oder durch Ändern eines oder mehrerer Nucleotide innerhalb irgendeiner der Sequenzen oder durch beides unterscheidet, jedoch unter der Voraussetzung, dass bei jedem der zuvor beschriebenen Sachverhalte die Sonde noch mit der gleichen Ziel-RNA oder -DNA wie die entsprechende unmodifizierte Sequenz hybridisiert.

3. Sonde zum Nachweisen eines oder mehrerer Neisseria-Stämme, welche eine der im nachfolgenden definierten Sequenzen oder die entsprechende Komplementärsequenz zum Ziel hat, wenn das Hybridisierungsmedium oder das Waschmedium oder beides, wenn angemessen, folgende sind:

Hybridisierungsmedium: enthält etwa 3 x SSC, (SSC = 0,15 M NaCl, 0,015 M Natriumcitrat, pH 7,0), etwa 25 mM Phosphatpuffer pH 7,1, 20 % entionisiertes Formamid, 0,02 5 Ficoll, 0,02 % Rinderserumalbumin, 0,02 % Polyvinylpyrrolidon und etwa 0,1 mg/ml zerkleinerte, denaturierte Lachsspermium-DNA,

Waschmedium: enthält etwa 3 x SSC, 25 mM Phosphatpuffer pH 7,1, und 20 % entionisiertes Formamid, wobei die Zielsequenzen und die entsprechenden relevanten Hybridisierungstemperaturen (HT) und Waschtemperaturen (WT) folgende sind:

```
AAGGCUGUUGCCAAUAUCGGCGGCCGA
HT und/oder WT : etwa 55°C
CAGGGAAGAAAAGGCUGUUGCCAAUAUCGGCGGCCGA
HT und/oder WT : etwa 60°C
CAGGGAAGAAAAGGCUGUUGCCAAUAUCGGCGGCCGAUGACGGUACC
HT und/oder WT : etwa 60°C
GGACUUUUGUCAGGGAAGAAAAGGCUGUUGCCAAUAUCGGCGGCCGAUGA
CGGUACC
HT und/oder WT : etwa 65°C
UUAGCUGUUGGGCAACUUGAUUGCUUGGUAGCGU
HT und/oder WT : etwa 55°C bis etwa 60°C
GUGGGGGAUACCAGAAGUAGGUAGGGUAACCGCAAGGAGUCCGCUUACCA
CGGUAU
HT und/oder WT : etwa 65°C bis etwa 70°C
ACCUAGGUCCGGCGGAAAUCGGACUGA
HT und/oder WT : etwa 55°C
ACCUAGGUCCGGUCGGAAAUCGGACUGA
HT und/oder WT : etwa 55°C bis etwa 60°C
GCAGCACAGGGAAGCUUGCUUCUCGGGUGGCG
HT und/oder WT : etwa 55°C bis etwa 60°C
```

UAUGCCCUCUAAGGUUAAGGACUUGCUCCGUAAGCCCCG

HT und/oder WT : etwa 60°C bis etwa 65°C

GCGGGGGAGCUGGCAAUAAAGCUAUGAUUCCGC

HT und/oder WT : etwa 55°C

GUUCUGAACUGGGUGACUCGAGUGUGUC

HT und/oder WT : etwa 55°C bis etwa 60°C

GCAUACGUCUUGAGAGGGAAAGCA

HT und/oder WT : etwa 45°C

UGUACGUAAUAACUGUCGAGA

HT und/oder WT : etwa 40°C bis etwa 45°C

UCUCAACAGCGGUACUAAGCGUACGAAA

HT und/oder WT : etwa 50°C bis etwa 55°C

UGUCUACGGACACGAAGCAACCGAUACCAC

HT und/oder WT : etwa 50°C bis etwa 60°C

UUAGGUUGGCAAGCUGUUGGAAUAGCUU

HT und/oder WT : etwa 50°C bis etwa 55°C

AGCUGGUUGGCGGGGUAAAGGCCCACCA

HT und/oder WT : etwa 45°C

4. Sonde zum Nachweisen eines oder mehrerer Neisseria ghonorrhoeae-Stämme aus anderen Bakterienstämmen und insbesondere aus anderen Neisseria-Stämmen, enthaltend entweder eine Sequenz, die zu einer Nucleinsäure aus den folgenden Gruppen von Nucleinsäuren gehört und welche 10 bis zur maximalen Anzahl an Nucleotiden der ausgewählten Nucleinsäure umfasst:

TCGGCCGCCGATATTGGCAACAGCCTT (1)

UCGGCCGCCGAUAUUGGCAACAGCCUU (1bis)

AAGGCTGTTGCCAATATCGGCGGCCGA (1ter)

AAGGCUGUUGCCAAUAUCGGCGGCCGA (1quater)

TCGGCCGCCGATATTGGCAACAGCCTTTTCTTCCCTG (2)

UCGGCCGCCGAUAUUGGCAACAGCCUUUUCUUCCCUG (2bis)

CAGGGAAGAAAAGGCTGTTGCCAATATCGGCGGCCGA (2ter)

CAGGGAAGAAAAGGCUGUUGCCAAUAUCGGCGGCCGA (2quater)

GGTACCGTCATCGGCCGCCGATATTGGCAACAGCCTTTTCTTCCCTG

(3)

GGUACCGUCAUCGGCCGCCGAUAUUGGCAACAGCCUUUUCUUCCCUG

(3bis)

CAGGGAAGAAAAGGCTGTTGCCAATATCGGCGGCCGATGACGGTACC

(3ter)

CAGGGAAGAAAAGGCUGUUGCCAAUAUCGGCGGCCGAUGACGGUACC

(3quater)

GGTACCGTCATCGGCCGCCGATATTGGCAACAGCCTTTTCTTCCCTGACA

AAAGTCC

(4)

GGUACCGUCAUCGGCCGCCGAUAUUGGCAACAGCCUUUUCUUCCCUGACA

AAAGUCC

(4bis)

GGACTTTTGTCAGGGAAGAAAAGGCTGTTGCCAATATCGGCGGCCGATGA

CGGTACC

(4ter)

GGACUUUUGUCAGGGAAGAAAAGGCUGUUGCCAAUAUCGGCGGCCGAUGA

CGGUACC

(4quater)

ACGCTACCAAGCAATCAAGTTGCCCAACAGCTAA (5)

ACGCUACCAAGCAAUCAAGUUGCCCAACAGCUAA (5bis)

TTAGCTGTTGGGCAACTTGATTGCTTGGTAGCGT (5ter)

UUAGCUGUUGGGCAACUUGAUUGCUUGGUAGCGU (5quater)

CGCCACCCGAGAAGCAAGCTTCCCTGTGCTGC (9)

CGCCACCCGAGAAGCAAGCUUCCCUGUGCUGC (9bis)

GCAGCACAGGGAAGCTTGCTTCTCGGGTGGCG (9ter)

GCAGCACAGGGAAGCUUGCUUCUCGGGUGGCG (9quater)

GCGGAATCATAGCTTTATTGCCAGCTCCCCCGC (11)

GCGGAAUCAUAGCUUUAUUGCCAGCUCCCCCGC (11bis)

GCGGGGGAGCTGGCAATAAAGCTATGATTCCGC (11ter)

GCGGGGGAGCUGGCAAUAAAGCUAUGAUUCCGC (11quater)

44

```
TGCTTTCCCTCTCAAGACGTATGC (13)

UGCUUUCCCUCUCAAGACGUAUGC (13bis)

GCATACGTCTTGAGAGGGAAAGCA (13ter)

GCAUACGUCUUGAGAGGGAAAGCA (13quater)

TGGTGGGCCTTTACCCCGCCAACCAGCT (18)

UGGUGGGCCUUUACCCCGCCAACCAGCU (18bis)

AGCTGGTTGGCGGGGTAAAGGCCCACCA (18ter)

AGCUGGUUGGCGGGGUAAAGGCCCACCA (18quater)
```

unter der Voraussetzung, dass die Sonde nicht aus der folgenden Sequenz: TCA TCG GCC GCC GAT ATT GGC besteht,

oder eine Sequenzvariante, die sich von irgendeiner der vorhergehenden Sequenzen (1) bis (18) entweder durch Hinzufügung oder Entfernung eines oder mehrerer Nucleotide von einem ihrer entsprechenden Enden oder durch Ändern eines oder mehrerer Nucleotide innerhalb irgendeiner der Sequenzen oder durch beides unterscheidet, jedoch unter der Voraussetzung, dass bei jedem der vorher beschriebenen Sachverhalte die Sonde noch mit der gleichen Ziel-RNA oder -DNA wie die entsprechende unmodifizierte Sequenz hybridisert.

5. Sonde zum Nachweisen eines oder mehrerer Neisseria gonorrhoeae-Stämme aus anderen Bakterienstämmen und insbesondere aus anderen Neisseria-Stämmen, welche eine der im nachfolgenden definierten Sequenzen oder die entsprechende

Komplementärsequenz zum Ziel hat, wenn das Hybridisierungsmedium oder das Waschmedium oder beides, wenn angemessen, folgende sind:

Hybridisierungsmedium: enthält etwa 3 x SSC, (SSC = 0,15 M NaCl, 0,015 M Natriumcitrat, pH 7,0), etwa 25 mM Phosphatpuffer mit pH 7,1, 20 % entionisiertes Formamid, 0,02 % Ficoll, 0,02 % Rinderserumalbumin, 0,02 % Polyvinylpyrrolidon und etwa 0,1 mg/ml zerkleinerte, denaturierte Lachspermium-DNA,

Waschmedium: enthält etwa 3 x SSC, 25 mM Phosphatpuffer mit pH 7,1 und 20 % entionisiertes Formamid, wobei die Zielsequenzen und die entsprechenden relevanten Hybridisierungstemperaturen (HT) und Waschtemperaturen (WT) folgende sind:

AAGGCUGUUGCCAAUAUCGGCGGCCGA

HT und/oder WT : etwa 50°C bis etwa 65°C

CAGGGAAGAAAAGGCUGUUGCCAAUAUCGGCGGCCGA

HT und/oder WT : etwa 60°C bis etwa 70°C

CAGGGAAGAAAAGGCUGUUGCCAAUAUCGGCGGCCGAUGACGGUACC

HT und/oder WT : etwa 65°C bis etwa 70°C

GGACUUUUGUCAGGGAAGAAAAGGCUGUUGCAAUAUCGGCGGCCGAUGAC
GGUACC

HT und/oder WT : etwa 70°C bis etwa 75°C

UUAGCUGUUGGGCAACUUGAUUGCUUGGUAGCGU

HT und/oder WT : etwa 65°C

GCAGCACAGGGAAGCUUGCUUCUCGGGUGGCG

HT und/oder WT : etwa 65°C

GCGGGGGAGCUGGCAAUAAAGCUAUGAUUCCGC

HT und/oder WT : etwa 65°C

GCAUACGUCUUGAGAGGGAAAGCA

HT und/oder WT : etwa 50°C bis etwa 55°C

AGCUGGUUGGCGGGGUAAAGGCCCACCA

HT und/oder WT : etwa 60°C.

6. Verfahren zum Nachweisen von Neisseria-Stämmen in einer biologischen Probe aus anderen Bakterienstämmen, wobei das Verfahren Inkontaktbringen der biologischen Probe, in der die Nucleinsäuren (DNAs und RNAs) der Stämme, erforderlichenfalls unter geeigneten Denaturierungsbedingungen der Hybridisierung zugänglich gemacht worden sind, mit einer Sonde gemäss einem der Ansprüche 1 bis 5 unter Bedingungen, die Hybridisierung zwischen der Sonde und komplementären Nucleinsäuren der Neisseria-Stämme, die in der Probe vorhanden sein können, ermöglicht, und Nachweisen der möglicherweise gebildeten Hybride umfasst.

7. Verfahren zum Nachweisen von Neisseria-Stämmen aus anderen Bakterienstämmen in einer biologischen Probe gemäss Anspruch 6, wobei die verwendeten Sonden derartige sind, die sowohl mit DNA wie auch mit RNA von Neisseria-Stämmen, welche in der biologischen Probe vorhanden sein können, hybridisieren.

8. Verfahren zum Nachweisen von Neisseria-Stämmen aus anderen Bakterienstämmen gemäss einem der Ansprüche 6 oder 7, wobei das
Hybridisierungsmedium etwa 3 x SSC (SSC = 0,15 M NaCl, 0,015 M Natriumcitrat, pH 7,0), etwa 25 mM Phosphatpuffer mit pH 7,1, 20 % entionisiertes Formamid, 0,02 % Ficoll, 0,02 % Rinderserumalbumin, 0,02 % Polyvinylpyrrolidon und etwa 0,1 mg/ml zerkleinerte denaturierte Lachsspermium-DNA enthält, oder das Waschmedium etwa 3 x SSC, 25 mM Phosphatpuffer mit pH 7,1, und 20 % entionisiertes Formamid enthält, und wobei die verwendete Sonde folgende ist: irgendeine der Sonden (1), (1bis), (1ter) oder (1quater) des Anspruchs 2, wobei die Hybridisierungstemperatur geeignetermassen auf den Bereich um etwa 55°C, vorzugsweise um etwa 53°C eingestellt wird, und/oder die Waschtemperatur auf den Bereich von etwa 55°C, vorzugsweise etwa 53°C, eingestellt wird, oder irgendeine der Sonden (2), (2bis), (2ter) oder (2quater) des Anspruchs 2, wobei die Hybridisierungstemperatur geeignetermassen auf den Bereich um etwa 60°C eingestellt wird, und/oder die Waschtemperatur auf den Bereich um etwa 60°C eingestellt wird, oder irgendeine der Sonden (3), (3bis), (3ter) oder (3quater) des Anspruchs 2, wobei die Hybridisierungstemperatur geeignetermassen auf den Bereich um etwa 60°C eingestellt wird, und/oder die Waschtemperatur auf den Bereich um etwa 60°C eingestellt wird, oder irgendeine der Sonden (4), (4bis), (4ter) oder (4quater) des Anspruchs 1, wobei die Hybridisierungstemperatur geeignetermassen auf den Bereich um etwa 65°C eingestellt wird, und/oder die Waschtemperatur auf den Bereich um etwa 65°C eingestellt wird, oder irgendeine der Sonden (5), (5bis), (5ter) oder (5quater) des Anspruchs 1, wobei die Hybridisierungstemperatur geeignetermassen auf den Bereich um etwa 55°C eingestellt wird, und/oder die Waschtemperatur auf den Bereich um etwa 55°C eingestellt wird, oder irgendeine der Sonden (9), (9bis), (9ter) oder (9quater) des Anspruchs 1, wobei die Hybridisierungstemperatur geeignetermassen auf den Bereich um etwa 60°C eingestellt wird, und/oder die Waschtemperatur auf den Bereich um etwa 60°C eingestellt wird, oder irgendeine der Sonden (6), (6bis), (6ter) oder (6quater) des Anspruchs 1, wobei die Hybridisierungstemperatur geeignetermassen auf den Bereich um etwa 65°C eingestellt wird, und/oder die Waschtemperatur auf den Bereich von etwa 65°C bis etwa 70°C eingestellt wird, oder irgendeine der Sonden (7), (7bis), (7ter) oder (7quater) des Anspruchs 1, wobei die Hybridisierungstemperatur geeignetermassen auf etwa 55°C eingestellt wird, und/oder die Waschtemperatur auf den Bereich um etwa 55°C eingestellt wird, oder irgendeine der Sonden (8), (8bis), (8ter) oder (8quater) des Anspruchs 1, wobei die Hybridisierungstemperatur geeignetermassen auf den Bereich um etwa 55°C eingestellt wird, und/oder die Waschtemperatur auf den Bereich von etwa 55°C bis etwa 60°C eingestellt wird, oder irgendeine der Sonden (10), (10bis), (10ter) oder (10quater) des Anspruchs 1, wobei die Hybridisierungstemperatur geeignetermassen auf den Bereich um etwa 55°C eingestellt wird, und/oder die Waschtemperatur auf den Bereich von etwa 55°C bis etwa 60°C eingestellt wird, oder irgendeine der Sonden (11), (11bis), (11ter) oder (11quater) des Anspruchs 1, wobei die Hybridisierungstemperatur geeignetermassen auf den Bereich um etwa 55°C eingestellt wird, und/oder die Waschtemperatur auf etwa 55°C eingestellt wird, oder irgendeine der Sonden (12), (12bis), (12ter) oder (12quater) des Anspruchs 1, wobei die Hybridisierungstemperatur geeignetermassen auf den Bereich von etwa 55°C bis etwa 60°C eingestellt wird, und/oder die Waschtemperatur auf den Bereich von etwa 55°C bis etwa 60°C eingestellt wird, oder irgendeine der Sonden (13), (13bis), (13ter) oder (13quater) des Anspruchs 1, wobei die Hybridisierungstemperatur geeignetermassen auf den Bereich um etwa 45°C eingestellt wird, und/oder die Waschtemperatur auf den Bereich um etwa 45°C eingestellt wird, oder irgendeine der Sonden (14), (14bis), (14ter) oder (14quater) des Anspruchs 1, wobei die Hybridisierungstemperatur geeignetermassen auf den Bereich von etwa 40°C bis etwa 45°C eingestellt wird, und/oder die Waschtemperatur auf den Bereich von etwa 40°C bis etwa 45°C eingestellt wird, oder irgendeine der Sonden (15), (15bis), (15ter) oder (15quater) des Anspruchs 1, wobei die Hybridisierungstemperatur geeignetermassen auf den Bereich von etwa 50°C bis etwa 55°C eingestellt wird, und/oder die Waschtemperatur auf den Bereich von etwa 50°C bis etwa 55°C eingestellt wird, oder irgendeine der Sonden (16), (16bis), (16ter) oder (16quater) des Anspruchs 1, wobei die Hybridisierungstemperatur geeignetermassen auf den Bereich von etwa 50°C bis etwa 60°C eingestellt wird, und/oder die Waschtemperatur auf den Bereich von etwa 50°C bis etwa 60°C eingestellt wird, oder irgendeine der Sonden (17), (17bis), (17ter) oder (17quater) des Anspruchs 1, wobei die Hybridisierungstemperatur geeignetermassen auf den Bereich von etwa 50°C bis etwa 55°C eingestellt wird, und/oder die Waschtemperatur auf den Bereich von etwa 50°C bis etwa 55°C eingestellt wird, oder irgendeine der Sonden (18), (18bis), (18ter) oder (18quater) des Anspruchs 1, wobei die Hybridisierungstemperatur geeignetermassen auf den Bereich von etwa 45°C eingestellt wird, und/oder die Waschtemperatur auf den Bereich von etwa 45°C eingestellt wird.

9. Verfahren zum Nachweisen von Neisseria gonorrhoeae-Stämmen aus anderen Bakterienstämmen und insbesondere aus anderen Neisseria-Stämmen, wobei das Verfahren umfasst: Inkontaktbringen der biologischen Probe, in der die Nucleinsäuren (DNAs und RNAs), erforderlichenfalls unter geeigneten Denaturierungsbedingungen der Hybridisierung zugänglich gemacht worden sind, mit einer Sonde gemäss der Erfindung, die spezifisch für Neisseria gonorrhoeae-Stämme ist und aus den Sonden (1), (1bis), (1ter), (1quater), (2), (2bis), (2ter), (2quater), (3), (3bis), (3ter), (3quater), (4), (4bis), (4ter), (4quater), (5), (5bis), (5ter), (5quater), (9), (9bis), (9ter), (9quater), (11), (11bis), (11ter), (11quater), (13),

47

(13bis), (13ter), (13quater), (18), (18bis), (18ter) und (18quater) der Ansprüche 1 oder 2 ausgewählt ist, wann immer erforderlich, unter Hybridisierungs- und Waschbedingungen, die so eingestellt sind, dass sie spezifische Hybridisierung mit komplementären Nucleinsäuren der Neisseria gonorrhoeae-Stämme, die in der Probe vorhanden sein können, jedoch nicht mit komplementärer DNA oder RNA anderer Neisseria-Arten gewährleisten, und Nachweisen der möglicherweise gebildeten Hybride.

10. Verfahren zum Nachweisen von N. gonorrhoeae-Stämmen aus anderen Bakterienstämmen und insbesondere aus anderen Neisseria-Stämmen gemäss Anspruch 9, wobei das Hybridisierungsmedium etwa 3 x SSC (SSC = 0,15 M NaCl, 0,015 M Natriumcitrat, pH 7,0), etwa 25 mM Phosphatpuffer mit pH 7,1, 20 %-iges entionisiertes Formamid, 0,02 % Ficoll, 0,02 % Rinderserumalbumin, 0,02 % Polyvinylpyrrolidon und etwa 0,1 mg/ml zerkleinerte denaturierte Lachsspermium-DNA enthält, oder das Waschmedium etwa 3 x SSC, 25 mM Phosphatpuffer mit pH 7,1, und 20 %-iges entionisiertes Formamid enthält, und wobei die verwendete Sonde irgendeine der Sonden (1), (1bis), (1ter) oder (1quater) des Anspruchs 2 ist, wobei die Hybridisierungstemperatur geeignetermassen auf den Bereich um etwa 55°C, vorzugsweise um etwa 53°C eingestellt ist, und/oder die Waschtemperatur auf den Bereich von etwa 55°C bis etwa 65°C, vorzugsweise etwa 53°C bis etwa 65°C und bevorzugter etwa 53°C, eingestellt ist, und wobei die verwendete Sonde irgendeine der Sonden (2), (2bis), (2ter) oder (2quater) des Anspruchs 2 ist, wobei die Hybridisierungstemperatur geeignetermassen auf den Bereich um etwa 60°C eingestellt ist, und/oder die Waschtemperatur auf den Bereich von etwa 65°C bis etwa 70°C eingestellt ist, oder irgendeine der Sonden (3), (3bis), (3ter) oder (3quater) des Anspruchs 2 ist, wobei die Hybridisierungstemperatur geeignetermassen auf den Bereich von etwa 60°C eingestellt ist, und/oder die Waschtemperatur auf den Bereich von etwa 65°C bis etwa 70°C eingestellt ist, oder irgendeine der Sonden (4), (4bis), (4ter) oder (4quater) des Anspruchs 1 ist, wobei die Hybridisierungstemperatur geeignetermassen auf den Bereich um etwa 65°C eingestellt ist, und/oder die Waschtemperatur auf den Bereich von etwa 70°C bis etwa 75°C eingestellt ist, oder irgendeine der Sonden (5), (5bis), (5ter) oder (5quater) des Anspruchs 1 ist, wobei die Hybridisierungstemperatur geeignetermassen auf den Bereich um etwa 60°C eingestellt ist, und/oder die Waschtemperatur auf einen Bereich um etwa 65°C eingestellt ist, oder irgendeine der Sonden (9), (9bis), (9ter) oder (9quater) des Anspruchs 1 ist, wobei die Hybridisierungstemperatur geeignetermassen auf den Bereich von etwa 60°C bis etwa 65°C eingestellt wird, und/oder die Waschtemperatur auf einen Bereich um etwa 65°C eingestellt ist, oder bevorzugter irgendeine der Sonden (1), (1bis), (1ter) oder (1quater) des Anspruchs 2 ist, wobei die Hybridisierungstemperatur geeignetermassen auf einen Bereich um etwa 55°C eingestellt ist, und/oder die Waschtemperatur auf den Bereich um etwa 55°C bis etwa 65°C eingestellt ist, oder irgendeine der Sonden (11), (11bis), (11ter) oder (11quater) des Anspruchs 1 ist, wobei die Hybridisierungstemperatur geeignetermassen auf etwa 65°C eingestellt ist, und/oder die Waschtemperatur auf etwa 65°C eingestellt ist, oder irgendeine der Sonden (13), (13bis), (13ter) oder (13quater) des Anspruchs 1 ist, wobei die Hybridisierungstemperatur geeignetermassen auf den Bereich von etwa 50°C bis etwa 55°C eingestellt ist, und/oder die Waschtemperatur auf den Bereich von etwa 50°C bis etwa 55°C eingestellt ist, oder irgendeine der Sonden (18), (18bis), (18ter) oder (18quater) des Anspruchs 1 ist, wobei die Hybridisierungstemperatur geeignetermassen auf etwa 60°C eingestellt ist, und/oder die Waschtemperatur auf etwa 60°C eingestellt ist.

11. Kit zum in vitro-Nachweis von Neisseria-Stämmen in einer biologischen Probe, wobei das Kit umfasst:
mindestens eine Sonde, ausgewählt aus denjenigen gemäss Ansprüchen 1 bis 4;
den Puffer oder die Komponenten, die erforderlich sind, um den Puffer herzustellen, der die Durchführung der Hybridisierungsreaktion zwischen diesen Sonden und den DNAs und/oder RNAs einer grossen Anzahl, vorzugsweise sämtlicher Stämme von Neisseria ermöglicht;
wenn angemessen, Mittel zum Nachweisen der aus der vorhergehenden Hybridisierung stammenden Hybride.

12. Kit zum in vitro-Nachweis von Neisseria gonorrhoeae-Stämmen in einer biologischen Probe, wobei das Kit enthält:
mindestens eine im Hinblick auf N. gonorrhoeae spezifische Sonde, wie zuvor definiert, d.h. eine Sonde aus den Sonden (2), (2bis), (2ter), (2quater), (3), (3bis), (3ter), (3quater), (4), (4bis), (4ter), (4quater), (5), (5bis), (5ter), (5quater), (9), (9bis), (9ter), (9quater), (11), (11bis), (11ter), (11quater), (13), (13bis), (13ter), (13quater), (18), (18bis), (18ter) und (18quater) gemäss Ansprüchen 1 oder 2, oder bevorzugter eine Sonde aus irgendeiner der Sonden (1), (1bis), (1ter) oder (1quater) gemäss Anspruch 2;
den Puffer oder die Komponenten, die erforderlich sind, um den Puffer herzustellen, der die Durchführung der Hybridisierungsreaktion zwischen diesen Sonden und den DNAs und/oder RNAs eines Stammes von Neisseria gonorrhoeae ermöglicht, wenn angemessen, Mittel zum Nachweisen der aus der vorhergehenden Hybridisierung stammenden Hybride.

13. Verfahren zum Nachweisen von N. gonorrhoeae-Stämmen aus anderen Bakterienstämmen und insbesondere aus anderen Neisseria-Stämmen, wobei das Verfahren umfasst: Inberührungbringen der biologischen Probe, in der die Nucleinsäuren (DNA und RNAs), erforderlichenfalls unter geeigneten Denaturierungsbedingungen der Hybridisierung zugänglich gemacht worden sind, mit zwei Sonden gemäss irgendeinem der Ansprüche 1 bis 5, die im Hinblick auf Neisseria gonorrhoeae-Stämme spezifisch sind und dementsprechend nicht dazu geeignet sind, an die gleichen Nicht-Ziel-Stämme zu hybridisieren, und die insbesondere aus den folgenden Kombinationen ausgewählt sind:
Sonde der Gruppe 9 und irgendeine der Sonden der folgenden Gruppen: 1, 2, 3, 5 und 13;
Sonde der Gruppe 13 und irgendeine der Sonden der folgenden Gruppen: 1, 2, 3 und 5;
Sonde der Gruppe 18 und irgendeine der Sonden der folgenden Gruppen: 1, 2, 3, 5, 9 und 13;
Sonde der Gruppe 5 und irgendeine der Sonden der folgenden Gruppen: 1, 2 und 3;
und die bevorzugter aus den folgenden Kombinationen ausgewählt sind:
Sonde der Gruppe 9 und eine der Gruppe 5;
Sonde der Gruppe 18 und eine der Sonden der folgenden Gruppen: 5 und 9;
wann immer erforderlich, unter Hybridisierungs- und Waschbedingungen, die so eingestellt sind, dass sie spezifische Hybridisierung mit komplementären Nucleinsäuren der Neisseria gonorrhoeae-Stämme, die in der Probe vorhanden sein können, jedoch nicht mit komplementärer DNA oder RNA anderer Neisseria-Arten ermöglichen, und Nachweisen der möglicherweise geformten Hybride.

14. Kit für Sandwich-Hybridisierungs-Assay für den in vitro-Nachweis von Neisseria gonorrhoeae-Stämmen in einer biologischen Probe, wobei das Kit enthält:
mindestens zwei für N. gonorrhoeae spezifische Sonden aus den folgenden Kombinationen:
Sonde der Gruppe 9 und irgendeine der Sonden der folgenden Gruppen: 1, 2, 3, 5 und 13;
Sonde der Gruppe 13 und irgendeine der Sonden der folgenden Gruppen: 1, 2, 3 und 5;
Sonde der Gruppe 18 und irgendeine der Sonden der folgenden Gruppen: 1, 2, 3, 5, 9 und 13;
Sonde der Gruppe 5 und irgendeine der Sonden der folgenden Gruppen: 1, 2 und 3;
und bevorzugter aus den folgenden Kombinationen:
Sonde der Gruppe 9 und eine der Gruppe 5;
Sonde der Gruppe 18 und eine der Sonden der folgenden Gruppen: 5 und 9;
den Puffer oder die Komponenten, die erforderlich sind, um den Puffer herzustellen, der die Durchführung der Hybridisierungsreaktion zwischen diesen Sonden und den DNAs und/oder RNAs eines Neisseria gonorrhoeae-Stammes ermöglicht,
wenn angemessen, Mittel zum Nachweisen der aus der vorhergehenden Hybridisierung stammenden Hybride.

**Revendications**

1. Sonde pour détecter une ou plusieurs souches de Neisseria, contenant :
   - soit une séquence appartenant un acide nucléique choise dans les groupes suivants d'acides nucléiques et qui comprend elle-même de 10 au nombre maximum de nucléotides de l'acide nucléique sélectionné :

```
GGTACCGTCATCGGCCGCCGATATTGGCAACAGCCTTTTCTTCCCTGACAAAAGTCC
                                                (4)
GGUACCGUCAUCGGCCGCCGAUAUUGGCAACAGCCUUUUCUUCCCUGACAAAAGUCC
                                                (4bis)
GGACTTTTGTCAGGGAAGAAAAGGCTGTTGCCAATATCGGCGGCCGATGACGGTACC
                                                (4ter)
GGACUUUUGUCAGGGAAGAAAAGGCUGUUGCCAAUAUCGGCGGCCGAUGACGGUACC
                                                (4quater)
ACGCTACCAAGCAATCAAGTTGCCCAACAGCTAA              (5)
ACGCUACCAAGCAAUCAAGUUGCCCAACAGCUAA              (5bis)
TTAGCTGTTGGGCAACTTGATTGCTTGGTAGCGT              (5ter)
UUAGCUGUUGGGCAACUUGAUUGCUUGGUAGCGU              (5quater)
ATACCGTGGTAAGCGGACTCCTTGCGGTTACCCTACCTACTTCTGGTATCCCCCAC
                                                (6)
AUACCGUGGUAAGCGGACUCCUUGCGGUUACCCUACCUACUUCUGGUAUCCCCCAC
                                                (6bis)
GTGGGGGATACCAGAAGTAGGTAGGGTAACCGCAAGGAGTCCGCTTACCACGGTAT
                                                (6ter)
GUGGGGGAUACCAGAAGUAGGUAGGGUAACCGCAAGGAGUCCGCUUACCACGGUAU
                                                (6quater)
TCAGTCCGATTTCCGCCGGACCTAGGT                     (7)
UCAGUCCGAUUUCCGCCGGACCUAGGU                     (7bis)
ACCTAGGTCCGGCGGAAATCGGACTGA                     (7ter)
ACCUAGGUCCGGCGGAAAUCGGACUGA                     (7quater)
TCAGTCCGATTTCCGACCGGACCTAGGT                    (8)
UCAGUCCGAUUUCCGACCGGACCUAGGU                    (8bis)
ACCTAGGTCCGGTCGGAAATCGGACTGA                    (8ter)
ACCUAGGUCCGGUCGGAAAUCGGACUGA                    (8quater)
```

```
CGCCACCCGAGAAGCAAGCTTCCCTGTGCTGC          (9)
CGCCACCCGAGAAGCAAGCUUCCCUGUGCUGC          (9bis)
GCAGCACAGGGAAGCTTGCTTCTCGGGTGGCG     ·    (9ter)
GCAGCACAGGGAAGCUUGCUUCUCGGGUGGCG          (9quater)
CGGGGCTTACGGAGCAAGTCCTTAACCTTAGAGGGCATA   (10)
CGGGGCUUACGGAGCAAGUCCUUAACCUUAGAGGGCAUA   (10bis)
TATGCCCTCTAAGGTTAAGGACTTGCTCCGTAAGCCCCG   (10ter)
UAUGCCCUCUAAGGUUAAGGACUUGCUCCGUAAGCCCCG   (10quater)
GCGGAATCATAGCTTTATTGCCAGCTCCCCCGC         (11)
GCGGAAUCAUAGCUUUAUUGCCAGCUCCCCCGC         (11bis)
GCGGGGGAGCTGGCAATAAAGCTATGATTCCGC         (11ter)
GCGGGGGAGCUGGCAAUAAAGCUAUGAUUCCGC         (11quater)
GACACACTCGAGTCACCCAGTTCAGAAC              (12)
GACACACUCGAGUCACCCAGUUCAGAAC              (12bis)
GTTCTGAACTGGGTGACTCGAGTGTGTC              (12ter)
GUUCUGAACUGGGUGACUCGAGUGUGUC              (12quater)
TGCTTTCCCTCTCAAGACGTATGC                  (13)
UGCUUUCCCUCUCAAGACGUAUGC                  (13bis)
GCATACGTCTTGAGAGGGAAAGCA                  (13ter)
GCAUACGUCUUGAGAGGGAAAGCA                  (13quater)
TCTCGACAGTTATTACGTACA                     (14)
UCUCGACAGUUAUUACGUACA                     (14bis)
TGTACGTAATAACTGTCGAGA                     (14ter)
UGUACGUAAUAACUGUCGAGA                     (14quater)
TTTCGTACGCTTAGTACCGCTGTTGAGA              (15)
UUUCGUACGCUUAGUACCGCUGUUGAGA              (15bis)
TCTCAACAGCGGTACTAAGCGTACGAAA              (15ter)
UCUCAACAGCGGUACUAAGCGUACGAAA              (15quater)
GTGGTATCGGTTGCTTCGTGTCCGTAGACA            (16)
GUGGUAUCGGUUGCUUCGUGUCCGUAGACA            (16bis)
TGTCTACGGACACGAAGCAACCGATACCAC            (16ter)
UGUCUACGGACACGAAGCAACCGAUACCAC            (16quater)
AAGCTATTCCAACAGCTTGCCAACCTAA              (17)
AAGCUAUUCCAACAGCUUGCCAACCUAA              (17bis)
TTAGGTTGGCAAGCTGTTGGAATAGCTT              (17ter)
UUAGGUUGGCAAGCUGUUGGAAUAGCUU              (17quater)
```

```
TGGTGGGCCTTTACCCCGCCAACCAGCT              (18)

UGGUGGGCCUUUACCCCGCCAACCAGCU              (18bis)

AGCTGGTTGGCGGGGTAAAGGCCCACCA              (18ter)

AGCUGGUUGGCGGGGUAAAGGCCCACCA              (18quater)
```

à condition que la sonde ne consiste pas en la séquence suivante :
    TCA TCG GCC GCC GAT ATT GGC
-   soit une séquence variante qui se distingue de l'une quelconque des séquences précédentes (4) à (18) :
    .   par l'addition ou l'élimination d'un ou de plusieurs nucléotides au niveau de l'une quelconque de leurs extrémités respectives;
    .   ou par le changement d'un ou de plusieurs nucléotides à l'intérieur de l'une quelconque de ces séquences,
    .   ou par les deux,
    à condition encore que dans l'une quelconque des circonstances ci-dessus, cette sonde s'hybride encore avec la même cible ARN ou ADN que la séquence non modifiée correspondante.

2.  Sonde suivant la revendication 1, contenant :
    -   soit une séquence appartenant à un acide nucléique choisi parmi les groupes suivants d'acides nucléiques et qui comprend elle-même de 10 au nombre maximum de nucléotides de l'acide nucléique choisi :

```
TCGGCCGCCGATATTGGCAACAGCCTT                    (1)

UCGGCCGCCGAUAUUGGCAACAGCCUU                    (1bis)

AAGGCTGTTGCCAATATCGGCGGCCGA                    (1ter)

AAGGCUGUUGCCAAUAUCGGCGGCCGA                    (1quater)

TCGGCCGCCGATATTGGCAACAGCCTTTTCTTCCCTG          (2)

UCGGCCGCCGAUAUUGGCAACAGCCUUUUCUUCCCUG          (2bis)

CAGGGAAGAAAAGGCTGTTGCCAATATCGGCGGCCGA          (2ter)

CAGGGAAGAAAAGGCUGUUGCCAAUAUCGGCGGCCGA          (2quater)

GGTACCGTCATCGGCCGCCGATATTGGCAACAGCCTTTTCTTCCCTG    (3)

GGUACCGUCAUCGGCCGCCGAUAUUGGCAACAGCCUUUUCUUCCCUG    (3bis)

CAGGGAAGAAAAGGCTGTTGCCAATATCGGCGGCCGATGACGGTACC    (3ter)

CAGGGAAGAAAAGGCUGUUGCCAAUAUCGGCGGCCGAUGACGGUACC (3quater)
```

à condition que la sonde ne consiste pas en la séquence suivante :
    TCA TCG GCC GCC GAT ATT GGC
-   soit une séquence variante qui se distingue de l'une quelconque des séquences précédentes (1) à (18) :
    .   par l'addition ou l'élimination d'un ou de plusieurs nucléotides au niveau de l'une quelconque de leurs extrémités respectives;
    .   ou par le changement d'un ou de plusieurs nucléotides à l'intérieur de l'une quelconque de ces séquences,
    .   ou par les deux,
    à condition encore que dans l'une quelconque des circonstances ci-dessus, cette sonde s'hybride encore avec la même cible ARN ou ADN que la séquence non modifiée correspondante.

**3.** Sonde pour détecter une ou plusieurs souches de Neisseria, qui cible l'une des séquences définies pas la suite ou la séquence complémentaire correspondante, lorsque le milieu d'hybridation ou le milieu de lavage ou les deux selon ce qui convient, sont les suivants :

milieu d'hybridation : contenant environ 3xSSC, (SSC = NaCl 0,15 M, citrate de sodium 0,015 M, pH 7,0), environ 25 mM de tampon au phosphate pH 7,1, 20% de formamide désionisé, 0,02% de ficoll, 0,02% d'albumine de sérum bovin, 0,02% de polyvinylpyrrolidone et environ 0,1 mg/ml d'ADN de sperme dénaturé, cisaillé,

milieu de lavage : contenant environ 3xSSC, dutampon au phosphate 25 mM, pH 7,1, et 20% de formamide désionisé, dans laquelle ces séquences cibles et les températures d'hybridation (TH) et les températures de lavage (TL) applicables correspondantes, sont respectivement les suivantes :

**AAGGCUGUUGCCAAUAUCGGCGGCCGA**

TH et/ou TL : environ 55°C

**CAGGGAAGAAAAGGCUGUUGCCAAUAUCGGCGGCCGA**

TH et/ou TL : environ 60°C

**CAGGGAAGAAAAGGCUGUUGCCAAUAUCGGCGGCCGAUGACGGUACC**

TH et/ou TL : environ 60°C

GGACUUUUGUCAGGGAAGAAAAGGCUGUUGCCAAUAUCGGCGGCCGAUGACGGUACC

TH et/ou TL : environ 65°C

UUAGCUGUUGGGCAACUUGAUUGCUUGGUAGCGU

TH et/ou TL : environ 55°C à environ 60°C

GUGGGGGAUACCAGAAGUAGGUAGGGUAACCGCAAGGAGUCCGCUUACCACGGUAU

TH et/ou TL : environ 65°C à environ 70°C

ACCUAGGUCCGGCGGAAAUCGGACUGA

TH et/ou TL : environ 55°C

ACCUAGGUCCGGUCGGAAAUCGGACUGA

TH et/ou TL : environ 55°C à environ 60°C

GCAGCACAGGGAAGCUUGCUUCUCGGGUGGCG

TH et/ou TL : environ 55°C à environ 60°C

UAUGCCCUCUAAGGUUAAGGACUUGCUCCGUAAGCCCCG

TH et/ou TL : environ 60°C à environ 65°C

GCGGGGGAGCUGGCAAUAAAGCUAUGAUUCCGC

TH et/ou TL : environ 55°C

GUUCUGAACUGGGUGACUCGAGUGUGUC

TH et/ou TL : environ 55°C à environ 60°C

GCAUACGUCUUGAGAGGGAAAGCA

TH et/ou TL : environ 45°C

UGUACGUAAUAACUGUCGAGA

TH et/ou TL : environ 40°C à environ 45°C

UCUCAACAGCGGUACUAAGCGUACGAAA

TH et/ou TL : environ 50°C à environ 55°C

UGUCUACGGACACGAAGCAACCGAUACCAC

TH et/ou TL : environ 50°C à environ 60°C

UUAGGUUGGCAAGCUGUUGGAAUAGCUU

TH et/ou TL : environ 50°C à environ 55°C

AGCUGGUUGGCGGGGUAAAGGCCCACCA

TH et/ou TL : environ 45°C.

4. Sonde pour détecter une ou plusieurs souches de Neisseria gonorrhoeae parmi d'autres souches bactériennes, et en particulier parmi d'autres souches de Neisseria, contenant :
   - soit une séquence appartenant à un acide nucléique choisi parmi les groupes suivants d'acides nucléiques et qui comprend elle-même de 10 au nombre maximum de nucléotides de l'acide nucléique sélectionné :

```
TCGGCCGCCGATATTGGCAACAGCCTT              (1)
UCGGCCGCCGAUAUUGGCAACAGCCUU              (1bis)
AAGGCTGTTGCCAATATCGGCGGCCGA              (1ter)
AAGGCUGUUGCCAAUAUCGGCGGCCGA              (1quater)
```

TCGGCCGCCGATATTGGCAACAGCCTTTTCTTCCCTG  (2)

UCGGCCGCCGAUAUUGGCAACAGCCUUUUCUUCCCUG  (2bis)

CAGGGAAGAAAAGGCTGTTGCCAATATCGGCGGCCGA  (2ter)

CAGGGAAGAAAAGGCUGUUGCCAAUAUCGGCGGCCGA  (2quater)

GGTACCGTCATCGGCCGCCGATATTGGCAACAGCCTTTTCTTCCCTG  (3)

GGUACCGUCAUCGGCCGCCGAUAUUGGCAACAGCCUUUUCUUCCCUG  (3bis)

CAGGGAAGAAAAGGCTGTTGCCAATATCGGCGGCCGATGACGGTACC  (3ter)

CAGGGAAGAAAAGGCUGUUGCCAAUAUCGGCGGCCGAUGACGGUACC(3quater)

GGTACCGTCATCGGCCGCCGATATTGGCAACAGCCTTTTCTTCCCTGACAAAGTCC

(4)

GGUACCGUCAUCGGCCGCCGAUAUUGGCAACAGCCUUUUCUUCCCUGACAAAAGUCC

(4bis)

GGACTTTTGTCAGGGAAGAAAAGGCTGTTGCCAATATCGGCGGCCGATGACGGTACC

(4ter)

GGACUUUUGUCAGGGAAGAAAAGGCUGUUGCCAAUAUCGGCGGCCGAUGACGGUACC

(4quater)

```
ACGCTACCAAGCAATCAAGTTGCCCAACAGCTAA       (5)
ACGCUACCAAGCAAUCAAGUUGCCCAACAGCUAA       (5bis)
TTAGCTGTTGGGCAACTTGATTGCTTGGTAGCGT       (5ter)
UUAGCUGUUGGGCAACUUGAUUGCUUGGUAGCGU       (5quater)
CGCCACCCGAGAAGCAAGCTTCCCTGTGCTGC         (9)
CGCCACCCGAGAAGCAAGCUUCCCUGUGCUGC         (9bis)
GCAGCACAGGGAAGCTTGCTTCTCGGGTGGCG         (9ter)
GCAGCACAGGGAAGCUUGCUUCUCGGGUGGCG         (9quater)
GCGGAATCATAGCTTTATTGCCAGCTCCCCCGC        (11)
GCGGAAUCAUAGCUUUAUUGCCAGCUCCCCCGC        (11bis)
GCGGGGGAGCTGGCAATAAAGCTATGATTCCGC        (11ter)
GCGGGGGAGCUGGCAAUAAAGCUAUGAUUCCGC        (11quater)
TGCTTTCCCTCTCAAGACGTATGC                 (13)
```

```
UGCUUUCCCUCUCAAGACGUAUGC                    (13bis)
GCATACGTCTTGAGAGGGAAAGCA                    (13ter)
GCAUACGUCUUGAGAGGGAAAGCA                    (13quater)
TGGTGGGCCTTTACCCCGCCAACCAGCT                (18)
UGGUGGGCCUUUACCCCGCCAACCAGCU                (18bis)
AGCTGGTTGGCGGGGTAAAGGCCCACCA                (18ter)
AGCUGGUUGGCGGGGUAAAGGCCCACCA                (18quater)
```

à condition que la sonde ne consiste pas en la séquence suivante :
    TCA TCG GCC GCC GAT ATT GGC

-   soit une séquence variante qui se distingue de l'une quelconque des séquences précédentes (1) à (18) :
    .   par l'addition ou l'élimination d'un ou de plusieurs nucléotides au niveau de l'une quelconque de leurs extrémités respectives;
    .   ou par le changement d'un ou de plusieurs nucléotides à l'intérieur de l'une quelconque de ces séquences,
    .   ou par les deux,
    à condition encore que dans l'une quelconque des circonstances ci-dessus, cette sonde s'hybride encore avec la même cible ARN ou ADN que la séquence non modifiée correspondante..

5.  Sonde pour détecter une ou plusieurs souches de Neisseria gonorrhoeae parmi d'autres souches bactériennes, et en particulier parmi d'autres souches de Neisseria, qui cible l'une des séquences définies pas la suite ou la séquence complémentaire correspondante, lorsque le milieu d'hybridation ou le milieu de lavage ou les deux selon ce qui convient, sont les suivants :

    milieu d'hybridation : contenant environ 3xSSC, (SSC = NaCl 0,15 M, citrate de sodium 0,015 M, pH 7,0), environ 25 mM de tampon au phosphate pH 7,1, 20% de formamide désionisé, 0,02% de ficoll, 0,02% d'albumine de sérum bovin, 0,02% de polyvinylpyrrolidone et environ 0,1 mg/ml d'ADN de sperme de saumon dénaturé, cisaillé.

    milieu de lavage : contenant environ 3xSSC, du tampon au phosphate 25 mM, pH 7,1, et 20% de formamide désionisé, dans laquelle ces séquences cibles et les températures d'hybridation (TH) et les températures de lavage (TL) applicables correspondantes sont respectivement les suivantes :

56

AAGGCUGUUGCCAAUAUCGGCGGCCGA

TH et/ou TL : environ 50°C à environ 65°C

CAGGGAAGAAAAGGCUGUUGCCAAUAUCGGCGGCCGA

TH et/ou TL : environ 60°C à environ 70°C

CAGGGAAGAAAAGGCUGUUGCCAAUAUCGGCGGCCGAUGACGGUACC

TH et/ou TL : environ 65°C à environ 70°C

GGACUUUUGUCAGGGAAGAAAAGGCUGUUGCAAUAUCGGCGGCCGAUGACGGUACC

TH et/ou TL : environ 70°C à environ 75°C

UUAGCUGUUGGGCAACUUGAUUGCUUGGUAGCGU

TH et/ou TL : environ 65°C

GCAGCACAGGGAAGCUUGCUUCUCGGGUGGCG

TH et/ou TL : environ 65°C

GCGGGGGAGCUGGCAAUAAAGCUAUGAUUCCGC

TH et/ou TL : environ 65°C

GCAUACGUCUUGAGAGGGAAAGCA

TH et/ou TL : environ 50°C à environ 55°C

AGCUGGUUGGCGGGGUAAAGGCCCACCA

TH et/ou TL : environ 60°C.

6. Procédé pour détecter des souches de Neisseria dans un échantillon biologique parmi d'autres souches bactériennes, dans lequel ce procédé comprend la mise en contact de cet échantillon biologique dans lequel les acides nucléiques (ADN et ARN) des souches ont été rendus accessibles à une hybridation, si besoin est, dans des conditions dénaturantes convenables, avec une sonde suivant l'une quelconque des revendications 1 à 5, dans des conditions permettant une hybridation entre la sonde et les acides nucléiques complémentaires des souches de Neisseria, qui peuvent être présentes dans l'échantillon, et la détection des hybrides éventuellement formés.

7. Procédé pour détecter des souches de Neisseria dans un échantillon biologique parmi d'autres souches bactériennes suivant la revendication 6, dans lequel les sondes utilisées sont celles qui s'hybrident à la fois avec l'ADN et l'ARN des souches de Neisseria qui peuvent être présentes dans l'échantillon biologique.

8. Procédé pour détecter des souches de Neisseria parmi d'autres souches bactériennes suivant les revendications 6 ou 7, dans lequel le milieu d'hybridation contient environ 3xSSC, (SSC = NaCl 0,15M, citrate de sodium 0,015 M, pH 7,0), environ 25 mM de tampon au phosphate pH 7,1, 20% de formamide désionisé, 0,02% de ficoll, 0,02% d'albumine de sérum bovin, 0,02% de polyvinylpyrrolidone et environ 0,1 mg/ml d'ADN de sperme de saumon dénaturé, cisaillé, ou le milieu de lavage contient environ 3xSSC, du tampon au phosphate 25 mM, pH 7,1, et 20% de formamide désionisé, et dans lequel la sonde utilisée est l'une quelconque des sondes (1), (1 bis), (1 ter) ou (1 quater) de la revendication 2, la température d'hybridation étant convenablement ajustée dans la gamme d'environ 55°C, de préférence d'environ 53°C et/ou la température de lavage dans la gamme d'environ 55°C, de préférence d'environ 53°C, ou l'une quelconque des sondes (2), (2 bis), (2 ter) ou (2 quater) de la revendication 2, la température d'hybridation étant convenablement ajustée dans la gamme d'environ 60°C et/ou la température de lavage dans la gamme d'environ 60°C, ou l'une quelconque des sondes (3), (3 bis), (3 ter) ou (3 quater) de la revendication 2, la température d'hybridation étant convenablement ajustée dans la gamme d'environ 60°C et/ou la température de lavage dans la gamme d'environ 60°C, ou l'une quelconque des sondes (4), (4 bis), (4 ter) ou (4 quater) de la revendication 1, la température d'hybridation étant convenablement ajustée dans la gamme d'environ 65°C et/ou la température de lavage dans la gamme d'environ 65°C, ou l'une quelconque des sondes (5), (5 bis), (5 ter) ou (5 quater) de la revendication 1, la température d'hybridation étant convenablement ajustée dans la gamme d'environ 55°C et/ou la température de lavage dans la gamme d'environ 55°C, ou l'une quelconque des sondes (9), (9 bis), (9 ter) ou (9 quater) de la revendication 1, la température d'hybridation étant convenablement ajustée dans la gamme d'environ 60°C et/ou la température de lavage dans la gamme d'environ 60°C, ou l'une quelconque des sondes (6), (6 bis), (6 ter) ou (6 quater) de la revendication 1, la température d'hybridation étant convenablement ajustée dans la gamme d'environ 65°C et/ou la température de lavage dans la gamme d'environ 65°C à environ 70°C, ou l'une quelconque des sondes (7), (7 bis), (7 ter) ou (7 quater) de la revendication 1, la température d'hybridation étant convenablement ajustée dans la gamme d'environ 55°C et/ou la température de lavage dans la gamme d'environ 55°C, ou l'une quelconque des sondes (8), (8 bis), (8 ter) ou (8 quater) de la revendication 1, la température d'hybridation étant convenablement ajustée dans la gamme d'environ 55°C, et/ou la température de lavage dans la gamme d'environ 55°C à environ 60°C, ou l'une quelconque des sondes (10), (10 bis), (10 ter) ou (10 quater) de la revendication 1, la température d'hybridation étant convenablement ajustée dans la gamme d'environ 55°C et/ou la température de lavage dans la gamme d'environ 55°C à environ 60°C, ou l'une quelconque des sondes (11), (11 bis), (11 ter) ou (11 quater) de la revendication 1, la température d'hybridation étant convenablement ajustée dans la gamme d'environ 55°C et/ou la température de lavage dans la gamme d'environ 55°C, ou l'une quelconque des sondes (12), (12 bis), (12 ter) ou (12 quater) de la revendication 1, la température d'hybridation étant convenablement ajustée dans la gamme d'environ 55°C à environ 60°C et/ou la température de lavage dans la gamme d'environ 55°C à environ 60°C, ou l'une quelconque des sondes (13), (13 bis), (13 ter) ou (13 quater) de la revendication 1, la température d'hybridation étant convenablement ajustée dans la gamme d'environ 45°C et/ou la température de lavage dans la gamme d'environ 45°C, ou l'une quelconque des sondes (14), (14 bis), (14 ter) ou (14 quater) de la revendication 1, la température d'hybridation étant convenablement ajustée dans la gamme d'environ 40°C à environ 45°C, et/ou la température de lavage dans la gamme d'environ 40°C à environ 45°C, ou l'une quelconque des sondes (15), (15 bis), (15 ter) ou (15 quater) de la revendication 1, la température d'hybridation étant convenablement ajustée dans la gamme d'environ 50°C à environ 55°C, et/ou la température de lavage dans la gamme d'environ 50°C à environ 55°C, ou l'une quelconque des sondes (16), (16 bis), (16 ter) ou (16 quater) de la revendication 1, la température d'hybridation étant convenablement ajustée dans la gamme d'environ 50°C à environ 60°C et/ou la température de lavage dans la gamme d'environ 50°C à environ 60°C, ou l'une quelconque des sondes (17), (17 bis), (17 ter) ou (17 quater) de la revendication 1, la température d'hybridation étant convenablement ajustée dans la gamme d'environ 50°C à environ 55°C et/ou la température de lavage dans la gamme d'environ 50°C à environ 55°C, ou l'une quelconque des sondes (18), (18 bis), (18 ter) ou (18 quater) de la revendication 1, la température d'hybridation étant convenablement ajustée dans la gamme d'environ 45°C et/ou la température de lavage dans la gamme d'environ 45°C.

9. Procédé pour détecter des souches de Neisseria gonorrhoeae parmi d'autres souches bactériennes et en particulier parmi d'autres souches de Neisseria, lequel procédé comprend la mise en contact de cet échantillon biologique, dans lequel les acides nucléiques (ADN et ARN) des souches ont été rendus accessibles à une hybridation, si besoin est, dans des conditions dénaturantes convenables, avec une sonde selon l'invention spécifique pour des souches de Neisseria gonorrhoeae et choisie parmi les sondes (1), (1 bis), (1 ter), (1 quater), (2), (2 bis), (2 ter), (2 quater), (3), (3 bis), (3 ter), (3 quater), (4), (4 bis), (4 ter), (4 quater), (5), (5 bis), (5 ter), (5 quater), (9), (9 bis), (9 ter), (9 quater), (11), (11 bis), (11 ter), (11 quater), (13), (13 bis), (13 ter), (13 quater), (18), (18 bis), (18 ter) et (18 quater) selon les revendications 1 ou 2, chaque fois qu'il est nécessaire, dans des conditions d'hybridation et de lavage ajustées de façon à assurer une hybridation spécifique avec les acides nucléiques complémentaires des souches de Neisseria gonorrhoeae, qui peuvent être présentes dans l'échantillon, mais non avec l'ADN ou l'ARN complémentaire d'autres espèces de Neisseria, et la détection des hybrides éventuellement formés.

10. Procédé pour détecter des souches de Neisseria gonorrhoeae parmi d'autres souches bactériennes et en particulier parmi d'autres souches de Neisseria, suivant la revendication 9, dans lequel le milieu d'hybridation contient environ 3xSSC, (SSC = NaCl 0,15 M, citrate de sodium 0,015 M, pH 7,0), environ 25 mM de tampon au phosphate pH 7,1, 20% de formamide désionisé, 0,02% de ficoll, 0,02% d'albumine de sérum bovin, 0,02% de polyvinylpyrrolidone et environ 0,1 mg/ml d'ADN de sperme de

58

saumon dénaturé, cisaillé, ou le milieu de lavage contient environ 3xSSC, du tampon au phosphate 25 mM, pH 7,1, et 20% de formamide désionisé, et

dans lequel la sonde utilisée est l'une quelconque des sondes (1), (1 bis), (1 ter) ou (1 quater) de la revendication 2, la température d'hybridation étant convenablement ajustée dans la gamme d'environ 55°C, de préférence d'environ 53°C et/ou la température de lavage dans la gamme d'environ 55°C à environ 65°C, de préférence d'environ 53°C à environ 65°C et de préférence d'environ 53°C, ou l'une quelconque des sondes (2), (2 bis), (2 ter) ou (2 quater) de la revendication 2, la température d'hybridation étant convenablement ajustée dans la gamme d'environ 60°C et/ou la température de lavage dans la gamme d'environ 65°C à environ 70°C, ou l'une quelconque des sondes (3), (3 bis), (3 ter) ou (3 quater) de la revendication 2, la température d'hybridation étant convenablement ajustée dans la gamme d'environ 60°C et/ou la température de lavage dans la gamme d'environ 65°C à environ 70°C, ou l'une quelconque des sondes (4), (4 bis), (4 ter) ou (4 quater) de la revendication 1, la température d'hybridation étant convenablement ajustée dans la gamme d'environ 65°C et/ou la température de lavage dans la gamme d'environ 70°C à environ 75°C, ou l'une quelconque des sondes (5), (5 bis), (5 ter) ou (5 quater) de la revendication 1, la température d'hybridation étant convenablement ajustée dans la gamme d'environ 60°C et/ou la température de lavage dans la gamme d'environ 65°C, ou l'une quelconque des sondes (9), (9 bis), (9 ter) ou (9 quater) de la revendication 1, la température d'hybridation étant convenablement ajustée dans la gamme d'environ 60°C à 65°C et/ou la température de lavage dans la gamme d'environ 65°C, ou plus avantageusement l'une quelconque des sondes (1), (1 bis), (1 ter) ou (1 quater) de la revendication 2, la température d'hybridation étant convenablement ajustée dans la gamme d'environ 55°C et/ou la température de lavage dans la gamme d'environ 55°C à environ 65°C, ou l'une quelconque des sondes (11), (11 bis), (11 ter) ou (11 quater) de la revendication 1, la température d'hybridation étant convenablement ajustée dans la gamme d'environ 65°C et/ou la température de lavage dans la gamme d'environ 65°C, ou l'une quelconque des sondes (13), (13 bis), (13 ter) ou (13 quater) de la revendication 1, la température d'hybridation étant convenablement ajustée dans la gamme d'environ 50°C à environ 55°C et/ou la température de lavage dans la gamme d'environ 50°C à environ 55°C, ou l'une quelconque des sondes (18), (18 bis), (18 ter) ou (18 quater) de la revendication 1, la température d'hybridation étant convenablement ajustée dans la gamme d'environ 60°C et/ou la température de lavage dans la gamme d'environ 60°C.

11. Trousse pour détecter in vitro des souches de Neisseria dans un échantillon biologique, cette trousse contenant:
- au moins une sonde choisie parmi l'une quelconque de celles qui sont définies dans les revendications 1 à 4;
- le tampon ou les composants nécessaires pour produire le tampon permettant la réalisation de la réaction d'hybridation entre ces sondes et les ADN et/ou ARN d'un grand nombre, de préférence de toutes les souches de Neisseria;
- lorsque cela est approprié, des moyens pour détecter les hybrides résultant de l'hybridation précédente.

12. Trousse pour détecter in vitro des souches de Neisseria gonorrhoeae dans un échantillon biologique, cette trousse contenant:
- au moins une sonde spécifique pour N. gonorrhoeae telle que définie plus haut, par exemple, une sonde choisie parmi les sondes (2), (2 bis), (2 ter), (2 quater), (3), (3 bis), (3 ter), (3 quater), (4), (4 bis), (4 ter), (4 quater), (5), (5 bis), (5 ter), (5 quater), (9), (9 bis), (9 ter), (9 quater), (11), (11 bis), (11 ter), (11 quater), (13), (13 bis), (13 ter), (13 quater), (18), (18 bis), (18 ter) et (18 quater) suivant les revendications 1 ou 2, ou plus avantageusement, une sonde choisie parmi l'une quelconque des sondes (1), (1 bis), (1 ter), (1 quater), selon la revendication 2;
- le tampon ou les composants nécessaires pour produire le tampon permettant la réalisation de la réaction d'hybridation entre ces sondes et les ADN et/ou ARN d'une souche de Neisseria gonorrhoeae;
- lorsque cela est approprié, des moyens pour détecter les hybrides résultant de l'hybridation précédente.

13. Procédé pour détecter des souches de Neisseria gonorrhoeae parmi d'autres souches bactériennes et en particulier parmi d'autres souches de Neisseria, lequel procédé comprend la mise en contact de cet échantillon biologique dans lequel les acides nucléiques (ADN et ARN) des souches ont été rendus accessibles à une hybridation, si besoin est, dans des conditions dénaturantes convenables, avec deux sondes suivant l'une quelconque des revendications 1 à 5, qui sont spécifiques pour les souches de Neisseria gonorrhoeae et ne sont pas respectivement capables de s'hybrider aux mêmes souches non cibles, et en particulier choisies parmi les combinaisons suivantes:

- une sonde du groupe 9 et l'une quelconque des sondes des groupes suivants : 1, 2, 3, 5 et 13;
- une sonde du groupe 13 et l'une quelconque des sondes des groupes suivants : 1, 2, 3 et 5;
- une sonde du groupe 18 et l'une quelconque des sondes des groupes suivants : 1, 2, 3, 5, 9 et 13;
- une sonde du groupe 5 et l'une quelconque des sondes des groupes suivants : 1, 2 et 3; et plus particulièrement, choisies parmi les combinaisons suivantes :
- une sonde du groupe 9 et l'une du groupe 5;
- une sonde du groupe 18 et l'une quelconque des sondes des groupes suivants : 5 et 9;

chaque fois que cela est nécessaire, dans des conditions d'hybridation et de lavage ajustées de façon à assurer une hybridation spécifique avec les acides nucléiques complémentaires des souches de Neisseria gonorrhoeae, qui peuvent être présentes dans l'échantillon, mais non avec l'ADN ou l'ARN complémentaire d'autres espèces de Neisseria, et la détection des hybrides éventuellement formés.

14. Trousse pour un essai d'hybridation sandwich pour la détection in vitro de souches de Neisseria gonorrhoeae dans un échantillon biologique, cette trousse contenant :

- au moins deux sondes spécifiques pour N. gonorrhoeae choisies parmi les combinaisons suivantes :
  - une sonde du groupe 9 et l'une quelconque des sondes des groupes suivants : 1, 2, 3, 5 et 13;
  - une sonde du groupe 13 et l'une quelconque des sondes des groupes suivants : 1, 2, 3 et 5;
  - une sonde du groupe 18 et l'une quelconque des sondes des groupes suivants : 1, 2, 3, 5, 9 et 13;
  - une sonde du groupe 5 et l'une quelconque des sondes des groupes suivants : 1, 2 et 3; et plus particulièrement, choisies parmi les combinaisons suivantes :
  - une sonde du groupe 9 et l'une du groupe 5;
  - une sonde du groupe 18 et l'une quelconque des sondes des groupes suivants : 5 et 9;
- le tampon ou les composants nécessaires pour produire le tampon permettant la réalisation d'une réaction d'hybridation entre ces sondes et les ADN et/ou ARN d'une souche de Neisseria gonorrhoeae;
- lorsque cela est approprié, des moyens pour détecter les hybrides résultant de l'hybridation précédente.

FIG.1

Probe n° 1, 2, 3 and 4.

n°4

n°3

n°2

n°1

```
PT  C U G C U U U U G U A C G G A A C G A A A A G C C U G G G G C U A A U A U C C C C G G G U C A U G A C G G U A C C
NG  G G A C U U U U G U C A G G G A A G A A A A G G C U G U U G C C A A U A U C G G C G G C C G A U G A C G G U A C C
EC  G U A C U U U C A G C G G G G A G G A A G G G A G U A A A G U U A A U A C C U U U G C U C A U U G A C G U U A C C
```

Probe n°5

```
PT  C U G G U U G U U G G G U C U   U A A C U G A C U C A G U A A C G A
NG  U U A G C U G U U G G G C A A C U U G A U U G C U U G G U A G C G U
EC  C U U G G A G G U U G U G C C C U U G A G G C G U G G C U U C C G G
```

FIG.2a

EP 0 337 896 B1

Probe n°6

PT  G C G G G U C U C G C C A G A A G U A G G U A G C C U A A C C G U A A G G A G G G C G C U U A C C A C G G C G G

NG  G U G G G G G A U A C C A G A A G U A G G U A G G G U A A C C G C A A G G A G U C C G C U U A C C A C G G U A U

EC  G U G G G U U G C A A A A G A A G U A G G U A G C U U A A C C U U C G G G A G G G C G C U U A C C A C U U U G U

EP 0 337 896 B1

Probe n°9

PT  G U A A C      A G G U C U U C G G A U      G C U G A C G

NG  G C A G C A C A G G G A A G C U U G C U U C U C G G G U G G C G

EC  G U A A C A G G A A G A A G C U U G C U C U U U   G C U G A C G

FIG. 2b

## Probe n° 7

```
NG   A C C U A G G U C C G G   C G G A A A U C G G A C U G A
         ■ ■   ■         ■     ■ ■   ■ ■ ■ ■   ■ ■ ■   ■         ■
EC   G G C U A A U C C U G G U C G G A C A U C A G G A G G U
```

## Probe n° 8

```
NG   A C C U A G G U C C G G U C G G A A A U C G G A C U G A
         ■ ■ ■         ■   ■ ■ ■ ■ ■ ■   ■ ■ ■   ■         ■
EC   G G C U A A U C C U G G U C G G A C A U C A G G A G G U
```

## Probe n° 10

```
NG   U A U G C C C U C   U A   A G G U U A A G G A   C U U G C U C C G U A A G C C C C G
         ■   ■ ■         ■     ■ ■ ■ ■   ■   ■     ■ ■   ■ ■               ■ ■ ■
EC   C A C G C U G A U A U G U A G G U G A G G U C C C U C G C G G A U G G A G C U G A A
```

## Probe n° 11

```
NG   G C G G G G G A G C T G G C A A T A A A G C T A T G A T T C C G C
         ■     ■ ■     ■ ■   ■ ■   ■     ■ ■     ■         ■     ■ ■ ■     FIG.3
EC   G T C G G T A A G G T G A T A T G A A C C G T T A T A A C C G G C
```

EP 0 337 896 B1

FIG. 4

FIG.5

# FIG. 6

Probe :  n° 3  n° 5  n° 6  n° 7

HT (°C) :  65  55  65  55

WT (°C) :  65  60  65  55

EP 0 337 896 B1

WASH TEMPERATURE (°C)

FIG.7

## E.coli 16S rRNA

```
         10         20         30         40         50         60
AAAUUGAAGA GUUUGAUCAU GGCUCAGAUU GAACGCUGGC GGCAGGCCUA ACACAUGCAA

         70         80         90        100        110        120
GUCGAACGGU AACAGGAAGA AGCUUGCUCU UUGCUGACGA GUGGCGGACG GGUGAGUAAU

        130        140        150        160        170        180
GUCUGGGAAA CUGCCUGAUG GAGGGGGAUA ACUACUGGAA ACGGUAGCUA AUACCGCAUA

        190        200        210        220        230        240
ACGUCGCAAG ACCAAAGAGG GGGACCUUCG GGCCUCUUGC CAUCGGAUGU GCCCAGAUGG

        250        260        270        280        290        300
GAUUAGCUAG UAGGUGGGGU AACGGCUCAC CUAGGCGACG AUCCCUAGCU GGUCUGAGAG

        310        320        330        340        350        360
GAUGACCAGC CACACUGGAA CUGAGACACG GUCCAGACUC CUACGGGAGG CAGCAGUGGG

        370        380        390        400        410        420
GAAUAUUGCA CAAUGGGCGC AAGCCUGAUG CAGCCAUGCC GCGUGUAUGA AGAAGGCCUU

        430        440        450        460        470        480
CGGGUUGUAA AGUACUUUCA GCGGGGAGGA AGGGAGUAAA GUUAAUACCU UUGCUCAUUG

        490        500        510        520        530        540
ACGUUACCCG CAGAAGAAGC ACCGGCUAAC UCCGUGCCAG CAGCCGCGGU AAUACGGAGG

        550        560        570        580        590        600
GUGCAAGCGU UAAUCGGAAU UACUGGGCGU AAAGCGCACG CAGGCGGUUU GUUAAGUCAG

        610        620        630        640        650        660
AUGUGAAAUC CCCGGGCUCA ACCUGGGAAC UGCAUCUGAU ACUGGCAAGC UUGAGUCUCG

        670        680        690        700        710        720
UAGAGGGGGG UAGAAUUCCA GGUGUAGCGG UGAAAUGCGU AGAGAUCUGG AGGAAUACCG

        730        740        750        760        770        780
GUGGCGAAGG CGGCCCCCUG GACGAAGACU GACGCUCAGG UGCGAAAGCG UGGGGAGCAA

        790        800        810        820        830        840
ACAGGAUUAG AUACCCUGGU AGUCCACGCC GUAAACGAUG UCGACUUGGA GGUUGUGCCC
```

## FIG.8a

```
        850          860          870          880          890          900
UUGAGGCGUG   GCUUCCGGAG   CUAACGCGUU   AAGUCGACCG   CCUGGGGAGU   ACGGCCGCAA

        910          920          930          940          950          960
GGUUAAAACU   CAAAUGAAUU   GACGGGGGCC   CGCACAAGCG   GUGGAGCAUG   UGGUUUAAUU

        970          980          990         1000         1010         1020
CGAUGCAACG   CGAAGAACCU   UACCUGGUCU   UGACAUCCAC   GGAAGUUUUC   AGAGAUGAGA

       1030         1040         1050         1060         1070         1080
AUGUGCCUUC   GGGAACCGUG   AGACAGGUGC   UGCAUGGCUG   UCGUCAGCUC   GUGUUGUGAA

       1090         1100         1110         1120         1130         1140
AUGUUGGGUU   AAGUCCCGCA   ACGAGCGCAA   CCCUUAUCCU   UUGUUGCCAG   CGGUCCGGCC

       1150         1160         1170         1180         1190         1200
GGGAACUCAA   AGGAGACUGC   CAGUGAUAAA   CUGGAGGAAG   GUGGGGAUGA   CGUCAAGUCA

       1210         1220         1230         1240         1250         1260
UCAUGGCCCU   UACGACCAGG   GCUACACACG   UGCUACAAUG   GCGCAUACAA   AGAGAAGCGA


       1270         1280         1290         1300         1310         1320
CCUCGCGAGA   GCAAGCGGAC   CUCAUAAAGU   GCGUCGUAGU   CCGGAUUGGA   GUCUGCAACU

       1330         1340         1350         1360         1370         1380
CGACUCCAUG   AAGUCGGAAU   CGCUAGUAAU   CGUGGAUCAG   AAUGCCACGG   UGAAUACGUU

       1390         1400         1410         1420         1430         1440
CCCGGGCCUU   GUACACACCG   CCCGUCACAC   CAUGGGAGUG   GGUUGCAAAA   GAAGUAGGUA

       1450         1460         1470         1480         1490         1500
GCUUAACCUU   CGGGAGGGCG   CUUACCACUU   UGUGAUUCAU   GACUGGGGUG   AAGUCGUAAC

       1510         1520         1530         1540
AAGGUAACCG   UAGGGGAACC   UGCGGUUGGA   UCACCUCCUU   A
```

# FIG.8b

70

# FIG.9

E. coli 23S rRNA (partial)

G. GTTAAGCGAC TAAGCGTACA CGGTGGATGC CCTGGCAGTC 41

AGAGGCGATG AAGGACGTGC TAATCTGCGA TAAGCGTCGG TAAGGTGATA TGAACCGTTA 101

TAACCGGCGA TTTCCGAATG GGGAAACCCA GTGTGTTTCG ACACACTATC ATTAACTGAA 161

TCCATAGGTT AATGAGGCGA ACCGGGGGAA CTGAAACATC TAAGTACCCC GAGGAAAAGA 221

AATCAACCGA GATTCCCCCA GTAGCGGCGA GCGAACGGGG AGCAGCCCAG AGCCTGAATC 281

EP 0 337 896 B1

FIG. 10

(n°12)  VI

VII

III

(n°5)

II
(n°1 to 4)

I
(n°9)

VIII

(n°18)

FIG.11

IV
(n°6)

V
(n°13)

FIG.12

FIG.13

FIG.14

FIG.15

| | | | |
|---|---|---|---|
| 1. | Neisseria gonorrhoeae | NCTC | 8375 T |
| 2. | Neisseria gonorrhoeae | ITG | 4339 |
| 3. | Neisseria gonorrhoeae | ITG | 4085 |
| 4. | Neisseria gonorrhoeae | ITG | 4308 |
| 5. | Neisseria gonorrhoeae | ITG | 3939 |
| 6. | Neisseria gonorrhoeae | ITG | 4363 |
| 7. | Neisseria gonorrhoeae | ITG | 4367 |
| 8. | Neisseria gonorrhoeae | ITG | 4401 |
| 9. | Neisseria gonorrhoeae | ITG | 4437 |
| 10. | Neisseria gonorrhoeae | ITG | 572 |
| 11. | Neisseria gonorrhoeae | ITG | 3043 |
| 12. | Neisseria gonorrhoeae | ATCC | 43831 |
| 13. | Neisseria meningitidis | NCTC | 10025 T |
| 14. | Neisseria meningitidis | ITG | 3342 |
| 15. | Neisseria meningitidis | ITG | 3343 |
| 16. | Neisseria meningitidis | ITG | 3345 |
| 17. | Neisseria meningitidis | ITG | 3346 |
| 18. | Neisseria meningitidis | ITG | 3348 |
| 19. | Neisseria meningitidis | ITG | 3349 |
| 20. | Neisseria meningitidis | ITG | 3350 |
| 21. | Neisseria meningitidis | ITG | 3357 |
| 22. | Neisseria meningitidis | ITG | 3362 |
| 23. | Pseudomonas testosteroni | ATCC | 17407 |
| 24. | Branhamella catarrhalis | ITG | 4197 |

| | | |
|---|---|---|
| 1 | 2 | 3 |
| 4 | 5 | 6 |
| 7 | 8 | 9 |
| 10 | 11 | 12 |
| 13 | 14 | 15 |
| 16 | 17 | 18 |
| 19 | 20 | 21 |
| 22 | 23 | 24 |

FIG.16

78

FILTER I

FILTER II

FIG.17

FIG.18